# EUROPEAN PATENT APPLICATION

(11) **EP 4 763 138 A1**
(43) Date of publication of application: **24.06.2026**
(21) Application number: 24853841.5
(22) Date of filing: 15.08.2024
(51) Int. Cl.: A61F 2/24

(54) **CONTROL MECHANISM FOR ARTIFICIAL IMPLANT, LOCKING MECHANISM, INTERVENTIONAL DELIVERY SYSTEM, CONTROL METHOD, AND LOADING METHOD**

(30) Priority: 17.08.2023 CN 202311041416
(71) Applicant: Venus MedTech (HangZhou), Inc., Hangzhou, Zhejiang 310052 (CN); Hangzhou Qianjiang Medical and Industrial Cross Innovation Technology Research Institute, Hangzhou, Zhejiang 310052 (CN)
(72) Inventor: LEI, Rongjun, Hangzhou, Zhejiang 310052 (CN); CHEN, Wei, Hangzhou, Zhejiang 310052 (CN); YANG, Lingfeng, Hangzhou, Zhejiang 310052 (CN); WANG, Xiang, Hangzhou, Zhejiang 310052 (CN); ZHU, Lingke, Hangzhou, Zhejiang 310052 (CN)
(74) Representative: KIPA AB
(86) International application number: PCT/CN2024/112212
(87) International publication number: WO 2025/036434

(57) **Abstract**

This application discloses a control mechanism, a locking mechanism, an interventional delivery system, a control method, and a loading method for an artificial implant. The control mechanism includes a base, a pulling wire and a locking member, wherein the base defines a locking hole, the pulling wire includes a free end that can pass through, wind around, or be detached from the artificial implant. The locking member as a whole is located at a distal end of the base, and rotatably engages with the base to lock the free end of the pulling wire. After optimized, components of the control mechanism can cooperate with each other to realize the control of in-vivo expansion, release, or retrieval of the artificial implant, which is more suitable for in-vivo work.

## Description

### TECHNICAL FIELD

This application relates to the technical field of medical devices, and in particular to a control mechanism, locking mechanism, interventional delivery system, control method, and loading method for an artificial implant.

### BACKGROUND

With the development of medical technology, artificial implants have been used to treat heart valve disorders. Common treatment methods generally include surgical repair or replacement of the valves, or implantation of artificial implants through flexible catheter intervention.

In interventional techniques, the artificial implant in a compressed and loaded state is installed at a distal end of a catheter assembly and delivered to a predetermined site. After the artificial implant expands radially and is released, the catheter assembly is withdrawn from the human body.

For the connection between the artificial implant and catheter assembly, in the art, direct clamping or wire control methods may be used. The wire control method involves using a flexible component to bind the artificial implant to the distal end of the catheter assembly and locking the flexible component to maintain the compressed state of the artificial implant. When it is necessary to release the artificial implant, the locking of the flexible component is released, and the flexible component then loosens until it separates from the artificial implant to complete the release of the artificial implant. However, the control methods and control structures for the flexible component in the art still need to be improved.

### SUMMARY

### TECHNICAL PROBLEM

Generally, the flexible component is controlled by a control handle. The existing control handle includes a support body, a transmission component movably mounted on the support body, and a drive component movably mounted on the support body to drive the transmission component. The support body needs to balance its structural strength with the installation of the transmission component and the drive component, resulting in a complex structure. Furthermore, as the wire control structure and operation become more complex, the structural complexity of the support body will increase, potentially requiring a trade-off between its structural strength and the convenience of installation or operation.

### TECHNICAL SOLUTION

This application provides a control mechanism for an artificial implant, which enables more reasonable control (including locking and unlocking) of a flexible member.

The present application provides a control mechanism for an artificial implant, including:
a base defining a locking hole;
a pulling wire having a free end capable of passing through and winding around the artificial implant or being detached from the artificial implant; and
a locking member as a whole located at a distal end of the base, the locking member rotatably engaged with the base to lock or unlock the free end of the pulling wire.

Optionally, the control mechanism further includes a first shaft, a second shaft, and a third shaft that are slidably nested in sequence from the inside out, the base is connected to a distal end of the third shaft, the pulling wire is connected to a distal end of the second shaft, and the locking member is connected to a distal end of the first shaft.

Optionally, the base has a locking area, the locking member includes a positioning portion that enters or exits the locking area during its rotation, and the positioning portion cooperates with the free end of the pulling wire to restrict the artificial implant;
the base includes a distal end and a proximal end opposite to each other, as well as an axial direction extending between the distal end and the proximal end, a first cavity is defined in the base, and a first opening is defined in a proximal end side of the base and communicates with the first cavity, and a second opening is defined in an outer circumferential surface of the base and communicates with the first cavity;
the pulling wire extends through the first cavity, one end of the pulling wire extends proximally out of the base through the first opening, and another end/free end of the pulling wire extends out of the base through the second opening for connecting the artificial implant.

Optionally, the locking area is located at the second opening.

Optionally, there are multiple second openings arranged at intervals along a circumferential direction of the base, and multiple ribs are formed between adjacent second openings.

Optionally, all of the ribs converge and are fixed to each other at the distal end of the base.

Optionally, all of the ribs converge to form an annular structure.

Optionally, an open direction of the locking hole is along the circumferential direction of the base.

Optionally, at least one rib defines the locking hole.

Optionally, each rib defines the locking hole.

Optionally, there are multiple ribs configured with the locking holes, the positioning portion as a whole includes a head end and a tail end opposite to each other, and the head end is engaged into or disengaged from the locking holes in sequence along with the rotation of the locking member.

Optionally, there is/are one locking hole or multiple locking holes on one rib.

Optionally, there are multiple locking holes arranged in sequence along an extending direction of the rib.

Optionally, the free end of the pulling wire has a first state being restricted to the base and a second state being released from the base, and the restriction on the artificial implant is released when the cooperation of the locking member and the free end of the pulling wire is released;
in the first state, the free end extends out of the base through a corresponding second opening, winds around the artificial implant, and returns to a locking area corresponding to the same second opening, or returns to a locking area corresponding to an adjacent second opening.

Optionally, the free end of the pulling wire in the first state is located in a current locking area, and two ends of the positioning portion located in the current locking area are inserted into the locking holes at corresponding sides, respectively.

Optionally, there are multiple locking areas arranged at intervals along a circumferential direction of the base, and the positioning portion enters or exits the locking areas in sequence along with the movement of the locking member.

Optionally, the locking member has a positioning portion that enters or exits the locking area during its rotation, and the positioning portion cooperates with the free end of the pulling wire to restrict the artificial implant;
the positioning portion has a curved shape and extends along the circumferential direction the base, and enters or exits the locking hole along with the rotation of the locking member.

Optionally, the positioning portion includes at least one arc-shaped structure that cooperates with the base.

Optionally, the positioning portion is configured as a helical structure.

Optionally, the helical structure is wound at least once.

Optionally, the helical structure is a multi-turn structure, and the turns are arranged along an axial direction of the base.

Optionally, the helical structure is a cylindrical helical structure or at least partially a conical helical structure.

Optionally, along a winding direction of the helical structure, the positioning portion as a whole includes a distal end and a head end opposite to each other, and a connecting portion is fixed to one of the head end and the distal end.

Optionally, the connecting portion is tubular-shaped, and the distal end of the positioning portion is mounted around an outer circumference of the connecting portion or abuts against a proximal end of the connecting portion.

Optionally, the connecting portion is movably mounted around the distal end of the first shaft, and is capable of being separated from the distal end of the first shaft in the axial direction.

Optionally, the free end of the pulling wire has a first state being restricted to the base and a second state being released from the base, and the restriction on the artificial implant is released when the cooperation of the locking member and the free end of the pulling wire is released;
in the first state, a limit mechanism is provided between the locking member and the base to prevent the locking member from rotating in an insertion direction.

Optionally, the positioning portion as a whole has a tail end and a head end opposite to each other, and a connecting portion is fixed to one of the tail end and the head end, a positioning mechanism is provided between the connecting portion and the base to maintain their axial positions;
the positioning mechanism includes a first end surface provided at a proximal end of the connecting portion and a second end surface provided at the distal end of the base, wherein the second end face abuts against the first end surface.

Optionally, the connecting portion is tubular-shaped with constant diameter or at least expands radially outward at its proximal end, a proximal end of the connecting portion serves as the connecting section, the distal end of the base includes an extension section, and the connecting section and the extension section are nested with each other.

Optionally, the free end of the pulling wire has a first state being restricted to the base and a second state being released from the base, and the restriction on the artificial implant is released when the cooperation of the locking member and the free end of the pulling wire is released.

Optionally, the pulling wire further includes a control end opposite to the free end, the control end is movable relative to the base; when the free end is in the first state, a length of the pulling wire exposed outside the base is adjustable by operating the control end; and when the free end is in the second state, the pulling wire is detachable from the artificial implant by operating the control end.

Optionally, the pulling wire further includes a control end opposite to the free end, there are multiple pulling wires, the free ends of the pulling wires move independently of each other, while the control ends of the pulling wires move synchronously.

Optionally, the control ends of the pulling wires are connected to the second shaft.

Optionally, the base has a locking area, the locking member has a positioning portion that enters or exits the locking area during its rotation, and the positioning portion cooperates with the free end of the pulling wire to restrict the artificial implant;
the free end has a ring, when the free end is in the first state, the positioning portion enters the ring; and when the free end is in the second state, the positioning portion exits the ring.

Optionally, the ring is configured independently or formed by winding the pulling wire.

Optionally, the positioning portion is helical-shaped with multiple turns, an axial gap is defined between adjacent turns; the pulling wire in the first state extends out of the base through a current axial gap, winds around the artificial implant, and is mounted around a turn adjacent to the current axial gap.

Optionally, the pulling wire in the first state extends out of the base through a current locking area, winds around the artificial implant, and returns to the current locking area.

Optionally, the artificial implant has an axial direction, and one end of the artificial implant in the axial direction is configured as a wire control end, the wire control end has eyelets for extending of the pulling wire therethrough, and the artificial implant is configurable between the following states based on its deformation:
a collapsed state, wherein the wire control end is radially folded close to the base;
an expanded state, wherein the wire control end expands radially relative to the base;
a round-trip path of the pulling wire winding around the artificial implant do not coincide.

Optionally, there are multiple eyelets being spaced from each each other, and in the expanded state, the pulling wire passes through at least two eyelets.

Optionally, the pulling wire winds around the artificial implant to form a triangular-shaped round-trip path.

Optionally, a number of the eyelets is twice that of the pulling wires, and in the expanded state, each pulling wire corresponds to two eyelets.

The present application further provides a locking mechanism for connecting an artificial implant to a delivery system, including:
a first shaft with a loading section fixed to its distal end, the first loading section opening towards a proximal end for accommodating a distal part of the artificial implant;
a pulling wire having a free end being capable of winding around and extending through the artificial implant or detached from the artificial implant;
a third shaft being slidably mounted around the first shaft, a locking assembly being set on the third shaft, wherein the artificial implant is bound to the locking assembly by the pulling wire during loading, and the locking assembly includes a locking member and a base, and has a locking state that the locking member and the base cooperate with each other to restrict the pulling wire from being detached from the artificial implant and an unlocking state that the locking member and the base are disengaged to allow the pulling wire to be detached from the artificial implant; and
a linkage assembly acting between the first shaft and the locking member to selectively link the first shaft and the locking member.

Optionally, the linkage assembly includes two mating parts, one of the two mating parts is connected to the loading section and another one of the two mating parts is connected to the locking member, and axial movement of the loading section makes the two mating parts engage with or disengage from each other.

Optionally, the two mating parts include a linkage key and a linkage groove which are separable through axial sliding, and the linkage key and the linkage groove are rotatably linked to each other when the linkage key is inserted into the linkage groove.

Optionally, there are multiple linkage keys connected to each other through a tubular component, and the multiple linkage keys are arranged along a circumferential direction of the tubular component.

Optionally, there are multiple linkage grooves connected to each other through the tubular component, and the multiple linkage grooves are arranged along the circumferential direction of the tubular component.

Optionally, at least a part of the locking member serves as the connecting portion, and the linkage key or the linkage groove is set at a distal end of the connecting portion.

Optionally, the connecting portion is located outside the base.

Optionally, the linkage groove is defined in a pipe wall at the distal end of the connecting portion, and the linkage key is provided on an inner circumferential wall of the loading section.

Optionally, the linkage groove includes a flared configuration formed at its open side.

Optionally, the two mating parts includes a linkage key and a linkage groove that are separable through rotating, and the linkage key and the linkage groove are axially linked to each other when the linkage key is inserted into the linkage groove.

Optionally, a support member is provided on an inner circumference of the loading section and fixed to the distal end of the first shaft, and one of the two mating parts is provided on the support member.

Optionally, a limit structure is provided between the support member and the loading section to prevent axial separation of the support member and the loading section.

Optionally, the limit structure includes a positioning piece protruding radially from an outer circumference of the support member, and a positioning groove defined in an inner circumference of the loading section to cooperate with the positioning piece.

Optionally, the support member is a cylindrical structure, and an outer circumference of the support member is partially flipped to form the positioning piece.

Optionally, the support member includes an outer cylinder connected to the loading section, and an inner cylinder configured with a fitting portion and fixed to the first shaft.

Optionally, the inner cylinder and the outer cylinder are separate structures.

The present application further provides a loading structure for an artificial implant, wherein the artificial implant includes an inner frame configured with multiple arm portions on its outer circumference, a first gap is defined between the arm portions and the inner frame to accommodate native tissue, and the loading structure includes:
a first shaft, wherein the inner frame in a compressed state is mounted around an outer circumference of the first shaft; and
a loading section being tubular-shaped and fixed to the distal end of the first shaft, wherein the loading section opens towards the proximal end, at least the distal portion of the inner frame is accommodated in the loading section, a circumferential wall of the loading section is provided with a pocket hole, and at least a part of the arm portion is located in the pocket hole.

Optionally, an outflow side of each arm portion is fixedly connected to the inner frame, and an inflow side expands to form the first gap with the inner frame, and the inflow side is located at the pockethole.

Optionally, the arm portion is at least partially located within the loading section.

Optionally, the loading structure includes a catheter sheath, and the arm portion is at least partially outside the loading section and within the catheter sheath.

The present application further provides a catheter sheath based on long-distance intervention, which has a distal end and a proximal end opposite to each other, the catheter sheath includes a catheter body and a hemostatic valve, wherein the catheter body includes:
a main section wherein the hemostatic valve is connected to a proximal end of the main section, and a catheter wall of the main section at least has a structural reinforcement layer near the distal end, and the reinforcement layer adopts a metal tube with a hollow structure; and
a deformation section located at the distal end of the main section and including multiple elastic sheets arranged at intervals along a circumference of the catheter body, and proximal ends of the elastic sheets being connected to the metal tube, wherein each elastic sheet has an initial state and an expanded state, and in the expanded state, the distal ends of the elastic sheets diverge such that the deformation section assumes an overall flared configuration.

Optionally, the catheter body has a length of at least 60cm.

Optionally, the catheter body has a length in ranging of 60~90 cm.

Optionally, the distal end of the catheter body has a pre-shaped contour matching the shape of the aortic arch.

Optionally, the metal tube is made of memory alloy.

Optionally, neighboring elastic sheets are connected by a connecting member.

Optionally, two ends of the connecting member are connected to elastic sheets at corresponding sides, respectively, and joints are adjacent the distal ends of the elastic sheets.
in the initial state of the elastic sheet, a middle portion of the connecting member is folded and received in a space between neighboring elastic sheets; and
in the expanded state of the elastic sheet, the middle portion of the connecting member is unfolded.

Optionally, there are two connecting members connected between two neighboring elastic sheets, which include a first connecting member and a second connecting member arranged along the axial direction.

Optionally, the first connecting member is connected to the axial distal end of the elastic sheet, and the second connecting member is connected to the middle portion of the elastic sheet.

Optionally, the first connecting member and the second connecting member each are V-shaped, and respectively has a first opening and a second opening, wherein the first opening and the second opening are facing away from each other.

Optionally, two sides of the middle portion of the first connecting member have protrusions extending proximally, and the apex of the second connecting member is located within the two protrusions.

Optionally, the deformation section as a whole is a mesh-like structure.

Optionally, the catheter body has three layers, namely an outer membrane layer, a reinforcement layer, and an inner membrane layer, from the outside to the inside.

Optionally, the elastic sheet and the metal tube are integrally formed as one piece or formed separately.

Optionally, the hemostatic valve includes a housing and a sealing member set inside the housing for preventing fluid leakage.

The present application further provides a control handle which has opposite distal end and proximal end, as well as an axial direction extending between the proximal and distal ends, a distal end of the control handle is provided with a push mechanism capable of telescoping in an axial direction, wherein the push mechanism includes:
a telescopic assembly, wherein a distal end of the telescopic assembly has an abutment, and a proximal end of the telescopic assembly is connected to the control handle in an axially movable manner; and
a push drive mechanism mounted on the control handle and linked with the proximal end of the telescopic assembly.

Optionally, the telescopic assembly includes one level or various levels from the distal end to the proximal end, a distal end of the first level is equipped with the abutment, a proximal end of the final level is connected to the control handle in an axially movable manner and is linked with the push drive mechanism.

Optionally, the various levels of the telescopic assembly are cylindrical-shaped and sequentially inserted and connected in a movable manner.

Optionally, for two neighboring levels, one of them is an outer cylinder and the other is an inner cylinder. When the telescopic assembly is extended, the inner cylinder moves distally relative to the outer cylinder.

Optionally, an anti-retreat structure is provided between adjacent levels.

Optionally, the anti- retreat structure includes:
a rack arranged outside the inner cylinder along the telescopic direction;
an operation button swingably mounted on the outer cylinder, wherein portions of the operation button at two opposite sides of its own swing axis are configured as an operating part and an engaging part, respectively, the engaging part meshes with the rack to restrict the movement between adjacent levels, the operating part is exposed to the outer cylinder where it is located, used to disengage the engaging part from the rack; and
an elastic member acting on the operation button, driving the engaging part to maintain its engagement with the rack.

Optionally, the tooth tips of the rack are inclined towards the proximal side, and the tooth surface facing the proximal side drives the engaging part to self-lock.

Optionally, the elastic member is positioned between the inner wall of the outer cylinder and the radial outer side of the engaging part.

Optionally, the engaging part has a self-locking surface that cooperates with the rack, and the self-locking surface faces the distal side and is generally perpendicular to the axial direction of the outer cylinder.

Optionally, the push drive mechanism includes:
an external thread section located on the outer circumference of the final level; and
a first drive sleeve rotatably mounted to the control handle and including an internal thread section that engages with the external thread section.

The present application further provides a control handle for an artificial implant, including:
a support body, including two rigid bars arranged side by side, a guiding channel defined between the two rigid bars; and
a plurality of drive mechanisms, at least two of which include a transmission member that is slidably mounted in the guiding channel, and a driving sleeve that is rotatably mounted around the outer circumference of the support body and threaded with the transmission member.

Optionally, a length of the two rigid bars accounts for at least 75% of the total length of the control handle.

The present application further provides a control handle for a catheter assembly which includes a first group and a second group, wherein the control handle includes:
a first handle used to connect the first group; and
a second handle used to connect the second group;
both the first handle and the second handle include a support body, and each support body includes two rigid bars arranged side by side.

Optionally, the rigid bar is metal strip.

Optionally, a plurality of lightening holes is defined in the rigid bar.

Optionally, the rigid bar has a clamping block that engages with the first handle and/or the second handle.

Optionally, the control handle includes a mounting base fixedly connected to the rigid bar, and the driving sleeve is mounted around the support body and rotatably fitted with the mounting base.

Optionally, the driving sleeve includes an operating portion and a connecting portion embedded in the mounting base arranged along its axial direction, and the mounting base separates the operating portions of two adjacent driving sleeves from each other.

The present application further provides a control handle for a catheter assembly which includes a first group and a second group arranged in a movable manner from the outside to the inside, wherein the control handle includes:
a first handle used to connect the first group; and
a second handle used to connect the second group;
wherein the first handle is equipped with a sliding seat and a driving sleeve located around an outer circumference of the sliding seat and threadedly engaged with the sliding seat; a distal end of the second handle is equipped with a connecting sleeve, which rotates in conjunction with the sliding seat, and a rotating locking mechanism is provided between the connecting sleeve and the sliding seat.

Optionally, the rotating locking mechanism includes:
meshing teeth located at the end face of the proximal end of the sliding seat and distributed around the rotational axis of the connecting sleeve;
an unlocking member, actively installed on the connecting sleeve, having a locking position that matches with meshing teeth and an unlocking position that is radially separated from the meshing teeth; and
a driving member acting on the unlocking member, driving the unlocking member to remain in the locking position.

Optionally, the proximal part of the unlocking member is installed on the connecting sleeve in a swingable manner, and the connecting sleeve defines an operation window, at least a part of the unlocking is exposed to the operation window.

Optionally, the unlocking members are arranged in pairs along the radial direction of the connecting sleeve, and the driving member is elastically compressed between the same pair of unlocking members.

Optionally, the inner wall of the connecting sleeve is provided with a guiding groove which extends radially, and at least a part of the unlocking member moves along the guiding groove.

Optionally, the connecting sleeve is provided with a guiding step, and the unlocking member cooperates with the guide step and has a tendency to move towards the unlocking position under the radially and inwardly guiding of the guiding step.

Optionally, the meshing teeth is triangular.

Optionally, the unlocking member includes:
a pressing portion, exposed to the operation window;
a swinging shaft, located at the proximal end side of pressing portion;
a guide portion, located at the distal end side of the pressing portion and cooperating with the guiding groove; and
a locking portion, located at the distal end side of the guide portion and meshing with the meshing teeth, wherein the locking portion as a whole is conical and converges towards the distal end.

Optionally, at least a portion of the unlocking member is exposed to the connecting sleeve, and this exposed portion is exposed to the first handle or accommodated inside the first handle in accordance with the axial movement of the second handle.

The present application further provides a control handle for controlling a catheter assembly to operate an artificial implant, the catheter assembly includes multiple shafts slidably nested with each other, the control handle includes a support body, a transmission member fixed to each shaft, and a driving sleeve threaded with the transmission member. The sliding seat slidably cooperates with the support body, and the driving sleeve rotatably cooperates with the support body. Among the multiple shafts, the innermost one is the first shaft, and the proximal end of the first shaft is connected to a fourth transmission member and an actuator that slidably cooperates with the support body. The first shaft slides along the axial direction, so that the actuator at least has a first position partially hidden in the support body and a second position exposed outside the support body. The actuator can drive the first shaft to rotate.

Optionally, the actuator is a cylindrical structure.

Optionally, the actuator includes an operating section and a connecting section set at the distal end of the operating section, wherein the connecting section is connected to the transmission member.

Optionally, the connecting section has a first section that extends into the transmission member and is fixedly connected to the transmission member, as well as an expanded section connected to the proximal end of the first section. The distal end of the operating section is provided with a clamping portion that accommodates the expanded section and cooperates with it in the circumferential direction.

Optionally, a cross-section of the expanded section is non-circular.

Optionally, a side wall of the clamping portion is provided with an elastic buckle that abuts against the expanded section to prevent the connecting section from disengaging from the operating section.

Optionally, the control handle includes a base set at the proximal end of the support body, and the actuator and the base have a radial clearance fit.

Optionally, the first shaft is fixedly connected to the connecting section.

Optionally, the connecting section is threaded with the fourth transmission member, and the operating section slides axially relative to the connecting section.

Optionally, the operating section rotates about the axis to drive the connecting section to move towards the distal end, and the operating section abuts against the proximal end of the fourth transmission member to prevent it from rotation.

The present application further provides a transmission member of a control handle for connecting an interventional catheter, including a main body having an axial through-hole for extending of the interventional catheter therethrough, wherein the main body includes:
a first half having threaded transmission teeth on its outer wall;
a second half interlocked with the first half, wherein the through-hole is located between the first half and the second half; and
a fixing member surrounded by the first half and the second half, wherein the fixing member has a catheter fixing hole corresponding to the through-hole, an outer circumference of the fixing member is provided with an anti-rotation structure with at least one of the first half and the second half.

Optionally, the fixing member is a ring-shaped structure.

Optionally, the through-hole has a first radial direction and a second radial direction that are perpendicular to each other, and the body has threaded transmission teeth on two sides along the first radial direction, the first half and the second half are interlocked along the second radial direction.

Optionally, the first half and the second half are interlocked with each other through an elastic buckle.

Optionally, one of the first half and the second half is provided with a positioning slot extending along the second radial direction, and the other is provided with a positioning key that matches the positioning slot.

Optionally, the setting method of the anti-rotation structure is that: the fixing member has a non-circular outer contour, and the first half and the second half both have anti-rotation inner edges that conform to the outer contour of the fixing member.

Optionally, two opposite sides of the main body along the second radial direction are smooth planes.

The present application further provides a delivery system for an artificial implant, including:
a catheter sheath for constructing an interventional channel, a proximal end of the catheter sheath being provided with a fixed seat;
a steering assembly configured with a distal end thereof being controllable to change its orientation, the steering assembly sliding through the fixed seat and extending towards the proximal end;
an inner shaft assembly configured with a distal end thereof being provided with a loading section for connecting the artificial implant, the loading section being always exposed to the distal end of the steering assembly; and
a control handle connecting proximal ends of the steering assembly and the inner shaft assembly, the control handle being located at a proximal end side of the fixed seat, and a distance between the control handle and the fixed seat being adjustable.

Optionally, the catheter sheath has a distal end and a proximal end opposite to each other, and includes a catheter body and a hemostatic valve, wherein the catheter body includes:
a main section wherein the hemostatic valve is connected to a proximal end of the main section, and a catheter wall of the main section at least has a structural reinforcement layer near the distal end, and the reinforcement layer adopts a metal tube with a hollow structure; and
a deformation section located at the distal end of the main section and including multiple elastic sheets arranged at intervals along a circumference of the catheter body, and proximal ends of the elastic sheets being connected to the metal tube, wherein each elastic sheet has an initial state and an expanded state, and in the expanded state, the distal ends of the elastic sheets diverge such that the deformation section assumes an overall flared configuration.

Optionally, the catheter body has a length of at least 60cm.

Optionally, the catheter body has a length in ranging of 60~90cm.

Optionally, the distal end of the catheter body has a pre-shaped contour matching the shape of the aortic arch.

Optionally, the fixed seat is configured with a mounting channel which communicates with the catheter sheath, the proximal end of the fixed seat is provided with a pipe joint connected to the mounting channel, and the pipe joint is configured with:
a main nozzle equipped with a sealing member; and
a branch nozzle equipped with a one-way valve.

Optionally, the fixed seat is a hemostatic valve.

Optionally, the control handle is equipped with a steering drive mechanism. The steering assembly includes:
a steerable sheath, configured with a proximal end thereof being fixed to a control handle; and
a steering member, configured with one end thereof extending and fixed to the distal end of the steerable sheath and acting on the catheter sheath, and the other end controlled by the steering drive mechanism.

Optionally, the inner shaft assembly includes a first shaft, a second shaft, and a third shaft that are slidably nested in sequence from the inside out,
a distal end of the first shaft is linked with a locking member;
a distal end of the second shaft is connected to a pulling wire;
a distal end of the third shaft is fixed with a base, the locking member is movably mounted on the base, the base defines a locking hole that matches the locking member;
the pulling wire is wound around the artificial implant from the second shaft and then constrained to the base by the locking member, and the second shaft and the first shaft slidably match the third shaft.

The present application further provides a control method for separating an artificial implant from an interventional delivery system, including:
providing an artificial implant and an interventional delivery system for delivering the artificial implant, wherein the artificial implant includes an inner frame defining a blood flow channel, an outer circumference of the inner frame is provided with a plurality of arm portions, a first gap is defined between the arm portions and the inner frame for accommodating native tissue, leaflets for controlling the blood flow channel are connected to the inner frame, a distal end of the inner frame is held in a compressed state by a tubular-shaped loading section, and a proximal end of the inner frame is held in a compressed state by the pulling wire;
expanding at least one arm portion to align the first gap with the native tissue;
driving the loading section towards the distal end to separate the loading section from the inner frame, so as to make a distal part of the inner frame expand;
elongating a part of the pulling wire exposed out of the locking member to make a proximal part of the inner frame expand, thereby the inner frame as a whole deforming to an expected amplitude, while always maintaining control of the pulling wire to the inner frame;
moving the loading section towards the proximal end to transfer at least a part of the loading section to a proximal end side of the leaflet through the blood flow channel; and
releasing the connection between the pulling wire and the inner frame.

The present application further provides a control method for retrieval of an artificial implant using an interventional delivery system, including:
providing an artificial implant and an interventional delivery system for delivering the artificial implant, wherein the interventional delivery system includes:
a catheter sheath for constructing an interventional channel, a fixed seat being provided at a proximal end of the catheter sheath;
an inner shaft assembly configured with a loading section at its distal end, wherein at least a part of the artificial implant before expansion is accommodated in the loading section and connected to the inner shaft assembly in a releasable manner;
a control handle connected to a proximal end of the inner shaft assembly, the control handle located at a proximal end side of the fixed seat, and a distal end of the control handle provided with a push mechanism for acting on the fixed seat;
wherein the artificial implant is kept at least partly in connection with the inner shaft assembly, and during retrieval of the artificial implant, the catheter sheath is driven to move relative to the inner shaft assembly, allowing the artificial implant to be accommodated in the catheter sheath.

Optionally, the distal end of the catheter sheath is close to or in contact with the artificial implant, and drives the push mechanism to abut against the fixed seat, and further pushes the fixed seat towards the distal end until the artificial implant is accommodated in the catheter sheath.

Optionally, the artificial implant is connected to the inner shaft assembly through a pulling wire, and during retrieval of the artificial implant, the pulling wire is tightened to bring a proximal end side of the artificial implant together.

Optionally, before retrieval of the artificial implant, the artificial implant is in an expanded state and is only connected to the inner shaft assembly through a pulling wire; and the pulling wire is tightened to bring a proximal end side of the artificial implant together.

Optionally, the artificial implant includes an inner frame defining the blood flow channel, an outer circumference of the inner frame is provided with multiple arm portions, a proximal end of the arm portion is fixed to the inner frame, a first gap is defined between a distal end of the arm portion and the inner frame for accommodating native tissue, and leaflets for controlling the blood flow channel are connected to the inner frame;
during retrieval of the artificial implant, the proximal end of the arm portion first enters the catheter sheath, and the distal end of the arm portion adapts to the movement of the catheter sheath relative to the artificial implant and enters the catheter sheath.

The present application further provides a loading method for loading an artificial implant into a delivery system, including:
providing an artificial implant and a delivery system, wherein the artificial implant includes an inner frame, an outer circumference of the inner frame is provided with multiple arm portions; and the delivery system includes:
a catheter sheath with a fixed seat at its proximal end;
an inner shaft assembly movably extending through the catheter sheath, and a distal end of the inner shaft assembly being provided with a loading section; and
a control handle connected to a proximal end of the inner shaft assembly.

Optionally, a pulling wire is used for winding around a proximal end of the inner frame.

Optionally, the delivery system further includes a control mechanism, the control mechanism includes a base connected to the inner shaft assembly, a locking member movably cooperating with the base, and multiple pulling wires,
one end of the pulling wire is connected to the control handle, and another end of the pulling wire is locked to the base and the locking member after passing through and winding around the inner frame, and the multiple pulling wires are locked one by one.

Optionally, during the locking process, the locking member rotates relative to the base.

Optionally, the pulling wire is tightened to radially compress the proximal end of the inner frame.

Optionally, the multiple pulling wires are synchronously tightened.

Optionally, the distal end of the inner frame is accommodated in the loading section.

Optionally, the catheter sheath moves towards the distal end to accommodate the proximal end of the inner frame and the exposed portion of the arm portion.

Optionally, the distal end of the inner frame and at least the distal end of the arm portion are accommodated in the loading section.

Optionally, when the catheter sheath moves towards the distal end, it pushes the fixed seat towards the distal end relative to the control handle.

### BENEFICIAL EFFECT

The control mechanism of this application can cooperate with each other to achieve the control of the expansion, release, or retrieval of the artificial implant in the body, and further optimization and improvement have been made to the structure, making the control of the pulling wire smoother.

### BRIEF DESCRIPTION OF THE DRAWINGS

Fig. 1a is a schematic diagram of a control mechanism according to an embodiment of the present application for restricting an artificial implant.
Fig. 1b is a schematic diagram showing that the pulling wire is detached from the locking member of the control mechanism of Fig. 1a.
Fig. 2a is a schematic diagram of a control mechanism according to an embodiment of the present application for restricting an artificial implant.
Fig. 2b is a schematic diagram showing that the pulling wire is detached from the locking member of the control mechanism of Fig. 2a.
Fig. 3 is a schematic diagram of a control mechanism according to an embodiment of the present application being configured with three pulling wires.
Fig. 4 is a schematic diagram showing a connection between each pulling wire and the second shaft of a control mechanism according to an embodiment of the present application.
Fig. 5 is a schematic diagram of a stent of a control mechanism according to an embodiment of the present application.
Fig. 6a to Fig. 6c are schematic diagrams showing the wire control end of the artificial implant of the present application being folded gradually.
Figs. 7a and 7b are schematic diagrams showing the artificial implant of the present application during threading and folding, respectively.
Fig. 8 is a schematic diagram showing the existing control mechanism that restricts the artificial implant by pulling wire.
Fig. 9 is a schematic diagram showing that the pulling wire in Fig. 8 is detached from the base to release the restriction on the artificial implant.
Fig. 10 is a schematic diagram of a delivery system according to an embodiment of the present application.
Fig. 11 is an enlarged view of part A in Fig. 10.
Fig. 12 is a schematic diagram showing the mating state between the base, the locking member, and the pulling wire of an embodiment of the present application.
Fig. 13a is an exploded view of the locking member, the base, and the pulling wire in Fig. 12.
Fig. 13b is a partial structural view of the distal end side of the second shaft in Fig. 13a.
Fig. 14 is a left side view of the delivery system in Fig. 10.
Fig. 15 is a partial sectional view of the base taken along direction of B-B in Fig. 14.
Fig. 16a is a schematic diagram of a free end of a pulling wire according to an embodiment of the present application.
Fig. 16b is a schematic diagram of a free end of a pulling wire according to another embodiment of the present application.
Fig. 17 is a schematic diagram of a base according to an embodiment of the present application.
Figs. 18a-19 are schematic diagrams showing the pulling wire sequentially passing through the locking member of a control mechanism of the present application.
Fig. 20a is a schematic diagram of the pulling wire in the first state according to an embodiment of the present application.
Fig. 20b is a schematic diagram of the pulling wire in the second state according to an embodiment of the present application.
Figs. 21a-21c are schematic diagrams showing the pulling wires are sequentially combined with the locking member according to an embodiment.
Fig. 22 is a front view of a base according to an embodiment of the present application.
Fig. 23a is a schematic diagram of the positioning portion of the locking member according to an embodiment of the present application.
Fig. 23b is a schematic diagram of the positioning portion of the locking member according to another embodiment of the present application.
Fig. 24a is a schematic diagram showing the mating of the locking member and the base according to an embodiment of the present application.
Fig. 24b is a schematic diagram of the locking member in Fig. 24a.
Fig. 24c is a schematic diagram of the locking member in Fig. 24a after separation.
Fig. 25a is a schematic diagram showing the mating of the locking member and the base according to another embodiment of the present application.
Fig. 25b is an exploded view of Fig. 25a.
Fig. 26 is a schematic diagram of the threading of artificial implant in the prior art.
Fig. 27a shows a folding route of the artificial implant in the prior art.
Fig. 27b is a schematic diagram of the artificial implant of Fig. 11b after being folded.
Fig. 28 shows a folding route of the artificial implant of the present application.
Fig. 29 is a front view showing that the proximal end side of the artificial implant is pulled by a pulling wire of a control mechanism of an embodiment of the present application to fold radially.
Fig. 30a is a schematic diagram showing that the loading section is withdrawn and matches with the locking member of a control mechanism according to an embodiment of the present application.
Fig. 30b is a front view of the control mechanism in Fig. 30a (with the wire being removed for ease of observation).
Fig. 31 is a semi-sectional diagram of a control mechanism according to an embodiment of the present application, wherein the loading section and the locking member are matched.
Fig. 32 is a schematic diagram of the loading section of a control mechanism according to an embodiment of the present application.
Fig. 33 is an exploded diagram of the loading section in Fig. 32.
Fig. 34a is an exploded diagram of the support member in Fig. 33.
Fig. 34b is a semi-sectional diagram of the support member.
Fig. 35 is a sectional diagram of a control mechanism according to an embodiment of the present application, wherein the loading section is away from the base.
Fig. 36a is a partial schematic diagram of a control mechanism according to an embodiment of the present application when the artificial implant is loaded on the loading section.
Fig. 36b is a schematic diagram showing the radial relationship between the loading section and the arm portions of the artificial implant of a control mechanism according to another embodiment of the present application.
Fig. 36c is a schematic diagram showing the radial relationship between the loading section, the artificial implant, and the catheter sheath of a control mechanism according to an embodiment of the present application.
Fig. 37 is a schematic view of an artificial implant according to an embodiment of the present application (leaflets, skirts, and etc. are omitted).
Fig. 38 is a front view of an artificial implant according to an embodiment of the present application (leaflets, skirts, and etc. are omitted).
Fig. 39 is a schematic view of a delivery system according to an embodiment of the present application.
Fig. 40 is a schematic view of a catheter sheath according to an embodiment of the present application.
Fig. 41 is an exploded view of the pipe joint and hemostasis valve in the catheter sheath of Fig. 40.
Fig. 42 is a schematic view of the distal end of the catheter body in the catheter sheath in a collapsed state, according to an embodiment of the present application.
Fig. 43 is a schematic view of the distal end of the catheter body in Fig. 42 when it is in an expanded state.
Fig. 44 is an exploded view of the pipe joint in the catheter sheath according to an embodiment of the present application.
Fig. 45 is a schematic view of the catheter sheath in an embodiment of the present application when its distal end is in contact with an artificial implant.
Fig. 46 is a schematic view of the retrieved artificial implant in Fig. 45.
Fig. 47 is a schematic view of the control handle regarding the steering mechanism in an embodiment of the present application.
Fig. 48 is a partial sectional view of the control handle at the ratchet wheel in an embodiment of the present application.
Fig. 49 is a partial sectional view of the part where the ratchet wheel is released from restriction in Fig. 48.
Fig. 50 is a schematic view of the limit member in the control handle according to an embodiment of the present application.
Fig. 51 is an exploded view of the control handle at the steering mechanism in an embodiment of the present application.
Fig. 52a is a schematic view showing the control mechanism wrapping and restraining an artificial implant of an embodiment of the present application.
Fig. 52b is a schematic diagram showing that the catheter sheath is driven to release the arm portions in the control mechanism of an embodiment of the present application.
Figs. 52c to 52e are schematic diagrams showing that the pulling wire is released by the control mechanism to gradually expand the proximal end of the artificial implant.
Fig. 52f is a schematic diagram showing the control mechanism of the present application, in which two mating portions cooperate with each other to release the pulling wire.
Fig. 52g is a schematic diagram showing the control mechanism of the present application being withdrawn from the body.
Fig. 53 is a schematic diagram of a control handle according to an embodiment of the present application.
Fig. 54 is an exploded view of the push mechanism in Fig. 53.
Fig. 55 is a schematic diagram showing the first level of the telescope assembly in Fig. 53 extending to abut the fixed seat.
Fig. 56 is a schematic diagram showing the telescopic assembly in Fig. 55 being driven by the push drive mechanism to move towards the distal end.
Fig. 57 is a schematic diagram of the middle level of the telescopic assembly of Fig. 55.
Fig.58 is a schematic diagram of the first level of the telescopic assembly of Fig. 55.
Fig. 59 is a half sectional view of the final level of Fig. 54.
Fig. 60 is a partial half sectional view of the telescopic assembly of the control handle of an embodiment of the present application.
Fig. 61 is a semi-sectional view showing the restriction to the rack being released by pressing the operating part of Fig. 60.
Fig. 62 is a schematic view of a control handle according to an embodiment of the present application.
Fig. 63 is an exploded view of Fig. 62.
Fig. 64 is a schematic view of the support body in Fig. 63.
Fig. 65 is a schematic view showing the connection between the support body and the mounting base.
Fig. 66 is a schematic view showing the connection between the first handle, the second handle, and the support body in Fig. 62.
Fig. 67 is a partial sectional view of the second handle in Fig. 62.
Fig. 68 is an exploded view of a control handle according to another embodiment of the present application.
Fig. 69 is an exploded view of the first handle, the second handle, the first support body, and the second support body of the control handle shown in Fig. 68.
Fig. 70 is a partial sectional view of the control handle of Fig. 68 at the joint of the first handle and the second handle.
Fig. 71 is an exploded view of the first handle and the support body in Fig. 68.
Fig. 72 is a schematic view showing the connection between the first support body and the sliding seat in Fig. 71.
Fig. 73 is a structural view of one petal of the sliding seat in Fig. 72.
Fig. 74 is an exploded view of a control handle according to another embodiment of the present application.
Fig. 75a is an exploded view of the distal end of the second handle and the second support body in Fig. 74.
Fig. 75b is a schematic view of the unlocking member in Fig. 75a.
Fig. 76 is a half-sectional view showing that the unlocking member and sliding seat in Fig. 74 are meshed to prevent the second handle from rotating relative to the first handle.
Fig. 77 is a half-sectional view showing the disengagement between the unlocking member and the sliding seat in Fig. 76.
Fig. 78 is a schematic view of a component with an operation window in Fig. 75a.
Fig. 79 is a schematic view of the component with the operation window of Fig. 78 from another perspective.
Fig. 80 is a cross sectional view of Fig. 77.
Fig. 81 is a sectional view showing the distal end of the control handle at the actuator of an embodiment of the present application.
Fig. 82 is a sectional view showing the actuator of Fig. 81 extending proximally out of the second handle.
Fig. 83 is an exploded view of the fourth transmission member and the actuator according to an embodiment of the present application.
Fig. 84 is a half-sectional view of an operating section in Fig. 83.
Fig. 85 is an exploded view of a transmission member according to an embodiment of the present application.
Fig. 86 is a schematic view of a transmission member according to an embodiment of the present application.
Fig. 87 is an exploded view of a transmission member according to another embodiment of the present application.
Fig. 88 is an exploded view of the transmission member of Fig. 87, taken from another aspect.
Fig. 89 is a schematic view of a catheter sheath according to an embodiment of the present application.
Fig. 90 is a schematic view showing the catheter sheath being delivered to the aortic arch in an embodiment of the present application.
Fig. 91 is a schematic view showing the catheter sheath of Fig. 2a assisting in the retrieval of artificial implant.
Fig. 92 is a schematic view showing the distal end of the catheter sheath during catheter adjustment of an embodiment of the present application.
Fig. 93 is a schematic view of the deformation section in an initial state according to an embodiment of the present application.
Fig. 94 is a partial enlarged view of Fig. 93.
Fig. 95 is a schematic view of the deformation segment in Fig. 93 when it is in an expanded state.
Fig. 96 is a partial enlarged view of Fig. 95.
Fig. 97 is a partially enlarged view of the deformation section in the initial state according to another embodiment of the present application.
Fig. 98 is a partial enlarged view of the deformation section in Fig. 97 in an expanded state.
Fig. 99 is a sectional view of the catheter body at the metal tube in an embodiment of the present application.
Fig. 100 is a schematic view of the deformation section in an expanded state according to another embodiment of the present application.
Fig. 101 is a schematic view of the deformation section in the initial state according to another embodiment of the present application.
Fig. 102 is a partial front view of the deformation segment in Fig. 101 in the initial state.

Reference numbers in the drawings:
10, base; 101/101a, locking area; 102/102a/102b, rib; 1021, bending section; 1022, avoidance zone; 103, first cavity; 104, first opening; 105/105a/105b/105c/105d/105e/105f, locking hole; 106, extension section; 107, annular structure; 108, second opening; 11/11a/11b/11c, pulling wire; 111/111a/111b/111c, free end; 112, forward section; 113, control end; 114, return section; 115, ring; 116, middle section; 13, locking member; 131, positioning portion; 1311, tail end; 1313, head end; 135, connecting portion; 1351, connecting section; 1352, linkage key; 1353, contraction structure;
21, first shaft; 23, second shaft; 25, third shaft; 27, extension tube; 28, steering member; 211, loading section; 212, support member; 213, outer cylinder; 2131, positioning piece; 214, inner cylinder; 2141, linkage groove; 2142, flared configuration ; 215, guiding head; 216, loading portion; 217, pocket hole; 231, connecting hole; 232, notch; 233, connecting arm; 234, deformation hole;
3, control handle; 31, proximal end; 32, distal end; 310, first handle; 320, second handle;
330, support body; 331, first support body; 332, second support body; 333, rigid bar; 334, guiding channel; 335, clamping block; 336, lightening hole; 337, clamping slot;
340, transmission member; 3401, main body; 3402, first half; 3403, second half; 3404, through-hole; 3405, fixing member; 3406, transmission tooth; 3407, elastic buckle; 3408, groove; 3409, positioning slot; 3410, positioning key; 341, first transmission member; 342, second transmission member; 343, third transmission member; 344, fourth transmission member; 345, sliding seat; 3451, meshing teeth; 3452, collar; 346, unlocking member; 3462, guiding groove; 3463, pressing portion; 3464, swinging shaft; 3465, guide portion; 3466, locking portion; 347, driving member; 348, operation window; 3491, fixing hole;
350, driving sleeve; 351, first driving sleeve; 352, second driving sleeve; 353, third driving sleeve; 354, fourth driving sleeve; 355, actuator; 3551, operating section; 3552, connecting section; 3553, first section; 3554, expansion section; 3555, latching portion; 3556, elastic buckle; 356, diving ring; 3561, operating portion; 3562, connecting portion; 357, first locking ring; 358, second locking ring;
360, mounting seat; 361, first mounting seat; 362, second mounting seat; 363, third mounting seat; 364, connecting sleeve; 3641, base portion; 3642, guiding step;
37, steering drive mechanism; 371, winding wheel; 3711, ratchet wheel; 372, knob; 374, limiting member; 3741, pawl; 375, elastic member; 376, toggle button;
38, telescopic assembly; 381, first level; 382, middle level; 383, final level; 3831, external thread section; 384, abutment; 3851, blocking portion; 3852, guiding slot; 3861, expansion section; 3862, rack; 3863, elastic buckle; 3864, guiding bar; 387, operation button; 3871, operating part; 3872, engaging part; 3873, self-locking surface; 388, elastic member; 39, push drive mechanism;
4, catheter assembly; 43, steerable sheath;
501, gap opening; 502, hollow area; 504, middle; 505, distal edge; 506, protrusion; 51, catheter body; 55, hemostatic valve; 510, main section; 511, metal tube; 513, outer membrane layer; 514, inner membrane layer; 520, deformation section; 521, elastic sheet; 522, connecting member; 523, first section; 524, second section; 525, second connecting member; 526, first connecting member; 527, first opening; 528, second opening; 529, apex; 551, first connecting channel; 552, second connecting channel; 56, steering member; 581, connecting portion; 582, first opening; 583, third section; 584, fourth section; 585, second gap; 586, third gap; 587, second opening; 588, connector; 591, first deformation bar; 592, second deformation bar;
60, artificial implant; 601, wire control end; 603, eyelets; 605, connecting ear; 61, inner frame; 63, arm portion; 631, inflow side; 632, outflow side; 604, blood flow channel;
70, catheter sheath; 720, fixing base; 721, mounting channel; 750, pipe joint; 751, main nozzle; 752, sealing member; 753, branch nozzle; 754, one-way valve.

### DESCRIPTION OF THE EMBODIMENTS

The following will provide a clear and complete description of the technical solutions in the embodiments of the present application, in combination with the accompanying drawings. Obviously, the described embodiments are only a part of the embodiments of the present application, not all of them. Based on the embodiments of this application, all other embodiments obtained by those skilled in the art without creative labor are within the scope of protection of this application.

It should be noted that when a component is referred to as "connected" to another component, it may be connected to another component directly or there may be a middle component connected therebetween. When a component is considered to be "set on" another component, it may be set on another component directly or there may be a middle component set therebetween.

Unless otherwise defined, all technical and scientific terms used in this disclosure have the same meanings as those commonly understood by those skilled in the art belonging to the present application. The terms used in the disclosure of this application are only for the purpose of describing specific embodiments and are not intended to limit the scope of this application. The term "and/or" used in this disclosure includes any and all combinations of one or more related listed items.

In this application, the terms "first", "second", etc. are only used for descriptive purposes and should not be understood as indicating or implying relative importance or implying the quantity or order of the indicated technical features. Thus, the features limited to "first" and "second" may explicitly or implicitly include one or more of these features. In the description of this application, the meaning of "multiple/a plurality of" refers to at least two, such as two, three, etc., unless otherwise specified.

This disclosure describes an artificial implant and a delivery system for delivering the artificial implant into a human body. The delivery system includes a control handle and a catheter assembly, wherein the control handle may be connected to and control the catheter assembly which is used for performing interventional surgery. The catheter assembly includes a plurality of controlled components, and distal ends of the controlled components cooperate with each other to operate the artificial implant, such as releasing, retrieval of, locking position, adjusting spatial posture, etc. Each controlled component may be configured as a hollow tube, a solid rod, a flexible wire, or a combination of various forms. There are multiple controlled components, and at least two of them (taking the proximal end as an example) can slide relative to each other along the axial direction or rotate relative to each other about the axis. The actuators on the control handle (i.e., the parts that the user directly operates and contacts), which are used to operate the controlled components, can be connected to the corresponding controlled component directly and fixedly for transmission, or, threads and rack-and-pinion may be used for transmission therebetween.

In the following description, many improvements regarding control handles or local structures can be implemented on the same control handle without obvious technical contradictions, but are not strictly limited to being implemented on the same control handle. For different numbers of controlled components and motion characteristics, or for control handles with further simplified structures, the embodiments may be implemented separately or combined appropriately.

The application site and structure of artificial implants are not strictly limited. Taking artificial heart valves as examples in some drawings or texts, the artificial heart valve generally includes a deformable stent and leaflets connected to the stent. The stent in whole is cylindrical-shaped, with a sidewall thereof being configured as a hollow mesh structure. Unless otherwise specified, the shape or size of the mesh structure is not strictly limited. An interior of the stent is configured as a blood flow channel, and multiple leaflets cooperate with each other to control the degree of opening and closing of the blood flow channel inside the stent. **In** order to locate within the body, positioning structures that can interact with surrounding native tissues, such as anchor spikes, arms, etc., may be provided around the stent. To prevent leakage, skirts or anti-leakage materials may be provided on the interior and/or exterior of the stent.

According to different expansion modes, the stent is processed using corresponding materials, such as nickel titanium alloy with shape memory and self-expanding ability, or stainless steel material for balloon expansion. The stent itself may be formed by cutting pipes or weaving wires, and the leaflets may be connected to the stent by sewing, bonding, or integral molding.

Taking the self-expanding stent as an example, its expansion and retrieval may be controlled by a sheath catheter wrapped around the outer circumference of the stent. According to the different parts of the stent exposed to the sheath catheter, corresponding control may be carried out. The stent may also be controlled by a pulling wire, which passes through the structural gap (or wire hole) of the stent. By adjusting the tension of the pulling wire through the control handle, the degree of expansion of the stent can be changed. When the pulling wire is pulled away from the stent, the stent is allowed to be completely released. Of course, the control of the pulling wire is also achieved through various controlled components in the catheter assembly.

The stent of artificial implant generally has a connection structure thast matches with the catheter assembly to limit positions relative to each other and prevent unnecessary positional deviation during delivery. The artificial implant is in a radially compressed state when delivery, that is, in a loaded state. After releasing the constraint of the catheter assembly and expanding radially in the human body, the stent is in an expanded state. Unless otherwise specified, the shape of the artificial implant is understood as the shape in the expanded state, without considering the local deformation caused by surrounding tissue pressure.

Due to the complexity of the internal of the human body, the catheter assembly often requires steering operations. Corresponding steering components may be configured as a tubes or a wire, with a distal end thereof acting on the component to be bended and a proximal end thereof operatively coupled to a control handle to adjust a bending amplitude and/or a bending direction.

When used to indicate direction, the proximal end in the disclosure generally refers to the side adjacent to the operator (such as a doctor), and the distal end is the side relatively far away from the operator. Along the intervention path, each component has its own distal and proximal ends that are opposite to each other. In theory, when the catheter assembly and control handle are completely straightened, a straight line between the proximal end and the distal end determines the axial direction, and correspondingly determines the radial direction which is perpendicular to the axial direction and the circumferential direction which is around the axial direction. When used to refer to a structure, the term "end" in the disclosure refers to the endpoint, or a point or region in this direction, or a specific structure connected to that point or region, of the structure.

The present application provides a control mechanism for an artificial implant, including a base 10, a pulling wire 11, and a locking member 13, wherein the base 10 defines a locking hole therein, and the pulling wire 11 includes a free end 111 that can pass through and wind around, or be detached from the artificial implant 60. The locking member 13 in whole is located at the distal end of the base 10 and rotatably engaged with the base 10 to lock the free end 111 of the pulling wire 11. The base 10 includes a distal end 32 and a proximal end 31 that are opposite to each other, as well as an axial direction extending between the distal end 32 and the proximal end 31. Unless otherwise specified, the proximal end, distal end, and axial direction are also applicable to other components of the control mechanism, as well as the control handle and the delivery system of the following embodiments.

The free end 111 is the end where the pulling wire 11 first passes through and finally detaches from the artificial implant 60, and may also be understood as the farthest end when the pulling wire 11 is straightened. The other end of the pulling wire 11 opposite to the free end 111 is the control end, which may be fixed to the base 10 directly or extend towards the proximal end for controllability.

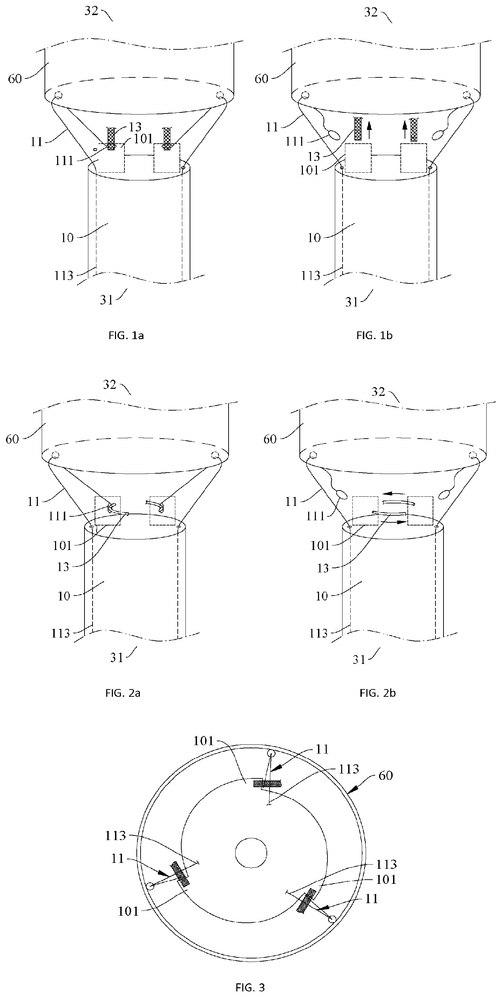

The free end 111 has a first state restricted to the base 10 (as shown in Fig. 1a) and a second state released from restriction (as shown in Fig. 1b). The locking member 13 is in active cooperation with the base 10, and the locking member 13 is in cooperation with the free end 111 of the pulling wire 11 to change the state of the free end 111. In the first state, the free end 111 is combined with the locking member 13, and the locking member 13 cooperates with the base 10 to prevent the pulling wire 11 from disengaging from the locking member 13. At this time, the free end 111 may be understood as being fixed to the locking member 13. In this state, the pulling wire 11 is always connected to the artificial implant 60, and the expansion process (i.e. expansion degree) of the artificial implant is adjusted based on the length of the pulling wire 11 exposed out of the control mechanism, as well as the expansion/folding speed of the artificial implant is controlled based on the rate of change of the pulling wire. When necessary, the pulling wire 11 may also be used for the retrieval of the artificial implant.

In the second state, the free end 111 is disengaged from the locking member 13. At this time, the free end 111 may be understood as being released, thereby releasing the interconnection between the artificial implant and the control mechanism. Subsequently, the control mechanism as a whole can be withdrawn out of the human body, leaving the artificial implant in the predetermined position inside the human body.

When the locking member, the base, and the pulling wire are pre-assembled out of the body, the extension path of the pulling wire 11 enters the proximal end of the base, and then extends out from the distal end of the base, and passes through and winds around the artificial implant before being combined with the locking member 13.

After releasing and detaching from the artificial implant, the control end is driven to move the pulling wire 11 towards the proximal end to withdrawal the pulling wire. During retrieval of the artificial implant, the pulling wire 11 is in the first state, and the control end 113 is operated to reduce the length of the pulling wire 11exposed out of the control mechanism.

The locking member 13 is located at the distal end of the base 10, which is conducive to observation and operation during pre-assembly outside the body, thereby facilitating assembly. Especially, the locking member 13 and the base 10 are not nested radially, so as to form sufficient radial space for extending of the pulling wire and make the pulling wire move smoother. In addition, the maximum radial size of the base 10 and/or the locking member 13 can be correspondingly reduced, facilitating interventional delivery in the body.

Of course, the control mechanism of this application, as well as other embodiments including the control mechanism of this embodiment, are also applicable to simulation training for interventional delivery operations performed outside the body.

The control end 113 of the pulling wire 11 can extend to and be controlled by the control handle, and its movement, which specifically may be axial movement, rotation, or winding, can change the length of the pulling wire 11 exposed out of the base 10.

Among them, the artificial implant 60 is a cylindrical structure with corresponding circumferential direction. There are multiple pulling wires 11. The control ends 113 of the pulling wires 11 can be controlled independently or move synchronously. The positions where the pulling wires 11 act on the artificial implant 60 are arranged at intervals along the circumferential direction of the artificial implant 60, which can improve the synchronization of the contraction and expansion of the artificial implant 60. For example, as shown in Fig. 3, there are three pulling wires 11, and there are three positions where the pulling wires 11 act on the artificial implant 60 arranged at intervals along the circumferential direction of the artificial implant.

Correspondingly, there are multiple locking areas 101. In the first state, the free end 111 of each pulling wire 11 corresponds to one of the multiple locking areas 101, thereby avoiding interference between the pulling wires 11. The multiple locking areas 101 may be separated through the structure of the base, or additional separation components may be provided on the base to separate the multiple locking areas 101.

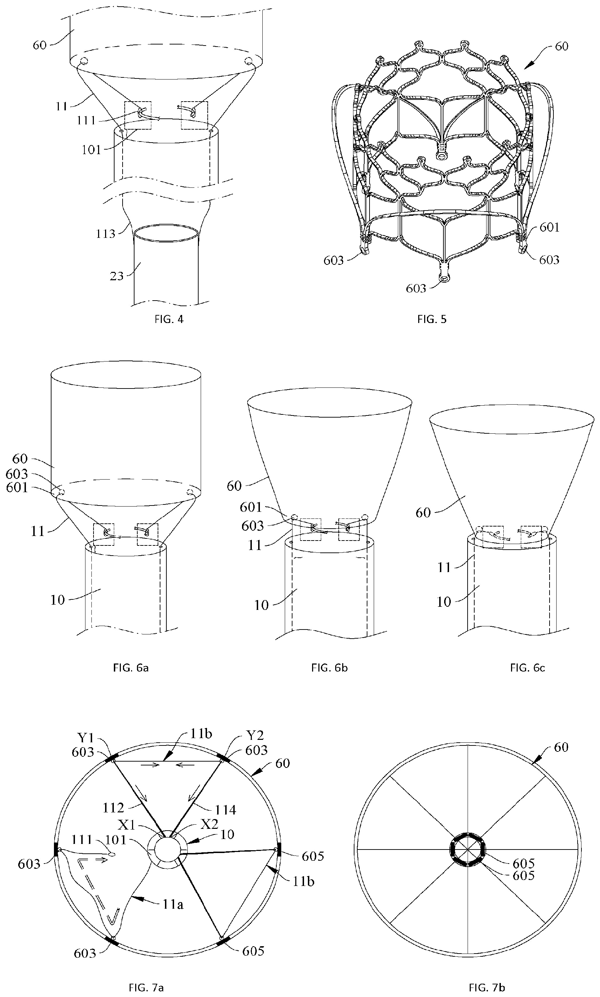

The control end 113 of each pulling wire 11 may extend to the control handle directly, or an intermediate component may be adopted to realize indirect transmission, so as to reduce the risk of the multiple pulling wires 11 intertwining with each other. For example, as shown in Fig. 4, the control mechanism further includes a second shaft 23, and the control end 113 of each pulling wire 11 is connected to the second shaft 23. By operating the second shaft 23, the pulling wires 11 are synchronously controlled.

As shown in Fig. 5, the artificial implant 60 has an axial direction, and one end of the artificial implant 60 in the axial direction is configured as a wire control end 601, which may be either the distal end or proximal end of the artificial implant 60. In this embodiment, the wire control end 601 is located at the proximal end of the artificial implant, and has eyelets 603 for extending of the pulling wires (not shown in the drawings for clarity) therethrough.

The eyelets 603 may be formed, for example, by means of:
the artificial implant including a hollow portion that form the eyelets, wherein the hollow portion is a kind of structural gaps in the artificial implant; or,
drilling holes directly into the artificial implant to form the eyelets (as shown in Fig. 5); or,
component with eyelets being configured independently relative to the artificial implant (such as connected to the artificial implant by welding, etc.).

Among them, there are multiple eyelets 603 spaced from each other. In the expanded state, the same pulling wire 11 passes through at least two of the multiple eyelets 603.

In the first state, the free end 111 of the pulling wire can limit the complete separation of the artificial implant 60 from the control mechanism, but the artificial implant can be allowed to deform to a certain extent by adjusting the length of the pulling wire exposed out of the base, which may be understood as changing the position of the artificial implant 60 relative to the base. Therefore, when the free end 111 of the pulling wire is maintained in the first state, the artificial implant 60 may be configurable between the following states based on the degree of its deformation:
an expanded state, wherein the wire control end 601 expands radially away from the base 10 (as shown in Fig. 6a), and the round-trip path of the same pulling wire 11 winding around the artificial implant 60 does not coincide;
a collapsed state, wherein the wire control end 601 is radially folded close to the base 10 (as shown in Fig. 6c); and
an intermediate state, wherein the wire control terminal 601 is in a state between the expanded state and the collapsed state (as shown in Fig. 6b).

Among them, as shown in Fig. 5 to Fig. 7a, the multiple eyelets 603 are arranged at intervals along the circumferential direction. In the expanded state, the same pulling wire 11 passes through at least two eyelets 603, that is, one pulling wire 11 can act on two circumferential positions of the wire control end 601. In a preferred embodiment, the number of the eyelets 603 is twice the number of the pulling wires 11, and each pulling wire 11 corresponds to two eyelets 603. This enables synchronous withdrawal and expansion of the wire control end 601 of the artificial implant 60 in the circumferential direction, reduces the number of the pulling wires, avoids entanglement and friction at the proximal ends of the pulling wires, and correspondingly reduces the space for accommodating the pulling wires.

For the artificial implant 60 as a whole, at least two pulling wires should be configured. In addition, for the same eyelet, only one pulling wire is allowed to pass, so as avoid interference caused by reciprocating threading.

As shown in Fig. 7a, in this state, the extension direction (radial direction) of the pulling wire between the artificial implant 60 and the base 10, i.e., the direction of traction force, is perpendicular to the moving direction (circumferential direction) for unlocking the locking member, which is more convenient for unlocking.

For example, as shown in Fig. 7a and Fig. 7b, the round-trip path of the same pulling wire 11 around the artificial implant generally encloses a triangular area, that is, the round-trip paths do not repeated. Otherwise, if the round-trip path is repeated, the extension path of the pulling wire 11 will be a single straight line or curve line, which will not enclose a certain area. The relationship between the extension path of the pulling wire 11 and the base 10 and locking member 13 will be described in the following embodiments.

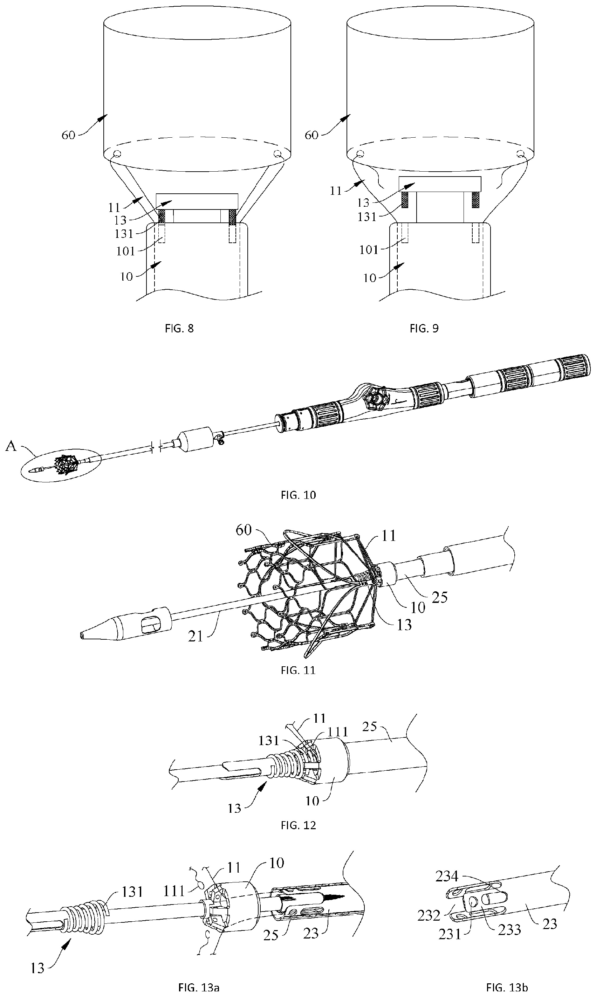

As shown in Fig. 10 to Fig. 15, the base 10 includes locking areas 101, and the locking member 13 includes a positioning portion 131 that enters or exits the locking areas 101 during its rotation. The positioning portion 131 cooperates with the free end 111 of the pulling wire 11 to restrict the artificial implant 60. The interior of the base 10 defines a first cavity 103, the proximal end side of the base 10 defines a first opening 104 communicating with the first cavity 103, and the outer circumferential surface of the base 10 defines a second opening 108 communicating with the first cavity 103. The pulling wire 11 passes through the first cavity 103. One end (i.e., the control end) of the pulling wire 11 extends through the first opening 104 towards the proximal end and out of the base 10. The other end of the pulling wire 11 (i.e., the free end 111) extends out of the base 10 through the second opening 108 to connect the artificial implant 60.

The pulling wire 11 passes through the first cavity 103 of the base 10, reducing the occupation of radial space. Moreover, the locking member 13 is not inside the first cavity 103, so that the movement of the pulling wire 11 will not be interfered by the locking member 13, making the movement of the pulling wire 11 smoother, and facilitating the expansion control of the artificial implant.

The locking area 101 may be located within the overall outer contour of the base 10, so as to accommodate the free end in the first state. In addition, the locking area 101 may be opened towards the distal end to optimize the extension path of the pulling wire 11. For example, the locking area 101 is located at the second opening.

In conventional techniques, such as shown in Fig. 8a and Fig. 8b, it is shown that the locking member 13 slides axially to make the positioning portion 131 disengage from the locking area 101, thereby allowing the pulling wire 11 to detach from the artificial implant. In this embodiment, the positioning portion 131 serves as a part of the locking member 13, and there is displacement at least in the circumferential direction during operation, which can reduce or even eliminate the axial stroke variation of the locking member 13, reduce the axial space occupation of related components and even the proximal control handle, being more conducive to the configuration of the transmission mechanism.

In the first state, the pulling wire 11 is constrained by the artificial implant 60 and mainly undergoes radial movement. In the second state, the free end of the pulling wire 11 is free. Among them, the range of the second opening 108 includes the radial side wall and distal end wall of the base 10, which is conducive to the movement of the pulling wire 11 in all directions in the first state, as well as the quick and smooth withdrawal in the second state.

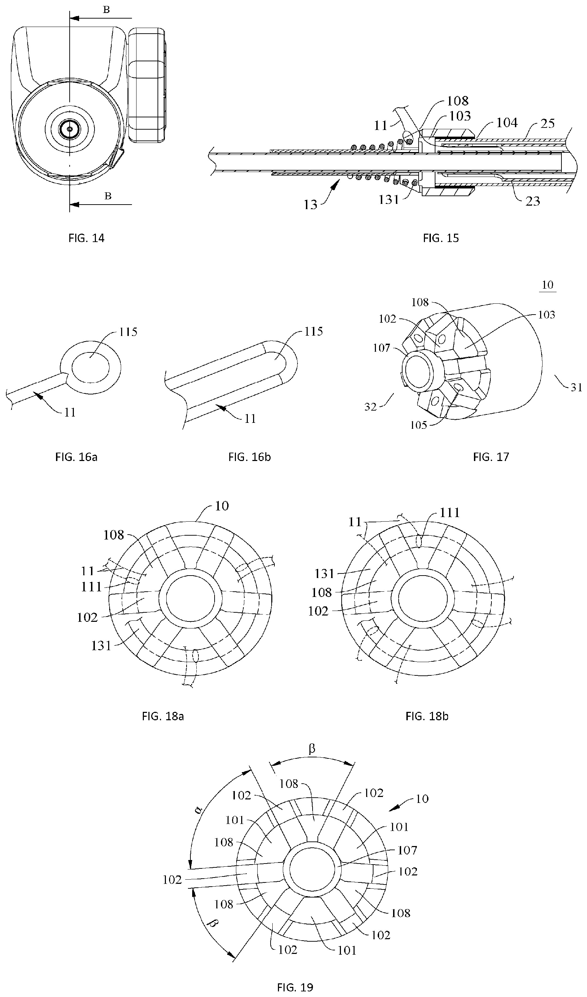

Regarding the cooperation between the free end 111 and the positioning portion 131, in one embodiment, the free end 111 includes a ring 115. In the first state of the free end 111, the positioning portion 131 extends into the ring 115; and in the second state of the free end 111, the positioning portion 131 exits the ring 115. The ring 115 can be independently configured (as shown in Fig. 16a) or formed by winding the pulling wire 11 (as shown in Fig. 16b). The pulling wire 11 may be single strand or multiple strands. For example, the pulling wire 11 may be single strand extending to the control end 113, or, may be double strand extending in parallel to the control end 113, or, may be double strand extending in parallel for a period of time and then merging and extending to the control end 113.

Along the circumferential direction of the base 10, there are multiple locking areas 101 arranged at intervals, and the positioning portion 131 engages into or disengages from the locking areas 101 in sequence along with the movement of the locking member 13.

In one embodiment, as shown in Fig. 12 and Fig. 13a, the control mechanism further includes a third shaft 25 fixedly connected to the base 10. The proximal end of the third shaft 25 extends to and can be controlled by the control handle. The third shaft 25 can keep the base 10 relatively fixed in the circumferential direction, allowing the positioning portion 131 to rotate relative to the base 10. In the radial direction, the third shaft 25 is located around the second shaft 23. The third shaft 25 and the second shaft 23 are both pipe fittings, wherein the distal end of the second shaft 23 is configured with connecting holes 231 for winding and connecting the control ends of the pulling wires. The connecting holes 231 correspond one-to-one to the pulling wires, including the mutual correspondence in quantity and position. In the drawings, there are three connecting holes 231 arranged at intervals along the circumferential direction.

As shown in Fig. 13a and Fig. 13b, in one embodiment, multiple notches 232 are defined in a pipe wall of the distal end the second shaft 23 and arranged at intervals, and a connecting arm 233 is formed between two neighboring notches 232. The connecting holes 231 are defined at the distal ends of the connecting arms 233. During the movement of the second shaft 23 towards the distal end or the proximal end, the connecting arms 233 adapt to the movement of the pulling wires and deform, including at least radial deformation. In one embodiment, a deformation hole 234 is defined in a portion of the connecting arm 233 which is located at the proximal end side of the connecting hole 231.

As shown in Fig. 17 to Fig. 18b, in one embodiment, the base 10 is configured with multiple separating members along the circumferential direction, and two adjacent separating members define the second opening 108. Therefore, there are multiple second openings 108 arranged at intervals along the circumferential direction of the base 10. The separating members are configured as the ribs 102 shown in the drawings. The strip-shaped structure minimizes its own circumferential span as much as possible, maximizing the circumferential span of the second opening and facilitating the extending of the pulling wires. For threading, for example, one pulling wire extends through a locking area and then returns to this locking area; or, one pulling wire extends through a locking area and then returns to an adjacent locking area, this will allow two action points to be sufficiently close. At the same time, the ribs 102 also serve as reinforcing ribs to maintain the basic strength requirements of the base.

In the first state, the free end 111 extends out of the base 10 through the corresponding second opening 108, winds around the artificial implant, and returns to the locking area corresponding to the same second opening 108 (as shown in Fig. 18a), or returns to a position adjacent to the second opening 108 in the circumferential direction (as shown in Fig. 18b).

As shown in Fig. 19, the position adjacent to the second opening 108 in the circumferential direction may be another neighboring locking area 101, or a channel separately configured for extending of the pulling wire. The formation of this channel can be based the same principle as the locking area in structure. For example, there are six second openings 108, wherein three of them spaced apart from each other serve as the channels for the free ends of the pulling wires to extend out of the base 10, and the other three of them spaced apart from each other serve as the locking areas 101. For example, as shown in Fig. 19, in the circumferential direction, the span of the locking area 101 is α, and the span of the channel neighboring to the locking area 101 is β, wherein α/β = 1.1~2.

As shown in Fig. 17, all of the ribs 102 are converged and fixed to each other at the distal end of the base 10 to form an annular structure 107. All of the ribs 102 are continuously distributed in the circumferential direction at the proximal end 32 of the base 10 to form a cylindrical structure. An inner cavity of the cylindrical structure is part of the first cavity 103. An inner hole of the annular structure 107 can be used for other pipe fittings, such as the first shaft described below, to pass through.

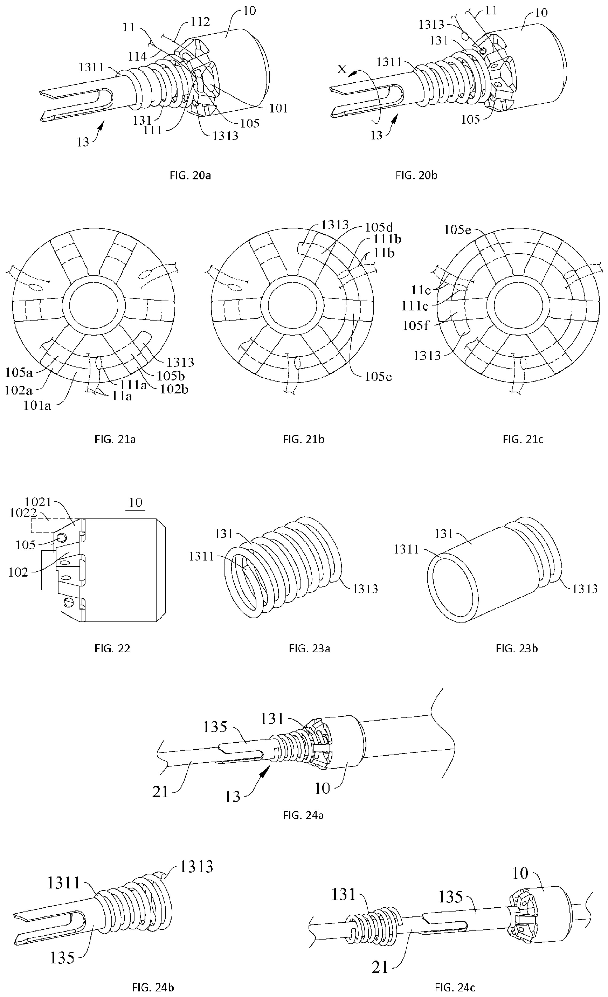

**In** one embodiment, as shown in Fig. 20a and Fig. 20b, the base 10 defines a locking hole 105 for inserting the positioning portion 131 therein. The positioning portion 131 includes:
a locking state, wherein the positioning portion 131 is inserted into the locking hole 105, restricting the free end **111** of the pulling wire 11 from disengaging (as shown in Fig. 20a); and
an unlocking state, wherein the positioning portion 131 exits the locking hole 105, allowing the free end **111** of the pulling wire **11** to disengage (as shown in Fig. 20b).

As shown in Fig. 21a, the locking area 101a is between two ribs 102a, 102b. In the first state, the free end 111 of the pulling wire 11 is located in the current locking area 101. Locking holes 105a, 105b are correspondingly defined in opposite sides of the two ribs102a, 102b. Of course, the locking holes may extend through the ribs where they are located. One section of the positioning portion 131 is located in the locking area 101a, and two ends of this section are inserted into the locking holes 105a, 105b, respectively.

Combined with the moving direction of the locking member 13, an open side of the locking hole 105 is oriented along the circumferential direction of base 10.

When the positioning portion 131 moves in a first direction (as indicated by X in Fig. 20b), the free end 111 switches from the second state to the first state. The locking hole 105 extends through the rib 102 along the first direction, and has forward and backward openings opposite to each other, wherein the forward opening faces the first direction.

In terms of quantity, there are multiple ribs 102 configured with the locking holes 105. The positioning portion 131 as a whole has a tail end 1311 and a head end 1313 opposite to each other. The head end 1313 is inserted into or exits from the locking holes 105 in sequence along with the rotation of the locking member 13 (as shown in Fig. 20a). Taking the insertion of the positioning portion in sequence as an example, as shown in Fig. 21a to Fig. 21c, the head end 1313 sequentially engages into the locking hole 105a, the free end 111a, the locking hole 105b, the locking hole 105c, the free end 111b, the locking hole 105d, the locking hole 105e, the free end 111c, and the locking hole 105f. The order of disengagement is opposite to the order of engagement. The advantage of sequential insertion is reflected in the sequential engagement between the positioning portion and the pulling wire during external pre-assembly, which facilitates assembly.

In a preferred embodiment, all of the ribs 102 are provided with locking holes 105 for the positioning portion 131 to sequentially rotate through and provide certain motion guidance. During the process of rotating and inserting the locking member into the locking holes, the head end 1313 finally abuts against the side wall of one of the ribs 102 or is inserted into the corresponding locking hole, playing a limiting role.

When the positioning portion 131 is configured with a helical shape in multiple turns, the positions of the locking holes 105 on the ribs 102 are adapted to the positioning portion 131. Among them, there are one or multiple locking holes 105 on the same rib 102, wherein the multiple locking holes 105 are arranged in sequence along the extension direction of the rib 102 where they are located.

Referring to Fig. 22, in one embodiment, one section of the rib 102 along the axial direction is a bending section 1021 extending radially and inwardly, and the locking hole 105 is located in the bending section 1021 of the corresponding rib 102. All of the bending sections 1021 as a whole form an avoidance zone 1022 (as shown by the dashed area in the drawings), and the proximal end of the artificial implant in the collapsed state is located within the avoidance zone 1022. It is convenient to guide the loading section described below to be partially placed in the avoidance zone 1022 and aligned with the outer circumference of the base 10 when retracting.

Based on the rotation characteristics of the locking member and the circumferential distribution of the pulling wires, the positioning portion 131 has a curved shape and the curved extension path is around the base. The curved configuration can fully utilize the circumferential space to maintain the necessary length of the locking member, realizing the control of the free ends of all pulling wires through one locking member. It also eliminates the axial dimension changes of the control mechanism, retains the original function of the locking member fitting with the pulling wires one by one, facilitating the assembly of the locking member, the pulling wire, and the base during the assembly process.

Specifically, the open side of the locking hole faces the circumferential direction of the base, and the positioning portion is rod-shaped. The rod-shaped positioning portion may be a straight rod or a curved rod, with a quantity of one and cooperating with the free ends of all of the pulling wires. The positioning portion is engaged into or disengaged from the locking hole along with the rotation of the locking member. For example, as shown in Fig. 23a and Fig. 23b, the positioning portion 131 is configured as a helical structure, which is wound at least once. For example, the helical structure is a multi-turns structure, with the multiple turns arranged along the axial direction (each turn extends around the base). The generatrix of the helical structure may be a diagonal line (adjacent to or away from the axis of the base) or a straight line, for example, the helical extension path is a cylindrical helical or at least partially a conical helical.

As shown in Fig. 24a to Fig. 24c, the locking member 13 includes a connecting portion 135 located at the distal end of the base 10, and the control mechanism further includes a first shaft 21 that cooperates with the connecting portion 135 in a transmission way, so as to drive the locking member 13 to rotate relative to the base 10. The proximal end of the first shaft 21 is connected to and controlled by the control handle.

Among them, along the winding direction of the helical structure, the positioning portion 131 as a whole has a tail end 1311 and a head end 1313 that are opposite to each other, with one end being fixed to the connecting portion 135 and the other end being free. The connection manner between the positioning portion 131 and the connecting portion 135 includes separately forming and then connecting by, for example, welding or assembly, or integrally forming as one piece.

In this embodiment, the head end 1313 of the positioning portion 131 cooperates with the base 10 and the pulling wire. A part of the positioning portion 131 near the distal end 1311 is fixed to the connecting portion 135, for example, to the outer circumference or distal end of the connecting portion 135. The connection manner may be direct welding of the ends, or welding and fixing after partial overlapping. The connecting portion 135 is tubular-shaped, and its interior is used for extending of other components, such as the first shaft 21 therethrough. In order to improve the connection strength and avoid interference with the extension of the components, the positioning portion 131 may be spirally wound around the outer circumference of the connecting portion 135.

In one embodiment, a positioning mechanism is provided between the connecting portion 135 and the base 10 for maintaining axial positions of the connecting portion 135 and the base 10 during pre-assembling. The connecting portion 135 may be tubular-shaped with constant diameter. The positioning mechanism includes a first end surface located at the proximal end of the connecting portion and a second end surface located at the distal end of the base, wherein the second end surface abuts against the first end surface. During pre-assembly, the first end surface abuts against the second end surface, and the positioning portion is in place with the locking hole of the base.

Among them, the head end 1313 of the positioning portion 131 needs to adapt to the radial position of the locking hole, and a part of the head end 1313 near the tail end 1311 needs to be connected to the connecting portion 135 fixedly. The outer diameter of the connecting portion has a significant deviation from the radial position of the locking hole, so that the helical extension path of the positioning portion 131 is a conical helical.

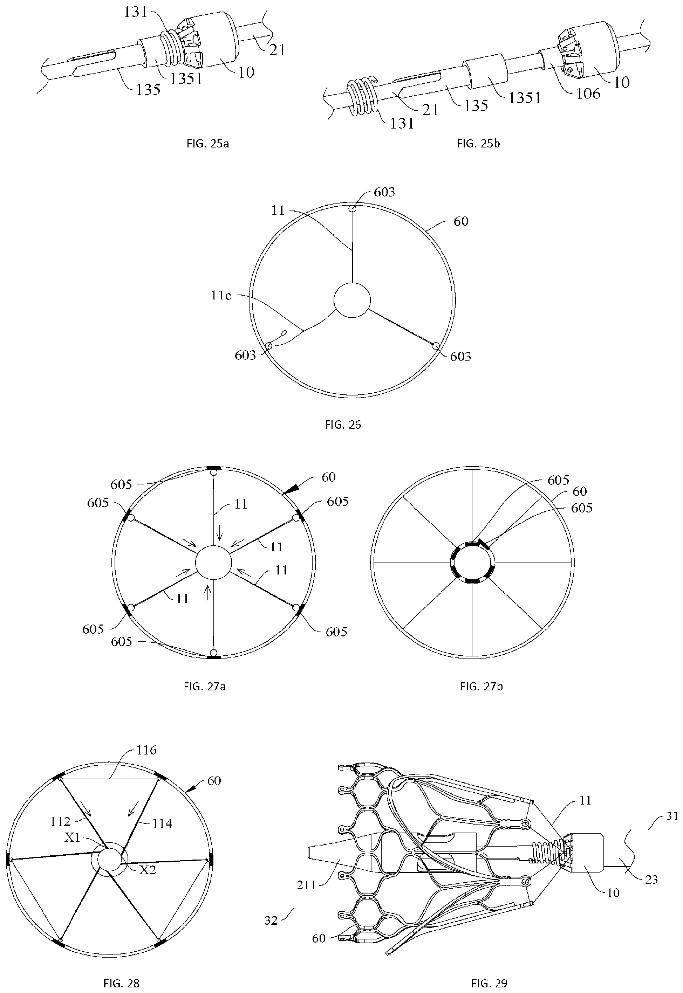

Alternatively, as shown in Figs. 25a and Fig. 25b, the proximal end of the connecting portion 135 expands radially and outwardly to form a connecting section 1351, and the distal end of the base 10 has an extension section 106, wherein the connecting section 1351 and the extension section 106 are nested with each other. In the drawings, the connecting section 1351 is a cylindrical structure and mounted around the extension section 106. The connecting section 1351 compensates for the aforementioned deviation, so that the helical extension path of the positioning portion 131 is a cylindrical helical. At this time, a bottom wall of the connecting section 1351 serves as the first end surface.

The connecting portion 135 is movably mounted around the distal end of the first shaft 21, and the connecting portion 135 can be axially separated from the distal end of the first shaft 21. When the connecting portion 135 and the first shaft 21 are matched, the connecting portion 135 and the first shaft 21 can be synchronized in the circumferential direction, and at least the proximal end of the first shaft 21 can be controlled to drive the locking member 13 to rotate. As for axial separation, it means that the connecting portion 135 and the first shaft 21 can be separated from each other to release their cooperation or combined with each other to maintain synchronization, and the specific cooperation relationship between the connecting portion 135 and the first shaft 21 can be switched according to operational requirements.

As shown in Fig. 28, regarding the extension path of the pulling wire 11, in the first state, the pulling wire 11 has a forward section 112, a return section 114, and a middle section 116 between the forward section 112 and the return section 114, cooperatively forming the triangular-shaped round-trip path in the above embodiment. The free end 111 is set at the distal end of the return section 114. In one embodiment, the positioning portion is a helical structure with multiple turns, and there is an axial gap between adjacent turns. The forward section can pass through the axial gap at the proximal end of the positioning portion and interact with it to form a first action point (X1), and the return section is connected to the positioning portion and interacts with it to form a second action point (X2). The first and second action points are close to each other, making different positions of the artificial implant move more synchronized during retrieval and expansion.

In another embodiment, as shown in Fig. 20a, the forward section 112 extends out through the locking area 101 (without through the axial gap) directly and interacts with it to form the first action point (X1), and the return section 114 is connected to the positioning portion 131 and interacts with it to form the second action point (X2). Based on the above, the space of the locking area 101 is larger than the axial gap, which facilitates the extending of the pulling wire. During pre-assembly, the pulling wire can extend through the base first, and then the locking member can be rotatably engaged with the base, and at the same time the free end can be inserted for easy assembly.

The pulling wire 11b is in a state after threading, and the bolded parts are the forward section 112 and the return section 114 of the pulling wire 11b.

As shown in Fig. 7a, the pulling wire 11b forms two action points (X1, X2) in the locking area 101 or the positioning portion 131, as well as two action points (Y1, Y2) between it and the artificial implant.

Here is a description based on existing technology: as shown in Fig. 26, there are three eyelets 603 arranged at intervals in the circumferential direction of the artificial implant 60, and correspondingly, there are three pulling wires. The specific extension path refers to pulling wire 11c, and its round-trip path is repeated. During the folding process, the force generated by the pulling wire is only in the radial direction, while the artificial implant will generate corresponding stress in the circumferential direction and react on the pulling wire, gradually increasing the force required to be applied to the pulling wire, affecting the operating feel.

However, in this embodiment, the pulling wire is configured as a triangle, which not only generates a radial force, but also generates a force along the connecting line of the two action points (Y1, Y2) (as shown by the solid arrows in Fig. 7a) to directly overcome the stress changes during the deformation process of the artificial implant. The overall force change of the pulling wire is smaller than that of the existing wire, and the operation feel is more delicate.

In conventional technology, the spacing between adjacent two eyelets 603 is relatively wide (i.e., the circumferential span is large). The eyelets 603 are opened on the connecting ears 605 (as shown in Fig. 27a), and neighboring connecting ears 605 are independently connected to the pulling wires. During the folding process, the neighboring connecting ears 605 may eventually be misaligned and stacked with each other (as shown in the bolded part of Fig. 27a), which affects subsequent expansion. However, in this embodiment, the extension path of the pulling wire makes the connecting ear 605 and the structural gap be mostly or completely controlled by the pulling wires, reducing or even eliminating the problem of misalignment and stacking during folding (as shown in Fig. 7b).

The two action points (X1, X2) are in the same locking area and adjacent to each other, and the round-trip path of the pulling wire roughly forms an equilateral triangle, that is, the lengths of the bolded forward section 112 and return section 114 in Fig. 7a are equal, and correspondingly the movement speeds of the forward section 112 and the return section 114 are the same, which improves the synchronization of the folding or expansion of the artificial plant 60. As shown in Fig. 28, when the two action points (X1, X2) have a large circumferential span, correspondingly the lengths of the forward section 112 and the return section 114 are different, and correspondingly the movement speeds of the forward section 112 and the return section 114 are different, resulting in abnormal folding/expansion of the artificial implant.

In the existing technology, in order to improve the synchronization of folding and expansion of the artificial implant, a larger number of pulling wires (such as six or more pulling wires) are needed. However, it is necessary to consider whether the spatial arrangement of each pulling wire at the proximal end is allowed, as there may be friction or entanglement between the pulling wires that are too close. The threading method of this embodiment reduces the required number of pulling wires, thereby meeting the space requirements.

The above control mechanism can be installed in a delivery system, which further includes a control handle located at its proximal end and an inner shaft assembly connected between the control handle and the control mechanism, wherein the inner shaft assembly includes the first shaft, the second shaft, and the third shaft described above.

In some embodiments, the delivery system further includes an outer sheath mounted around the inner shaft assembly. During the interventional delivery process, the distal end of the outer sheath surrounds the control mechanism and the artificial implant partially or entirely.

Referring to Fig. 11, Fig. 29, Fig. 30a and Fig. 30b, the present application also provides a locking mechanism for connecting an artificial implant to a delivery system, including a first shaft 21, a pulling wire 11, a third shaft 25, and a linkage assembly. The distal end of the first shaft 21 is fixed with a loading section 211, which is open towards the proximal end for accommodating the distal portion of the artificial implant 60. The pulling wire 11 includes a free end 111, which can pass through and wind around, or be detached from the artificial implant 60. The third shaft 25 is slidably mounted around the first shaft 21, and a locking assembly is provided on the third shaft 25. The artificial implant 60 is bound to the locking assembly by the pulling wire 11 during loading. The locking assembly includes a locking member 13 and a base 10, which have a locking state that cooperates with each other (corresponding to the first state of the pulling wire) and an unlocking state that disengages from each other (corresponding to the second state of the pulling wire), restricting the pulling wire 11 from detaching from the artificial implant 60 and allowing the pulling wire 11 to detach from the artificial implant 60, respectively. The relationship between the proximal end of the artificial implant and the pulling wire 11 and the locking assembly may refer to the previous embodiments. For example, the locking mechanism may further include a second shaft 23, which is slidably mounted between the first shaft 21 and the third shaft 25 and is controlled at the proximal end by the control handle. The proximal end of the pulling wire 11 is connected to the distal end of the second shaft 23.

The linkage assembly acts between the first shaft 21 and the locking member 13 to selectively link them together. It can be understood that, for the first shaft 21 and the locking member 13, there is an engaging state in which they cooperate with each other and the locking member 13 is driven to move by the first shaft 21, as well as a disengaging state in which they are separated from each other when the cooperation is released. The time for engagement should be after the accurate positioning and expansion of the artificial implant 60, and before the delivery system is ready for withdrawal.

The way of engagement/disengagement of the first shaft 21 and the locking member 13 may be at least one of them moving relative to the other. Combining the above, for example, the locking member 13 is located at the distal end of the base, and the linkage assembly includes two mating portions, one mating portion is connected to the loading section 211 and the other mating portion is connected to the locking member 13. The axial movement of the loading section 211 can cause the two mating portions to engage or disengage. In one embodiment, after accurate positioning and expansion of the artificial implant 60, the loading section 211 located at the distal end is first moved towards the proximal end to engage with the locking member 13. At this time, the loading section 211 is inevitably located inside the artificial implant 60 in the axial position. Then, the first shaft 21 is rotated and the control of the pulling wire 11 is released through the linkage assembly. During the rotation operation, the proximal end of the loading section 211 is not easily or even interfered with the distal end of the artificial implant, for example, the loading section 211 is hung on the distal end of the artificial implant, and thus there is no need to separately set up a drive tube for the locking member, simplifying the structure.

The two mating portions are configured as a linkage key and a linkage groove that can slide relative to each other axially to separate. When the linkage key is inserted into the linkage groove, they are rotatably linked with each other. Of course, the two mating portions may be configured as a linkage key and a linkage groove that can rotate relative to each other to separate. When the linkage key is inserted into the linkage groove, they are linked with each other along the axial direction.

Among them, there are multiple linkage keys, and the loading section and locking member are at least partially tubular, and the multiple linkage keys are arranged along the circumferential direction of the tubular part. Correspondingly, there are multiple linkage grooves.

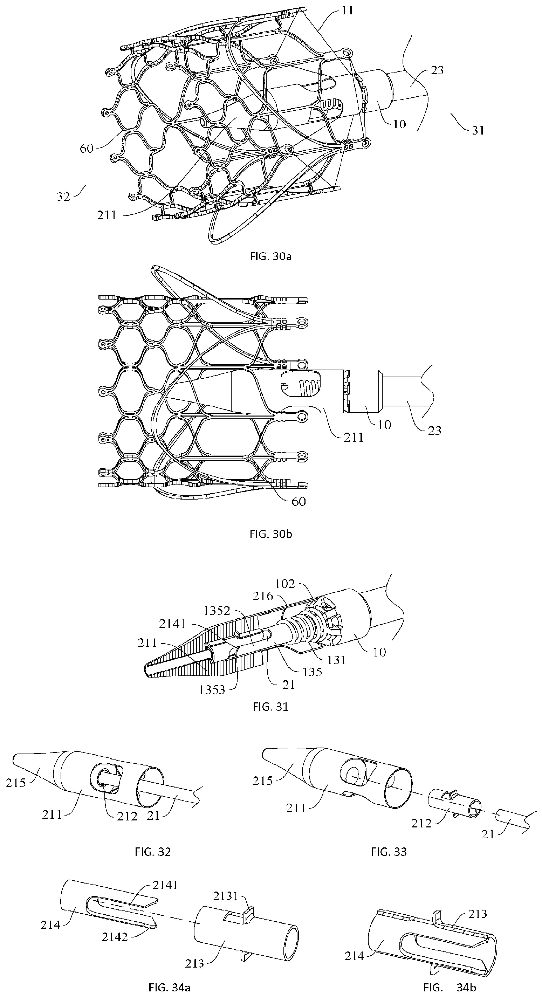

Referring to Fig. 31 to Fig. 34a, combined with the structure of the locking member 13 described above, in one embodiment, at least a part of the locking member 13 is configured as the connecting portion 135. The first shaft 21 and the connecting portion 135 are selectively matched through the linkage assembly to drive the locking member 13 to rotate relative to the base 10.

The linkage key 1352 protrudes radially from one of the connecting portion 135 and the first shaft 21; and the linkage groove 2141 cooperates with the linkage key 1352 and is set on the other of the connecting portion 135 and the first shaft 21, and the linkage key 1352 and the linkage groove 2141 are formed at the end surface of the component where they are located. In the drawings, the linkage key 1352 is set at the distal end of the connecting portion 135, and the linkage groove 2141 is provided on the loading section 211. The rotational linkage between the linkage key 1352 and the linkage groove 2141 is manifested in that: in the circumferential direction, the side wall of the linkage groove 2141 is in contact with the side wall of the linkage key 1352 (as shown in Fig. 31). The connecting portion 135 is a cylindrical structure, and the linkage key 1352 is a sheet-like structure. In one embodiment, there are 2~4 linkage keys 1352 distributed along the circumferential direction, for example, there are two linkage keys 1352.

Preferably, the open side of the linkage groove 2141 is provided with an flared configuration 2142 for guiding the insertion of the linkage key 1352 into the linkage groove 2141, and the distal end surface of the linkage key 1352 is provided with a contraction structure 1353 for guiding the linkage key 1352 into the linkage groove 2141.

The loading section 211 is nested with the connecting portion 135 in the engaging state, and the inner circumferential wall of the loading section 211 is provided with the linkage key 1352, so that the loading section 211 is inside the artificial implant in the engaging state. The inner circumference of the loading section 211 is provided with a support member 212, which is fixed to the distal end of the first shaft 21, and one of the matching portions (i.e., the linkage key) is provided on the support member 212. A limit structure is provided between the support member 212 and the loading section 211 to restrict their axial separation.

In one embodiment, the limit structure includes a positioning piece 2131 protruding radially from an outer circumference of the support member, and a positioning groove set on the inner circumferential wall of the loading section 211 to cooperate with the positioning piece 2131.

The support member 212 is a cylindrical structure, and the outer circumference of the support member 212 is partially flipped to form the positioning piece 2131. The support member 212 includes an outer cylinder 2130 connected to the loading section 211 and an inner cylinder 2140 configured with the linkage key and fixed to the first shaft 21. The inner cylinder and outer cylinders may be integrally formed as one piece or formed as separate structures and then connected together by means of, such as bonding. The outer circumference of the outer cylinder 2130 is partially flipped to form the positioning piece 2131, and the inner cylinder 2140 is partially concaved to form the linkage groove 2141.

The loading section 211 includes a guiding head 215 located at its distal end and connected to the first shaft 21, and a loading portion 216 connected to the guiding head 215 for accommodating the distal portion of the artificial implant. The support member 212 is set inside the guiding head 215. The distal end of the guiding head 215 gradually converges towards the distal end, and the proximal end surface of the guiding head 215 is a flat surface perpendicular to the axial direction. In the engaging state of the loading section 211, the proximal end surface of the loading portion 216 abuts the base 10. Specifically, in combination with the aforementioned embodiments, the proximal end surface of the loading portion 216 abuts the ribs 102 of the base 10. In one embodiment, the loading section 211 has a diameter equal to that of the base 10, and in the engaging state, the outer circumferential surfaces of the loading section 211 and the base 10 are flush.

Among them, the locking member will move towards the distal end during the rotation unlocking process. In one embodiment, when the loading section 211 and the locking member 10 are in the engaging state, there is an axial gap L1 between the linkage key and the linkage groove for the movement of the connecting portion towards the distal end. Similarly, the proximal end surface of the guiding head has the same axial gap L1 from the positioning portion 131. The axial gap L1 is greater than the movement distance of the locking member 13 towards the distal end.

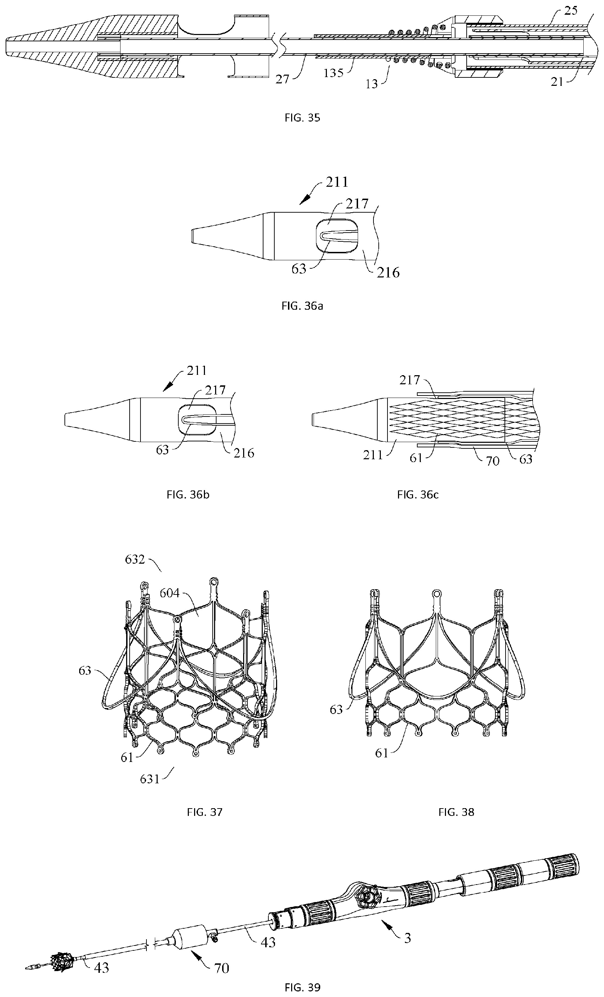

As shown in Fig. 35, the distal end of the first shaft 21 is fixed with a tubular-shaped extension tube 27 extending out of the base, and the locking member 13 is mounted around the outer circumference of the extension tube 27. The loading section 211 is set on the distal end of the extension tube 27. In one embodiment, the first shaft 21 is connected to and mounted around the proximal end of the extension tube 27, that is, the outer diameter of the first shaft 21 is larger than the outer diameter of the extension tube 27. The distal end of the first shaft 21 extends close to the base 10, and the proximal end of the first shaft 21 extends with constant diameter to connect the control handle. The larger diameter of the first shaft 21 facilitates force transmission and control. The smaller diameter of the extension tube 27 facilitates the arrangement of components such as the locking assembly at the distal end, the loading section, etc., reducing the overall size in the radial direction. The proximal end of the extension tube 27 extends into the base and is connected to the first shaft 21 fixedly. In the following embodiments, unless otherwise specified, the extension tube 27 is considered as a part of the first shaft 21.

As shown in Fig. 35 to Fig. 38, an embodiment of the present application also provides a loading structure for an artificial implant. The artificial implant 60 includes an inner frame 61 configured with multiple arm portions 63 on its outer circumference. A first gap is formed between each arm portion 63 and the inner frame 61 to accommodate native tissue. The loading structure includes a first shaft 21 and a loading section 211, wherein the inner frame 61 in a compressed state is mounted around the outer circumference of the first shaft 21. The loading section 211 is tubular-shaped and fixed to the distal end of the first shaft 21. The loading section 211 opens towards the proximal end, and the entire inner frame 61 or at least the distal portion of the inner frame 61 is accommodated in the loading section 211. The circumferential wall of the loading section 211 is provided with a pocket hole 217, and at least a part of the arm portion 63 is located in the pocket hole 217.

The loading section 211 binds the distal portion of the artificial implant 60, maintaining the artificial implant in the collapsed state to ensure safe intervention and delivery. At the same time, a part of the arm portion 63 enters the pocket hole 217, which is located in a portion of the loading section 211 configured with maximum radial space, i.e., the aforementioned loading portion 216. Compared to the existing structure that completely wraps artificial implant, the radial size of the loading section in this embodiment is smaller, which is conducive to interventional delivery.

In the loading state of the artificial implant, a radial relationship between the arm portion and the loading section, as well as the corresponding method of releasing the arm portion, are as follows:
as shown in Fig. 36a, at least part of the arm portion 63 is located within the loading section 211. The loading section 211 moves axially to release its restraint. If there is a catheter sheath mounted around the loading section 211, the catheter sheath 70 needs to be moved first.

Alternatively, as shown in Fig. 36b and Fig. 36c, the loading structure includes a catheter sheath 70, which is slidably mounted on the outermost side of the inner shaft assembly, and the arm portion 63 is located between the loading section 211 and catheter sheath 70. After moving the catheter sheath 70, the restraint on the arm portion 63 can be released. In one embodiment, the distal portion of the artificial implant is located within the loading section, and the proximal portion of the artificial implant is located outside the loading section and within the catheter sheath.

An outflow side 632 of each arm portion 63 is fixedly connected to the inner frame 61, and the inflow side 631 expands to form a first gap with the inner frame 61, wherein the inflow side 631 is located at the pocket hole 217. The artificial implant includes an inner frame that defines a blood flow channel, and multiple arm portions are provided on the outer circumference of the inner frame. A first gap is formed between each arm portion and the inner frame to accommodate native tissue, and leaflets configured for controlling the blood flow channel are connected to the inner frame. The inner frame 61 and the arm portion 63 are formed separately and then fixed together or integrally formed as one piece. The inner frame 61 and the arm portion 63 are formed integrally by cutting a pipe blank. Along the axial direction of the pipe blank, an inflow side of the inner frame 61 and an inflow side of the arm portion 63 are away from each other. Among them, the inner frame 61 is a radially deformable cylindrical structure with blood flow channel 604 defined therein. The leaflets are connected to the inner frame 61 to change the opening degree of the blood flow channel. The arm portion 63 is an integrated annular structure, and the outflow side 632 of each arm portion 63 is fixedly connected to the inner frame 61.

As shown in Fig. 39 to Fig. 41, an embodiment of the present application also provides an interventional delivery system for an artificial implant, including a catheter sheath 70 (i.e. the outer sheath mentioned above), a steering assembly, an inner shaft assembly, and a control handle 3. The catheter sheath 70 is used to construct an interventional channel extending from the outside of the body to a position adjacent to the lesions. The proximal end of the catheter sheath 70 is provided with a fixed seat 7200 (located outside the body). The distal end of the steering assembly can be controlled to change its orientation. The steering assembly slides through the fixed seat 7200 and further extends towards the proximal end. The distal end of the inner shaft assembly is provided with a loading section for connecting the artificial implant 60, which is always exposed to the distal end of the steering assembly. The control handle 3 is connected to the proximal ends of the steering assembly and the inner shaft assembly. The control handle 3 is located at the proximal end side of fixed seat 720, and a distance between the control handle 3 and the fixed seat 720 is adjustable. The catheter sheath 70 of this embodiment is used to assist in the retrieval of the artificial implant.

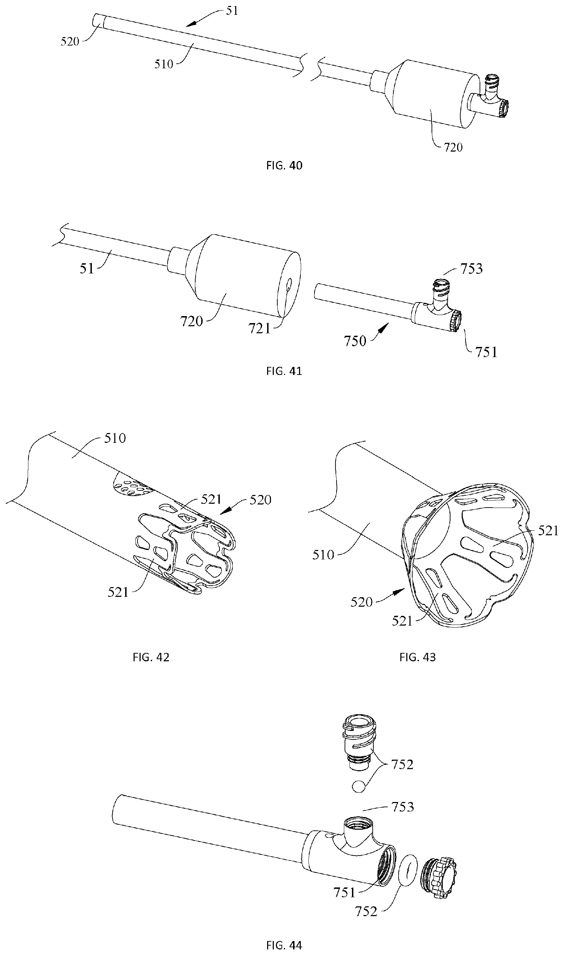

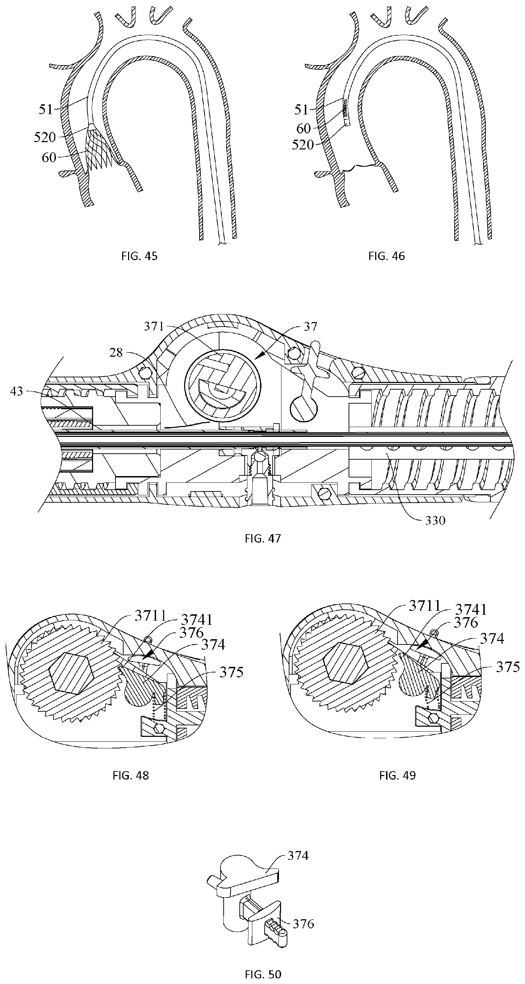

Specifically, the length of the catheter sheath allows it to reach a distant surgical site. During the intervention delivery process, the catheter sheath wraps around the control mechanism, the proximal portion of the artificial implant, and/or the loading section as described in the above embodiments. The catheter sheath 70 includes a catheter body 51, which includes a main section 510 and a deformation section 520. The fixed seat 720 is connected to the proximal end of the main section 510, and the deformation section 520 is located at the distal end of the main section 510. The deformation section 520 includes multiple elastic sheets 521 arranged at intervals along a circumferential direction of the catheter body 51. Each elastic sheet 521 has an initial state (as shown in Fig. 42) and an expanded state (as shown in Fig. 43). In the expanded state, the distal ends of the elastic sheets 521 diverge such that the deformation section 520 assumes an overall flared configuration. In the initial state, the elastic sheets 521 are gathered such that the deformation section 520 assumes an overall cylindrical or radially inwardly contracted configuration. As shown in Fig. 45 and Fig. 46, during retrieval of the artificial implant, the catheter sheath 70 can be moved distally, or the inner shaft assembly can be moved proximally, until the deformation section 520 interacts with the artificial implant 60, at which point the deformation section 520 switches to the expanded state to retract the artificial implant into the catheter body 51.

It should be noted here that during the retrieval operation, it is necessary to ensure that the loading section 211 is located at the distal end of the artificial implant.

In an embodiment, the catheter body 51 has a length of at least 60cm, for example, in ranging of 60~90cm.

The distal end of the catheter body 51 has a pre-shaped contour that matches the shape of the aortic arch. This reduces the safety hazards of the catheter sheath 70 during the intervention delivery process and ensures better shape matching after placement. The pre-shaped catheter body 51 establishes a delivery channel for the steering assembly and the inner shaft assembly, facilitating the steering assembly and the inner shaft assembly to navigate through bends in the body, and simplifies the structure of the steering assembly, such as reducing the number of steering members. The pre-shaped catheter body 51 may also work in conjunction with the steering assembly to achieve more precise bending adjustment and centering effects.

As shown in Fig. 41 and Fig. 44, in one embodiment, the fixed seat 720 is a hemostatic valve, which can adopt the existing structure. In another embodiment, the fixed seat 720 is configured with a mounting channel 721 which communicates with the catheter sheath 70. The proximal end of the fixed seat 720 is provided with a pipe joint 750 connected to the mounting channel 721, and the pipe joint 750 is configured with:
a main nozzle 751 equipped with a sealing member 752; and
a branch nozzle 753 equipped with a one-way valve 754. In one embodiment, the control handle 3 is installed with a steering drive mechanism 37, and the steering assembly includes:
a steerable sheath 43, configured with a proximal end thereof being fixed to a control handle 3;
a steering member, configured with one end thereof extending and fixed to the distal end of the steerable sheath 43 and acting on the catheter sheath, and the other end controlled by the steering drive mechanism.

The distal end of the steering member can be fixed to or move relative to the distal end of the steerable sheath 43. The steering member may be wires, pipes, or a combination of wires and pipes. For example, the steering member is an adjusting wire fixed to the sheath tube, which causes the steerable sheath to bend, or the steering member includes an adjusting tube and an adjusting wire connected to the adjusting tube, and the adjusting wire drives the adjusting tube to drive the steerable sheath to bend.

The distal end of the steering member is fixed to the steerable sheath 43, while the proximal end of the steering member is controlled by the steering drive mechanism 37 and moves relative to the distal end of the steering member, making the distal end of the steering member drive the steerable sheath 43 to bend, thereby bending the catheter sheath 70 and changing the path of the distal end of the delivery system.

As shown in Fig. 47 to Fig.51, the steering drive mechanism includes:
a winding wheel 371 being rotatably mounted on the support body of the control handle 3 (see the following embodiment for details). The proximal end of the steering member 28 is made of flexible material and is wound around the winding wheel 371;
a knob 372 linked with the winding wheel 371; and
a one-way latching structure interacting with the winding wheel 371 and/or the knob 372, and having a relative limiting state and releasing state. In the limiting state, only one-way rotation is allowed for the winding wheel 371.

The bending amplitude is related to the variation of the steering member 28. The steering member 28, by rotating and winding at the proximal end, reduces the axial length of the control handle 3 and increases the bending amplitude. The one-way latching structure is in a limiting state when no force is applied manually, avoiding accidental touch of the knob and restricting the rotation of the knob to maintain the direction of the steerable sheath 43.

The rotational axis of the winding wheel 371 is perpendicular to the axial direction of the control handle. When rotating, the winding wheel 371 has a first direction for bending adjustment and a second direction for resetting by the steering member 28. The unidirectional rotation direction is the first direction, and the one-way latching structure can restrict the rotation of the winding wheel 371 in the second direction before it is released.

In an embodiment, as shown in the drawings, the one-way latching structure includes:
a ratchet wheel 3711; fixed to and coaxial with the winding wheel 371, for example, being formed integrally as one piece;
a limiting member 374, movably mounted on the support body 330 and having a pawl 3741 engaging with the ratchet wheel;
an elastic member 375, acting on the limiting member 374 to make the pawl 3741 engage with the ratchet wheel 3711; and
a toggle button 376, movably mounted on the support body 330 and cooperating with the limiting member 374, serving to drive the limiting member 374 to move, thereby making the one-way latching structure in either a limiting state or a releasing state.

The toggle button 376 extends at least partially out of the support body 330 to the control handle, allowing for operation by the operator.

As shown in the drawings, the limiting member 374 is rotatably mounted on the support body 330. The limiting member 374 and the toggle button 376 are either of an integrated structure or formed separately and then fixed together. During normal operation, the toggle button 376 is toggled to switch the one-way latching structure to the releasing state. Upon releasing the toggle button 376, it resets to the limiting state under the action of the elastic member 375.

If the limiting member 374 fails to reset normally, such as due to the failure of the elastic member 375, this out-of-control state can also be determined by the fact that the toggle button 376 cannot reset and remains in a free-moving state after being released. At this time, it is necessary to control the toggle button 376 to directly act on the limiting member 374 to control the state of the one-way latching structure, which serves as an emergency relief measure to enhance surgical safety.

In an embodiment, the inner shaft assembly includes a first shaft 21, a second shaft 23, and a third shaft 25, which are sequentially slidably fitted from the inside out. The distal end of the first shaft 21 is linked to a locking member 13, the distal end of the second shaft 23 is connected to a pulling wire 11, and the distal end of the third shaft 25 is fixed to a base 10. The locking member 13 is movably mounted on the base 10, and the base 10 is provided with a locking hole 105 matching with the locking member 13. The pulling wire 11 starts from the second shaft 23, wraps around the artificial implant 60, and is then bound to the base 10 by the locking member 13. The proximal ends of the second shaft 23 and the first shaft 21 slidably match with the third shaft 25. The corresponding control method refers to the following embodiments.

An embodiment of the present application also provides a control method for an interventional delivery system, which is used for interventional delivery of the artificial implant of the aforementioned embodiments. The delivery system further includes the control mechanism, the control handle, and the inner shaft assembly connecting the control handle, the control mechanism, and the loading section of the aforementioned embodiments. The control method includes:
expanding at least a part of the arm portion to make the first gap being aligned with native tissue;
driving the loading section 211 towards the distal end to disengage from the inner frame 61, making the distal portion of the inner frame 61 expand;
expanding the proximal portion of the inner frame 61 by releasing the pulling wire 11 exposed to the base, making the entire inner frame deform to an expected amplitude, and maintaining control of the inner frame 61 by the pulling wire 11 throughout the process;
moving the loading section 211 towards the proximal end to make the loading section 211 enter the blood flow channel 604; and
disconnecting the pulling wire 11 from the inner frame 61.

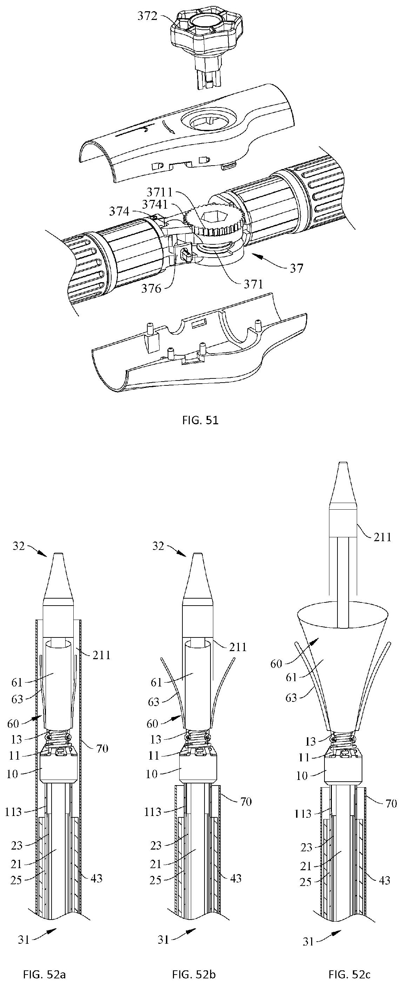

Specific details will be described in combination with the accompanying drawings:
after the artificial implant is delivered to the predetermined position inside the body, the loading section 211 is driven towards the distal end, or the catheter sheath or its internal structure is driven, so as to make the loading section 211 and the catheter sheath move axially in opposite directions (depending on the different settings of the arm portion) until at least a part of the arm portion is released and expanded, the distal end of the inner frame is still bound by the loading section 211, and the first gap is aligned with the native tissue. This embodiment takes the scheme of setting the arm portion between the loading section and the catheter sheath 70 as an example and describes it with reference to the accompanying drawings:
As shown in Fig. 52a and Fig. 52b, by means of driving the catheter sheath 70 towards the proximal end or driving the internal structure of the catheter sheath towards the distal end, the constraint of the catheter sheath on the arm portion 63 is released, and thus the arm portions 63 expand radially and outwardly.

As shown in Fig. 52c, by means of further pushing the first shaft 21 towards the distal end, the constraint of the loading section on the artificial implant is released, that is, the distal end of the inner frame is no longer constrained and expands radially and outwardly, and then it is prepared to expand the proximal end of the inner frame.

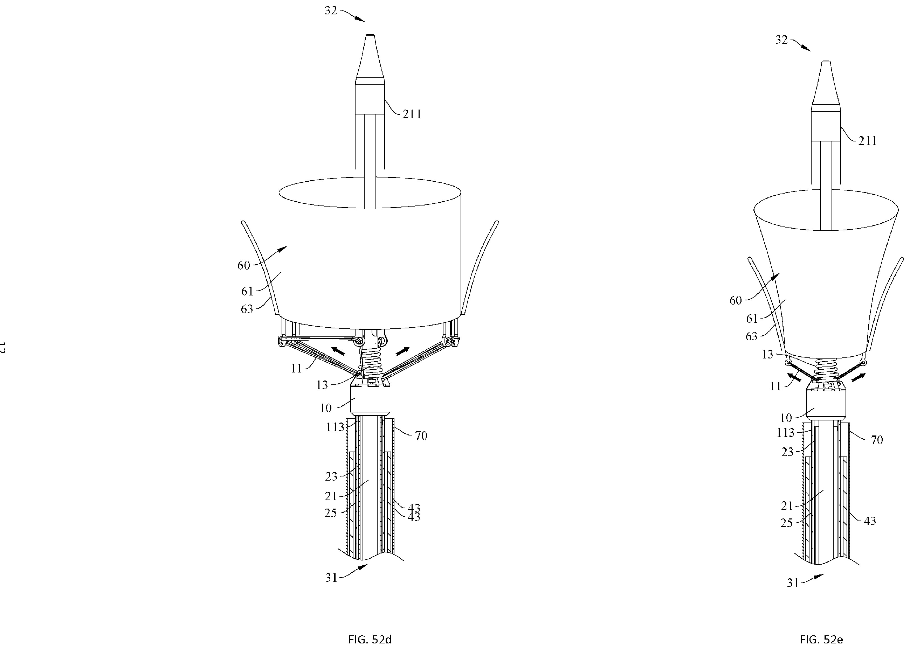

As shown in Fig. 52d and Fig. 52e, the third shaft 25 is kept stationary and the second shaft 23 is pushed towards the distal end, causing the control end 113 of the pulling wire 11 to move towards the distal end 32, gradually extending the exposed part of the pulling wire 11 out of the base, allowing the artificial implant 60 (mainly the proximal portion) to gradually expand. The pulling wire out of the base 10 will gradually extend in the direction of the arrow. The control end 113 continues to move until to the state shown in Fig. 52d, and the artificial implant 60 is in an expanded state (i.e., the expected amplitude). After confirming that it is compatible with the surrounding tissue as expected, the pulling wire can be released.

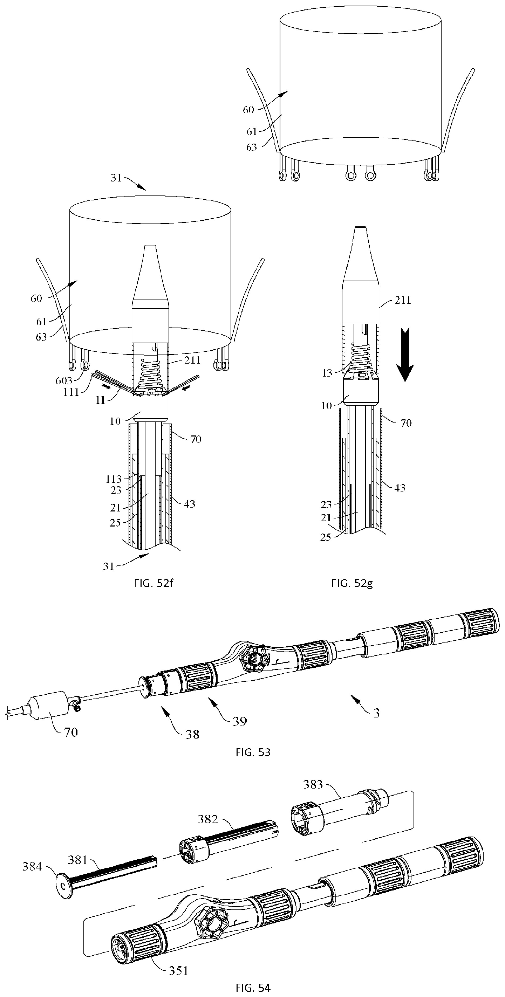

When releasing the pulling wire, as shown in Fig. 52f, the first shaft moves towards the proximal end until the loading section 211 enters the blood flow channel and is combined with the locking member 13 through the linkage assembly. The rotation of the first shaft 21 drives the locking member 13 to rotate relative to the base 10, and sequentially releases all of the pulling wires 11 until all free ends 111 completely detach from the locking member 13.

Then, the second shaft 23 is withdrawn towards the proximal end, causing the control end 113 of each pulling wire to move towards the proximal end 31, so that the free end disengages from the corresponding eyelet 603, i.e., the pulling wire completely disengages from the artificial implant, which can also be understood as releasing the artificial implant.

Finally, as shown in Fig. 52g, the entire delivery system is withdrawn to the outside of the body.

During the process of withdrawal the loading section 211 into the blood flow channel, the pulling wire remains in the first state to maintain the relative fixed spatial posture between the various components of the control mechanism and the artificial implant, guiding the loading section 211 to smoothly retract into the blood flow channel and reducing or eliminating the safety risk of the loading section 211 hanging on the distal end of the artificial implant during retraction.

In addition, before the pulling wire is unlocked, retrieval may be done according to need. The retraction operation generally requires the combination of other components, such as the catheter sheath described in the previous embodiments. The specific operation includes: when the pulling wire is in the first state, the control end of the pulling wire is driven to move towards the proximal end, the proximal end of the artificial implant is retracted by the pulling wire, and then the remaining components inside the catheter sheath are moved towards the proximal end and retracted into the catheter sheath as needed, or the catheter sheath is pushed towards the distal end to receive the artificial implant.

In the initial stage of retrieval of the artificial implant, there is a certain gap between the distal end of the catheter sheath and the proximal end of the artificial implant, or the distal end of the catheter sheath and the proximal end of the artificial implant are already in a state of mutual contact. In this state, there is a significant force between the catheter sheath and the artificial implant, which is fed back to the control handle to make the surgeon feel a significant operational resistance. In this state, the artificial implant can be retracted by referring to the following embodiments of the telescopic assembly and push drive mechanism.

If there is a certain gap between the distal end of the catheter sheath and the proximal end of the artificial implant, a telescopic assembly can be used to directly drive the catheter sheath to move towards the distal end and quickly eliminate the gap. The following method can also be used to drive the inner shaft assembly to move towards the proximal end to eliminate the gap. Alternatively, the above two methods can be combined to eliminate the gap until the surgeon clearly feels the operational resistance, proving that the catheter sheath and artificial implant abut against each other.

It should be noted that the position of the axis in the drawings is not limited, and the control end can be very long or short, or the pulling wire can be directly connected to the control handle and directly retracted and extended. For the control of the pulling wire, the control end of the pulling wire may extend to and directly controlled by the control handle.

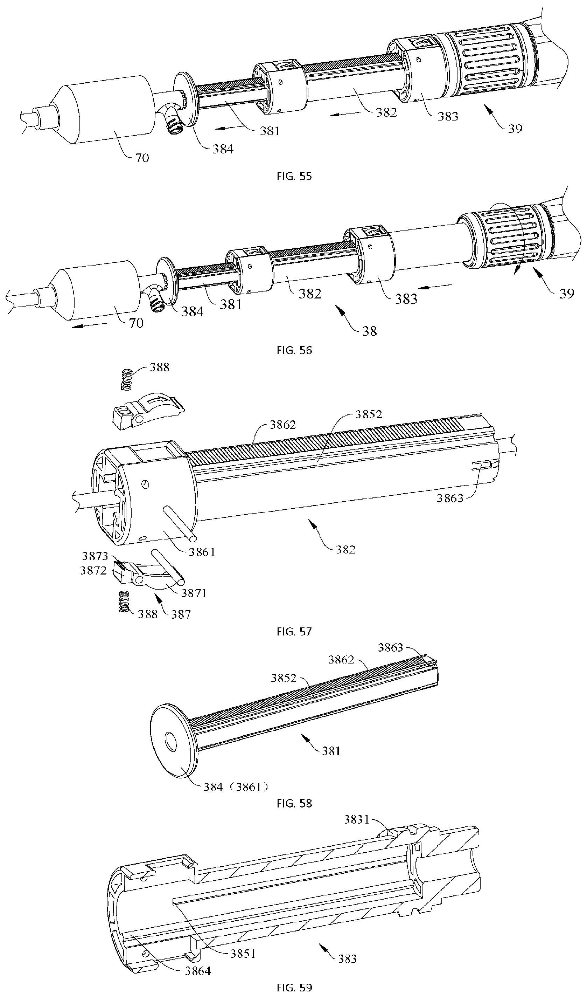

The above control handle can be a conventional control handle. This disclosure provides another type of control handle, as shown in Fig. 53 to Fig. 56. The control handle has opposite distal end and proximal end, as well as an axial direction extending between the proximal and distal ends. The distal end of the control handle 3 is equipped with a push mechanism capable of telescoping in the axial direction. The push mechanism includes a telescopic assembly 38 and a push drive mechanism 39. The distal end of the telescopic assembly 38 has an abutment 384, and the proximal end of the telescopic assembly 38 is connected to the control handle 3 in an axially movable manner. The push drive mechanism 39 is installed on the control handle 3 and linked with the proximal end of the telescopic assembly 38.

The telescopic assembly 38 is used to adjust the distance between the abutment 384 and the catheter sheath in the previous embodiments, and can keep the abutment in any position. The telescopic assembly 38 can maintain a distance between the abutment 384 and the catheter sheath, facilitating intervention in delivery operations. During retrieval of the artificial implant, the abutment 384 is driven to move towards the distal end until it abuts the proximal end of the catheter sheath, and then the inner shaft assembly is driven to move towards the proximal end relative to the catheter sheath 70 until the artificial implant is withdrawn into the catheter sheath 70.

Alternatively, the push mechanism 39 can act on the final level 383 to drive the telescopic assembly 38 to move further towards the distal end, and drive the catheter sheath to move relative to the inner shaft assembly towards the distal end until the artificial implant is withdrawn into the catheter sheath 70.

Alternatively, the retrieval of the artificial implant can only be completed within a certain area of the body. Therefore, the telescopic assembly 38 is first extended to that area, and at this time, the distal end of the catheter sheath 70 is close to the control mechanism, reducing the distance between the catheter sheath and the artificial implant. Then, the inner shaft assembly is withdrawn until the artificial implant is also located in that area. Finally, the push drive mechanism 39 completes the retrieval of the artificial implant according to the above operation.

Among them, the telescopic assembly 38 can move quickly to eliminate the distance between the abutment 384 and the catheter sheath, which belongs to coarse adjustment. The push drive mechanism 39 is controllable and precisely adjusts the axial movement distance of the telescopic assembly 38, which belongs to fine adjustment.

In one embodiment, the telescopic assembly 38 is configured in form of one level or multiple levels from the distal end to the proximal end. The distal end of the first level 381 is equipped with an abutment 384, and the proximal end of the final level 383 is linked with the push drive mechanism 39.

Referring to Fig. 54 to Fig. 61, in an embodiment, the various levels of the telescopic assembly 38 are cylindrical structures and can be movably inserted in sequence to ensure distance adjustment while saving axial space and reducing the axial size of the control handle. For two adjacent levels, one is an outer cylinder and the other is an inner cylinder. When the telescopic assembly 38 extends, the inner cylinder moves towards the distal end relative to the outer cylinder. On the contrary, when the telescopic assembly 38 is shortened, the inner cylinder moves towards the proximal end relative to the outer cylinder. The telescopic assembly 38 has a first limit of maximum length and a second limit of minimum length. Among them, the telescopic assembly 38 includes a first level 381, an intermediate level 382, and a final level 383 from inside to outside.

In one embodiment, anti-retreat structures cooperating with each other are set between adjacent levels. At least the movement of the inner cylinder relative to the outer cylinder towards the proximal end is restricted. During operation, the restriction of the anti-retreat structures needs to be released. For the movement of the inner cylinder relative to the outer cylinder towards the distal end, the anti-retreat structures can restrict it or only provide a certain resistance, for example, the restriction of the anti-retreat structures can be released under the action of an external force.

In one embodiment, the anti-retreat structure includes:
a rack 3862 arranged outside the inner cylinder along the telescopic direction;
an operation button 387 swingably mounted on the outer cylinder, wherein portions of the operation button 387 at two opposite sides of its own swing axis are configured as an operating part 3871 and an engaging part 3872, respectively, the engaging part 3872 engages with the rack 3862 to restrict the movement between adjacent levels, the operating part 3871 is exposed to the outer cylinder where it is located, used to disengage the engaging part 3872 from the rack 3862; and
an elastic member 388 acting on the operation button 387, driving the engaging part 3872 to maintain its engagement with the rack 3862.

The operating part 3871 is pressed to release the restriction of the anti-retreat structure (i.e., unlocking). After release, the operation button 387 is reset by the elastic member 388 and engages with the rack 3862 (i.e., locking). The elastic member 388 is positioned between the inner wall of the outer cylinder and the radial outer side of the engaging part 3872. The engaging part 3872 includes a self-locking surface 3873 that cooperates with the rack 3862. The self-locking surface 3873 faces the distal end side and is approximately perpendicular to the axial direction of the outer cylinder. Among them, the first level 381 and the intermediate level 382 are equipped with racks, while the intermediate level 382 and the final level 383 are equipped with operation buttons 387 and the elastic members 388, wherein the elastic members 388 are springs.

In a preferred embodiment, two operation buttons 387 are symmetrically arranged, and they move towards each other to perform unlocking and locking operations. Correspondingly, two racks 3862 are symmetrically arranged, achieving a better locking effect.

In one embodiment, the tooth tips of the rack 3862 are inclined towards the proximal side, and the tooth surfaces facing the proximal side drive the engaging part 3872 to self-lock. Therefore, the anti- retreat structure is a one-way latching structure, allowing the surgeon to quickly operate the telescopic assembly 38 to extend without unlocking the anti- retreat structure. When the inner cylinder extends to the first limit, it stops extending further due to the limitation of the outer cylinder. Specifically, the proximal end of the inner cylinder is equipped with an elastic buckle 3863, and the inner cavity of the outer cylinder is equipped with a blocking portion 3851 that abuts against the elastic buckle 3863 to restrict the inner cylinder from escaping. During assembly, the elastic buckle 3863 deforms to allow the inner cylinder to be inserted into the outer cylinder, and then extend to the first limit to abut the blocking portion 3851.

In one embodiment, the elastic buckle 3863 is set at the proximal end of the inner cylinder, and the blocking portion 3851 is set at the distal end of the outer cylinder. The blocking portion 3851 is the inner wall of the outer cylinder.

In an embodiment, a sliding guide structure is provided between the inner cylinder and the outer cylinder, specifically including a guiding slot 3852 provided on one of them and extending axially, and a guiding bar 3864 provided on the other of them and cooperating with the guiding slot 3852. In one embodiment, the proximal end of the guide bar is provided with the elastic buckle 3863, and the slot wall at the distal end of the guide slot serves as the blocking portion 3851.

In one embodiment, the distal end of each inner cylinder includes an expansion section 3861 that is exposed to the distal end of a neighboring outer cylinder at the second limit. Surgeons can hold the expansion section to quickly move the corresponding level distally. At the second limit, the inner cylinder abuts the distal end of the neighboring outer cylinder through its expansion section 3861. The abutment 384 also serves as the expansion section 3861 of the first level (i.e., the inner cylinder). The distal end of the final level (i.e., the outer cylinder) includes an expansion section 3861 that is exposed to the distal end of the control handle at the second limit. The operation button 387 and the elastic member 388 are mounted in the expansion section 3861.

In an embodiment, the push drive mechanism includes:
an external thread section 3831 located on the outer circumference of the final level 383;
a first drive sleeve 351 rotatably mounted to the control handle 3 and including an internal thread section that engages with the external thread section.

In an embodiment, a control method for retrieval of an artificial implant using an interventional delivery system is provided, including:
providing an artificial implant and an interventional delivery systems for delivering the artificial implant, the interventional delivery system including:
a catheter sheath, configured for constructing an interventional channel, a fixed seat being provided at the proximal end of the sheath;
an inner shaft assembly, configured with a loading section at its distal end, the artificial implant being at least partially located in the loading section and connected to the inner shaft assembly in a releasable manner before expansion;
a control handle, connected to the proximal end of the inner shaft assembly, the control handle being located at the proximal end side of the fixed seat, and the distal end of the control handle being provided with a push mechanism that can interact with the fixed seat;
at least a portion of the artificial implant maintaining in connection to the inner shaft assembly, and during retrieval of the artificial implant, the catheter sheath being driven to move relative to the inner shaft assembly, allowing the artificial implant to be received into the catheter sheath.

The proximal end and distal end of this embodiment are not strictly limited to installation positions, but can also be directional indicators, such as the push mechanism can extend to the control handle.

The relative movement between the catheter sheath and the inner shaft assembly can be either the catheter sheath moving towards the distal end, or the inner shaft assembly moving towards the proximal end, or the inner shaft assembly moving towards the proximal end and the catheter sheath moving towards the distal end, until the distal end of the catheter sheath approaches or contacts the artificial implant, eliminating the aforementioned gap, and waiting for the next operation. During retrieval of the artificial implant, the push mechanism or other components can be pressed against the proximal end of the fixed seat to provide sufficient force transmission, facilitating the retrieval operation. The artificial implant, the catheter sheath, the inner shaft assembly, and the control handle are constructed according to the aforementioned embodiments. Specifically, in one embodiment, the distal end of the catheter sheath approaches or contacts the artificial implant, driving the push mechanism to abut against the fixed seat. First, the telescopic assembly extends towards the distal end until the first level abutting the fixed seat. Then, the push drive mechanism is controlled to drive the entire telescopic assembly to move towards the distal end and act on the catheter sheath through the fixed seat to move it towards the distal end until the retrieval of the artificial implant is completed.

The expansion of at least a portion of the artificial implant relative to the inner shaft assembly is understood as deformation to expand outwardly, or expansion after the distal end side detaching from the loading section or catheter sheath.

In one embodiment, the artificial implant is connected to the inner shaft assembly through a pulling wire. During retrieval of the artificial implant, the pulling wire is kept in a tightened state to bring the proximal end side of the artificial implant together, which facilitates smooth entry of the proximal end of the artificial implant into the catheter sheath during retrieval of the artificial implant. Before retrieval of the artificial implant, if the artificial implant is in an expanded state, it is necessary to first tighten the pulling wire to bring the proximal end side of the artificial implant together. Among them, during retrieval of the artificial implant, the proximal end of the arm portion of the artificial implant enters the catheter sheath first, and the distal end of the arm portion enters the catheter sheath adaptively with the movement of the catheter sheath relative to the artificial implant.

In one embodiment, a loading method for loading an artificial implant into a delivery system is provided, including: providing an artificial implant and a delivery system, wherein the artificial implant includes an inner frame configured with multiple arm portions on its outer circumference, and the delivery system includes:
a catheter sheath configured with a fixed seat at its proximal end;
an inner shaft assembly, extending movably through the catheter sheath, a loading section being provided at the distal end of the inner shaft assembly; and
a control the handle connected to the proximal end of the inner shaft assembly.

Specifically, the loading section is driven by operating the control handle, and the catheter sheath is operated to receive and wrap the artificial implant in an expanded state inside the loading section and the catheter sheath, so that the artificial implant is in a loading state.

Before loading, the artificial implant is in an expanded state and separated from the delivery system. In one embodiment, the pulling wire is used to pass through and wind around the proximal end of the inner frame to complete the connection between the artificial implant and the delivery system. Referring to the aforementioned structure, the delivery system further includes a control mechanism, which includes a base connected to the inner shaft assembly, a locking member that movably cooperates with the base, and multiple pulling wires. The inner shaft assembly may refer to the previous embodiments.

One end of the pulling wire (i.e., the control end) is connected to and controlled by the control handle, and the other end of the pulling wire (i.e., the free end) extends out of the base and, under the operation of the control handle, elongates the length exposed out of the base to provide sufficient length for the pulling wire to pass through and wind around the proximal end of the inner frame and return to the corresponding locking area of the base, waiting to be locked to the base and the locking member, wherein the multiple pulling wires are locked one by one. During the locking process, the locking member rotates relative to the base.

Subsequently, the control handle is operated to drive the control end of the of the pulling wire to move towards the proximal end to tighten the pulling wire, i.e., reducing the length of the pulling wire exposed out of the base, thereby compressing the proximal end of the inner frame radially until it is close to the base. During the tightening process, all of the pulling wires are tightened synchronously.

Next, compression and loading operations are performed to the distal end of the inner frame:

In one embodiment, the distal end of the inner frame is compressed, and the control handle is operated to drive the loading section to move towards the proximal end to accommodate the distal end of the inner frame. At this time, the arm portions are still in an expanded state and not been accommodated by the loading section. Then, the control handle is operated again to drive the catheter sheath to move towards the distal end to receive the arm portions and the proximal end of the inner frame. Among them, the method of compressing the inner frame may be done by using a gripper in a cold water bath or manually compression.

In another embodiment, the inner frame and the distal ends of the arm portions are compressed, and the control handle is operated to drive the loading section to move towards the proximal end to receive the distal end of the inner frame and at least the distal ends of the arm portions. Then, the control handle is operated again to drive the catheter sheath to move towards the distal end for receiving the proximal end of the inner frame and the exposed parts of the arm portions. Among them, the method of compressing the inner frame and the arm portions may be done by using a pressure gripper in a cold water bath or manual compression. In one embodiment, the arm portions avoid the loading section or are placed on the outer circumference of the loading section, and the distal end of the catheter sheath abuts the loading section in the axial direction, or wraps around the proximal end of the loading section. In one embodiment, the method of moving the catheter sheath towards the distal end includes driving the catheter sheath to push the fixed seat towards the distal end relative to the control handle.

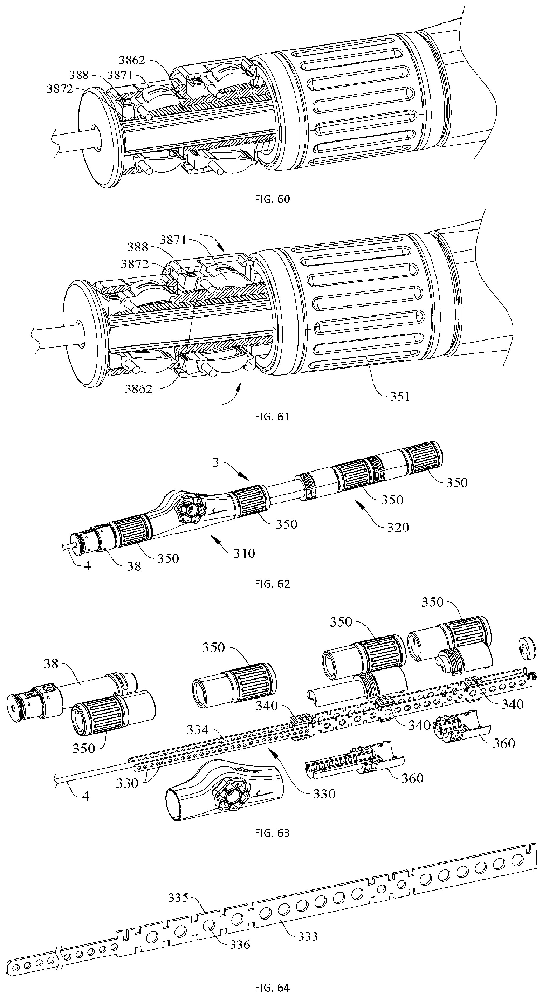

In the above two embodiments, the loading section has a pocket hole, and during the process of receiving the distal end of the inner frame, it also includes first aligning the pocket hole with the circumferential direction of the arm portion. The method of aligning in circumferential direction includes operating the control handle to drive the loading section to rotate in the circumferential direction. As shown in Fig. 62 to 64, the present application also provides a control handle 3, which is connected to a control catheter assembly 4 and includes:
a support body 330, including two rigid bars 333 arranged side by side, with a guiding channel 334 defined between the two rigid bars 333;
a plurality of drive mechanisms, at least two of which include a transmission member 340 that is slidably mounted in the guiding channel 334, and a driving sleeve 350 that is rotatably mounted around the outer circumference of the support body 330 and threaded with the transmission member 340.

The support body is configured as two rigid bars, on the one hand, the radially opposite sides of the guiding channel are penetrated, which facilitates the installation of the transmission member along the radial direction; at the same time, the guiding channel is also penetrated in the axial direction, which meets the motion requirements of the transmission member. Moreover, compared with the existing support structure, the strip-shaped structure is more convenient for processing and is not limited by materials. For example, the rigid bars 333 are metal strips, which effectively improve the structural strength of the support body.

In addition, if the number of components in the control mechanism increases or the operation becomes more complex, such as an increase in the number of transmission members and driving sleeves, rigid bars can be extended along the axial direction to meet the installation needs of excess transmission members and driving sleeves. The installation methods between each transmission member and the support body, and between each driving sleeve and the support body, are the same, achieving modular installation without sacrificing its own structural strength to meet installation requirements.

In one embodiment, the length of the two rigid bars 333 accounts for at least 75% of the total length of the control handle 3, where the length of the two rigid bars 333 is the length between two axial ends, and the total length of the control handle is the length of the control handle in a fully expanded state, i.e., starts from the distal end surface of a first driving sleeve 351 and ends at the proximal end surface of a actuator 355 described in the following embodiment.

In one embodiment, the rigid bar 333 is provided with multiple lightening holes 336 to reduce the weight of the support body and decrease the contact area between the support body and the transmission member, thereby reducing friction and making the movement of the transmission member smoother.

In one embodiment, the distal end of the rigid bar 333 extends into the telescopic assembly 38, specifically the distal end of the rigid bar 333 extends close to the first level.

In another embodiment, the catheter assembly 4 includes a first group and a second group that are arranged in a movable manner from the outside to the inside. The control handle 3 includes a first handle 310 and a second handle 320. The first handle 310 is used to connect the first group, and the second handle 320 is used to connect the second group. Both the first handle 310 and the second handle 320 include a support body 330, and each support body 330 includes two rigid bars 333 arranged side by side. The first handle 310 and the second handle 320 can share the same support body (as shown in Fig. 63), or the support bodies of the first handle 310 and the second handle 320 are not shared. The first handle 310 and the second handle 320 are far away from each other, and the longest distance between the first handle 310 and the second handle 320 corresponds to the fully expanded state of the control handle.

As shown in Fig. 63 to Fig. 67, in one embodiment, the rigid bar 333 includes a clamping block 335 that engages with the first handle 310 and/or the second handle 320. To maintain the relative fixation between the rigid bar and the corresponding handle, a clamping slot 337 matching with the clamping block 335 is defined in the corresponding handle. This mating method is also applicable to other components, such as transmission members. For example, when the first handle 310 and the second handle 320 are in active engagement (with relative changes in the axial and/or circumferential directions between the two handles), the rigid bar 333 is connected and fixed to one of the handles via the clamping block 335, while it is in sliding and/or rotational engagement with the other handle.

In one embodiment, the control handle 3 includes a mounting seat 360 fixedly connected to the two rigid bars 333, and the driving sleeve 350 is embedded between the rigid bars 333 and rotatably cooperates with the mounting seat 360. The mounting seat 360 defines a clamping slot mating with the clamping block 335 and provides the installation foundation for the driving sleeve, while maintaining relative fixation between the two rigid bars. Further description of modular installation is described as follows:

The mounting seat 360 includes two flaps that are interlocked radially with each other. Before installing the driving sleeve, the two flaps are first fastened and fixed to the support body, then the transmission member is inserted into the guiding channel, and finally the driving sleeve is mounted and combined with the transmission member. Among them, the installation direction of the mounting seat 360 does not interfere with the installation direction of the transmission member, and the installation sequence of the mounting seat 360 and the transmission member is not strictly limited, making the installation more flexible.

The first handle 310 and the second handle 320 share the same support body, with the second handle 320 fixedly connected to the support body and slidably cooperating with the first handle 310. The first group of catheter assembly 4 includes a steerable sheath 43, and the second group of catheter assembly 4 includes a first shaft 21, a second shaft 23, and a third shaft 25. Multiple driving sleeves, from the distal end to the proximal end, sequentially include:
a first driving sleeve 351, as described in the previous embodiment, used to control the movement of the telescopic assembly 38;
a second driving sleeve 352, installed on the first handle 310 to control the second handle 320 to slide axially relative to the first handle 310;
a third driving sleeve 353, installed on the second handle 320 to control the axial movement of the second shaft 23; and
a fourth driving sleeve 354, set on the second handle 320 to control the axial movement of the first shaft 21.

Multiple transmission members, from far to near, include:
a first transmission member 341, fixedly connected to the support body 330, fixed to the third shaft 25 and threaded with the second driving sleeve 352;
a second transmission member 342, slidably installed in the guiding channel 334, fixedly connected to the second shaft 23 and threaded with the third driving sleeve 353; and
a third transmission member 343, slidably installed in the guiding channel 334, fixedly connected to the first shaft 21 and threaded with the fourth driving sleeve 354.

In one embodiment, there are multiple mounting seats 360 arranged along the axial direction, and the head and tail of the driving sleeve 350 are limited by two adjacent bases and rotate in cooperation with one of the two adjacent bases. Specifically, from far to near, a first mounting seat 361, a second mounting seat 362, and a third mounting seat 363 are arranged on the second handle 320 in sequence. The third driving sleeve 353 is embedded in the first mounting seat 361, with its head and tail being limited to the first mounting seat 361 and the second mounting seat 362, and the fourth driving sleeve 354 is embedded in the second mounting seat 362, with its head and tail being limited to the second mounting seat 362 and the third mounting seat 363.

Among them, the first mounting seat 361 is set at the distal end of the second handle 320, and the third mounting seat 363 is set at the proximal end of the second handle 320. Regarding the structure of the driving sleeve 350, the driving sleeve 350 includes a driving ring 356, as well as a first locking ring 357 and a second locking ring 358 connected to two axial ends of the driving ring 356, respectively. The driving ring 356 includes two pieces interlocking with each other, the first locking ring 357 is threaded to the driving ring 356, and the second locking ring 358 is mounted around the driving ring 356.

In one embodiment, the driving sleeve 350 includes an operating portion 3561 and a connecting portion 3562 embedded in the mounting seat 360 arranged along its axial direction. The mounting seat 360 separates the operating portions 3561 of two neighboring driving sleeves 350 from each other, reducing the probability of misoperation.

In one embodiment, the first transmission member 341 is located at the distal end of the first handle 310, specifically at the distal or proximal end of the first mounting seat 361. The first transmission member 341 is directly or indirectly connected and fixed to the support body 330, for example, the first transmission member 341 is fixedly connected to the support body 330 directly, or the first transmission member 341 is fixedly connected to the first mounting seat 361 (indirectly connected).

In the drawings, the first mounting seat 361 has a connecting sleeve 364 that extends towards the distal end into the first handle 310, and the first transmission member 341 is provided at the distal end of the connecting sleeve 364. The connecting sleeve 364 is fixedly connected to the support body and wrapped around the outer circumference of the support body 330, serving as a guide for the second handle sliding relative to the first handle and covering the support body.

In one embodiment, a section of the rigid bar in the second handle has a width larger than that of a section in the first handle.

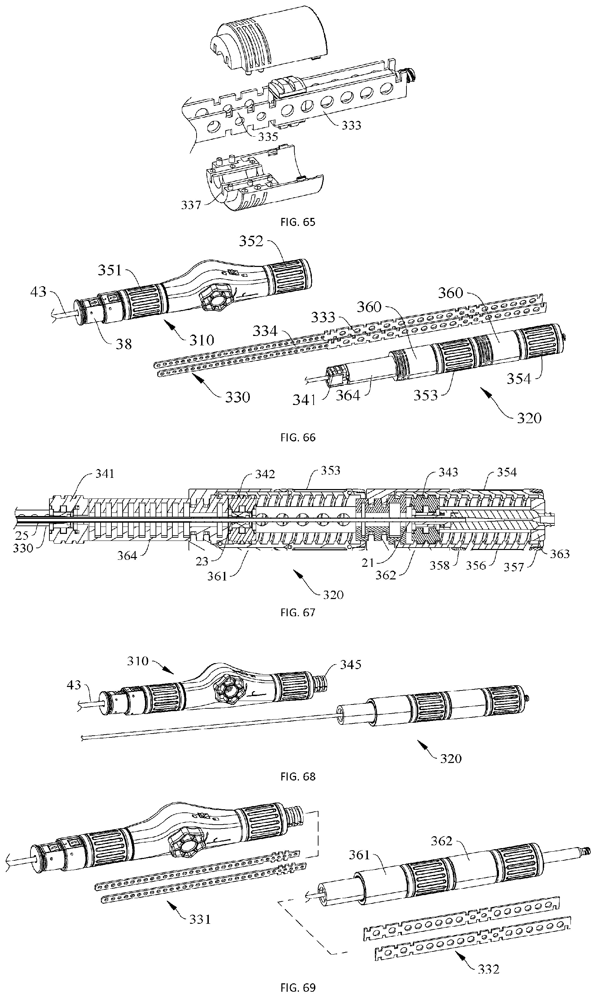

In another embodiment, as shown in Fig. 68 to Fig. 70, the first handle 310 and the second handle 320 do not share the same support body. That is, the first handle 310 includes a first support body 331, and the second handle 320 includes a second support body 332. The second handle 320 is movable and cooperates with the first handle 310. Specifically, the second handle 320 rotates and slides axially relative to the first handle 310, and the configuration of the driving sleeve refers to the previous embodiments.

The proximal end of the first handle 310 is provided with a sliding seat 345 that is threaded with the second driving sleeve 352. The sliding seat 345 is fixedly connected to the first support body 331. The first mounting seat 361 of the second handle 320 is rotatably matched with the sliding seat 345, and a clamping structure that restricts axial movement is provided between the first mounting seat 361 and sliding seat 345. The third shaft is fixedly connected to the first mounting seat 361. The second handle 320 rotates relative to the first handle 310, allowing the remote artificial implant to rotate with the control mechanism to adjust its spatial orientation.

In the drawings, the connecting sleeve 364 of the first mounting seat 361 rotataby cooperates with the sliding seat 345, and the proximal end of the third shaft extends through the sliding seat 345 and is fixed inside the connecting sleeve 364. The first support body 331 is fixedly connected to the sliding seat 345, and the second support body 330 is fixedly connected to the first mounting seat 361, the second mounting seat 362, and the third mounting seat (not visible in the assembled state), respectively. The distal end of the first support body 331 extends adjacent to the first level of the telescopic assembly, and the proximal end of the second support body 332 extends to the connecting sleeve 364 and is close to the sliding seat 345.

Among them, the rigid bar corresponding to the second support body has a width larger than that of the rigid bar corresponding to the first support body.

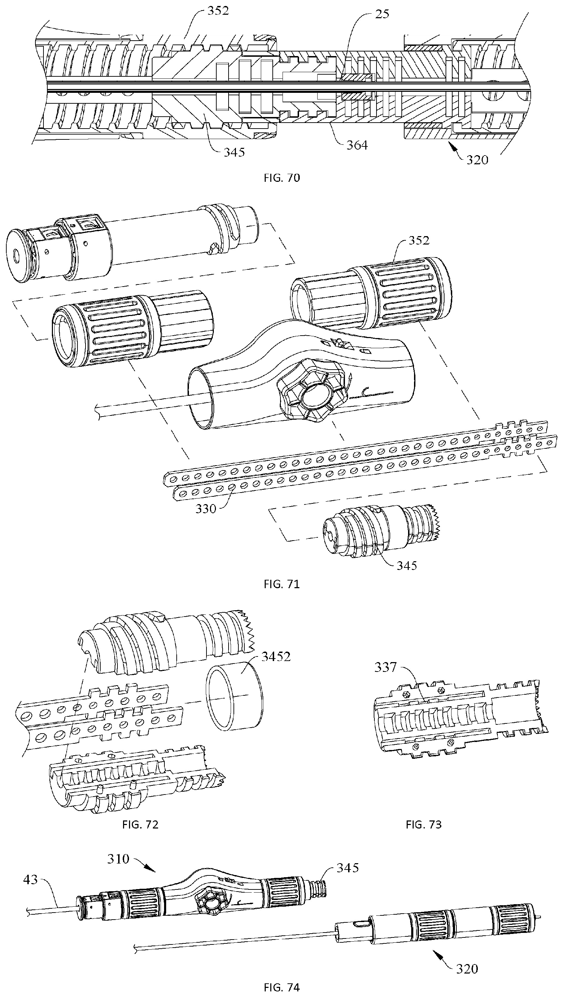

As shown in Fig. 70 to Fig. 73, in one embodiment, the sliding seat 345 includes two flaps interlocking with each other and a collar 3452 that is mounted around the two flaps to restrict their separation. Each flap defines a clamping slot 337 that engages with the first support body.

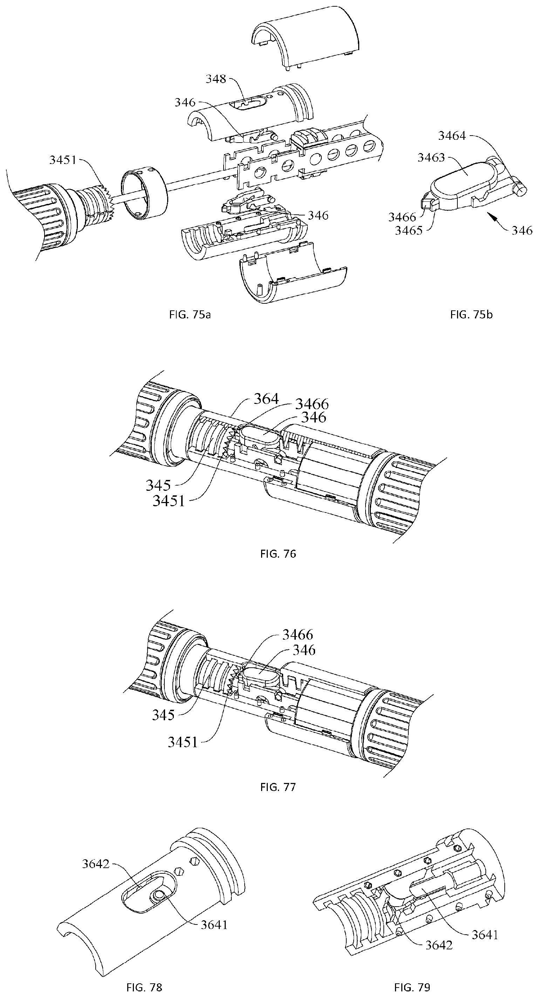

As shown in Fig. 74 to Fig. 80, in one embodiment, a rotating locking mechanism that cooperates with each other is provided between the sliding seat 345 and the connecting sleeve 364. The locking mechanism includes:
a locking state, restricting the second handle 320 from rotating relative to the first handle 310; and an unlocking state, allowing the second handle 320 to rotate relative to the first handle 310. That is, when the second handle 320 rotates, it is necessary to first unlock the rotating locking mechanism to prevent accidental operation.

The rotating locking mechanism includes:
meshing teeth 3451 located at the end face of the proximal end of the sliding seat 345 and distributed around the rotational axis of the connecting sleeve 364;
an unlocking member 346, actively installed on the connecting sleeve 364, having a locking position that matches with meshing teeth 3451 and an unlocking position that is radially separated from the meshing teeth 3451; and
a driving member 347 acting on the unlocking member 346, driving the unlocking member 346 to remain in the locking position. Among them, the proximal part of the unlocking member is installed on the connecting sleeve 364 in a swingable manner, and the connecting sleeve 364 defines an operation window 348. At least a part of the unlocking member 346 is exposed to the operation window 348, being easy to operate and control.

In one embodiment, the unlocking members 346 are arranged in pairs along the radial direction of the connecting sleeve 364, and the driving member 347 is elastically compressed between the same pair of unlocking members 346. In the drawings, the connecting sleeve 364 has a base portion 3641 extending axially between the two unlocking members 346, and the driving member 347, such as a spring, is elastically compressed between the unlocking member 346 and the base portion 3641. The connecting sleeve 364 is provided with a guiding step 3642 therein, and the unlocking member cooperates with the guide step and has a tendency to move towards the unlocking position under the radially and inwardly guiding of the guiding step3642.

Among them, the meshing teeth 3451 gradually converges towards the proximal end, and has a shape of , for example triangle.

In one embodiment, the inner wall of the connecting sleeve is provided with a guiding groove 3462 which extends radially, and at least a part of the unlocking member 346 moves along the guiding groove 3462.

The unlocking member 346 includes:
a pressing portion 3463, exposed to the operation window 348;
a swinging shaft 3464, located at the proximal end side of pressing portion 3463;
a guide portion 3465, located at the distal end side of the pressing portion 3463 and cooperating with the guiding groove 3462; and
a locking portion 3466, located at the distal end side of the guide portion 3465 and meshing with the meshing teeth 3451, wherein the locking portion 3466 as a whole is conical and converges towards the distal end.

In one embodiment, at least a portion of the unlocking member 346 is exposed to the connecting sleeve 364, and this exposed portion is exposed to the first handle 310 or accommodated inside the first handle in accordance with the axial movement of the second handle 320. For example, when the unlocking member 346 is in the locking position, the exposed portion is flush with the outer circumferential surface of the connecting sleeve 364, which facilitates the storage of the connecting sleeve 364 into the first handle 310 when the second handle 320 slides towards the distal end.

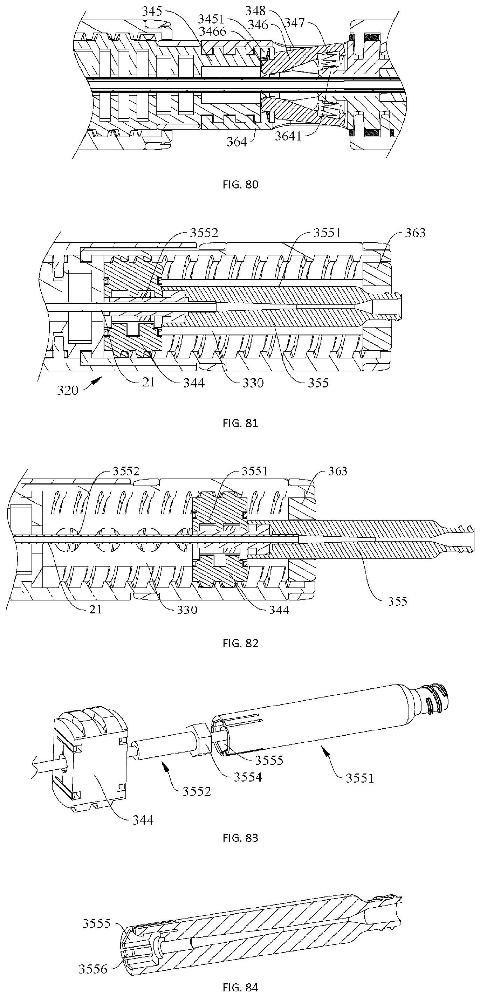

As shown in Fig. 81 to Fig. 84, the present application also provides a control handle 3 for controlling a catheter assembly to operate an artificial implant. The catheter assembly includes multiple shafts sliding nested inside and outside. The control handle 3 includes a support body 330, a transmission member 340 set on the support body 330 and fixed to each shaft, and a driving sleeve 350 rotatably mounted on the support body 330 and threaded with the transmission member 340. Among the multiple shafts, the first shaft 21 is located at the innermost layer, and the proximal end of the first shaft 21 is connected to a fourth transmission member 344 and a actuator 355 that slidably matches with the support body 330. The first shaft 21 slides along the axial direction to make the actuator 355 have a first position that is at least partially hidden inside the support body 330 and a second position exposed to the support body, wherein the actuator 355 can drive the first shaft 21 to rotate.

Based on the above, the actuator 355 is at least partially located inside the second handle when in the first position. When the actuator 355 is in the second position, an exposed portion is added compared to the first position, making it easier to operate. When the actuator 355 is in the first position, the total length of the control handle 3 is shortened, which is beneficial for packaging and reduces the probability of accidental contact during initial operation.

The distal end of the first shaft 21 is set according to the previous embodiment, that is, the actuator 355 is always in the first position after the distal end of the artificial implant is expanded until the loading section is combined with the locking member, to avoid misoperation, avoid circumferential displacement of the linkage structure, and ensure successful combination of the loading section and the locking member. Correspondingly, when the actuator 355 is in the second position, the loading section is combined with the locking member.

In one embodiment, the actuator 355 is fixedly connected to the first shaft 21 and is in transmission cooperation with the fourth transmission member. The transmission cooperation between the actuator 355 and the fourth transmission member includes synchronous movement in the axial direction to meet the axial movement of the distal end of the first shaft, such as the loading section, and rotation of the actuator 355 relative to the fourth transmission member to meet the operation of releasing the pulling wire. In one embodiment, the actuator 355 is in threaded engagement with the fourth transmission member.

The actuator 355 is a cylindrical structure, including an operating section 3551 and a connecting section 3552 located at the distal end of the operating section 3551. The connecting section 3552 is connected to the fourth transmission member 344. The connecting section 3552 has a first section 3553 extending into and connected to the fourth transmission member, and an expansion section 3554 connected to the proximal end of the first section. The distal end of the operating section 3551 is provided with a latching portion 3555 that accommodates the expansion section 3554 and cooperates with the expansion section 3554 in the circumferential direction. The proximal end of the first shaft 21 extends through the connecting section 3552 and is connected to the operating section 3551.

The outer circumferential surface of the expansion section 3554 is a polygonal structure, and the inner cavity of the latching portion 3555 matches the outer circumferential surface of the expansion section 3554 to restrict the relative rotation of the operating section 3551 and the connecting section 3552. The side wall of the latching portion 3555 is provided with an elastic buckle 3556 that resists the expansion section 3554 to prevent the connecting section 3552 from disengaging from the operating section 3551. In one embodiment, the operating section 3551 has an axial sliding stroke relative to the connecting section 3552.

Among them, the connecting section 3552 is a bolt, the first section 3553 has external threads, and the fourth transmission member has internal threads matching with the external threads.

Combined with the aforementioned control mechanism, during the process of releasing the pulling wire, the locking member will undergo a spiral motion accompanied by axial movement towards the distal end, thereby driving the loading section and the first shaft connected to the loading section to move towards the distal end. In one embodiment, the connecting section 3552 slides axially relative to the operating section 3551. Specifically, the expansion section 3554 can slide within the latching portion 3555. During the actual release of the pulling wire, the operating section 3551 rotates in the first direction around the axial direction, driving the connecting section 3552 to rotate synchronously and move axially towards the distal end relative to the fourth transmission member until the operating section 3551 contacts the proximal end of the fourth transmission member and the expansion section 3554 contacts the elastic buckle 3556, thereby restricting the rotation of the operating section. At the same time, all of the pulling wires are released. It should be noted that the axial movement of the connecting section 3552 is adapted to the axial movement when the locking member rotates.

In one embodiment, the actuator 355 is a hollow structure, and its proximal end can be connected to a Y-shaped valve. Among them, the actuator 355 extends partially before expanding the distal end of the artificial implant and moves towards the distal end to enter the support body 330 during the expansion of the artificial implant, so as to avoid touching the control handle with the Y-shaped valve. At this time, the axial length of the portion of the actuator 355 exposed outside the support body is smaller than the axial length of the portion of the actuator 355 exposed outside the support body when in the second position. Correspondingly, the axial size of the control handle has been reduced, which is beneficial for packaging and holding during delivery.

Before expanding the artificial implant, although the actuator 355 is exposed outside the support body, the loading section of the first shaft connected to it is limited by the force (such as friction) between the loading section and the artificial implant, which in reverse acts on the actuator 355 to restrict its rotation, or make its rotation require a large driving force, so as to reduce the probability of misoperation.

The other structures of the control handle refer to the previous embodiments, that is, a third mounting seat 363 is provided at the proximal end of the support body 330. In one embodiment, the third mounting seat 363 is a cylindrical structure, and its inner cavity is in clearance with the outer circumferntial surface of the actuator 355, playing a sliding guidance role for the actuator.

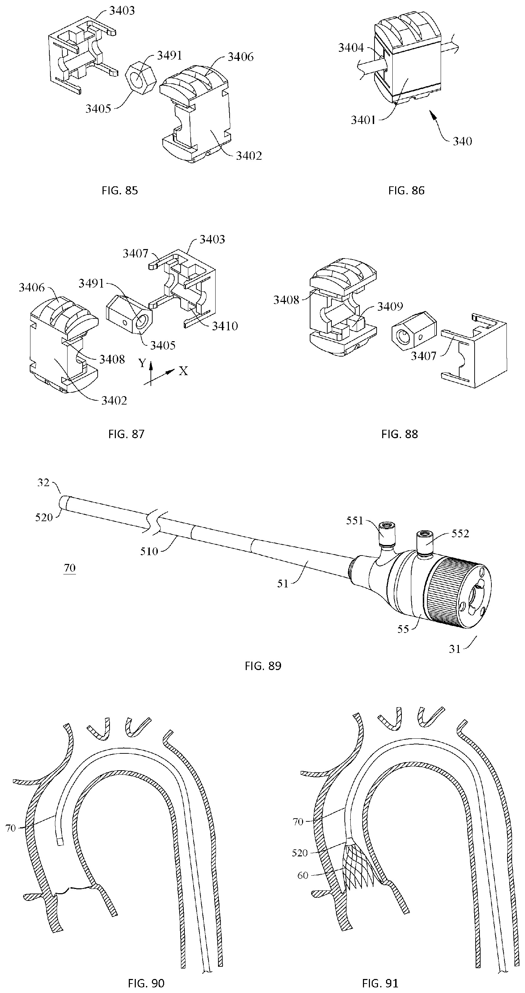

As shown in Fig. 85 to Fig. 88, this embodiment provides a transmission member 340 of a control handle, for connecting an interventional catheter. The transmission member 340 includes a main body 3401. The main body 3401 has an axial through-hole 3404 for inserting the interventional catheter, and the main body 3401 includes:
a first half 3402 having threaded transmission teeth 3406 on its outer wall;
a second half 3403 interlocked with the first half 3402, wherein the through-hole 3404 is located between the first half 3402 and the second half 3403; and
a fixing member 3405 surrounded by the first half 3402 and the second half 3403, the fixing member 3405 has a catheter fixing hole 3491 corresponding to the through-hole. The outer circumference of the fixing member 3405 is provided with an anti-rotation structure with at least one of the first half 3402 and the second half 3403.

The interventional catheter can be any of the shafts in the above embodiments, including the first shaft and the second shaft. The interventional catheter is fixedly connected to the fixing member 3405, for example, by bonding or tight fitting. After connecting the interventional catheter to the fixing member, the first half 3402 and the second half 3403 are interlocked and wrapped around the fixing member 3405 to complete the assembly. The control handle adopts the structure of the previous embodiments, that is, the assembled transmission member 340 is placed inside the support body 330, and then threaded with the driving sleeve through the transmission teeth 3406.

In one embodiment, the through-hole 3404 has a first radial direction Y and a second radial direction X that are perpendicular to each other. The body 3401 has threaded transmission teeth 3406 on two sides along the first radial direction Y. The first half 3402 and the second half 3403 are interlocked along the second radial direction X. In the drawings, the first half 3402 is provided with threaded transmission teeth 3406 on two sides of the first radial direction Y, which maintain the integrity of the threaded transmission teeth 3406, facilitate processing, and maintain the accuracy of the fit with the driving sleeve.

In one embodiment, the first half 3402 and the second half 3403 are interlocked with each other through an elastic buckle. In the drawings, the second half 3403 is provided with an elastic buckle 3407 extending along the second radial direction X. The first half 3402 has a groove 3408 adapted to the elastic buckle 3407, so that two opposite sides of the mian body 3401 along the second radial direction X are smooth planes, which facilitates cooperation with the rigid bars 333 at two sides.

In one embodiment, one of the first half 3402 and the second half 3403 is provided with a positioning slot 3409 extending along the second radial direction X, and the other is provided with a positioning key 3410 that matches the positioning slot, for positioning during fastening.

In one embodiment, the setting method of the anti-rotation structure is that: the fixing member 3405 is a ring-shaped structure, and its inner hole is a fixing hole 3491 with a non-circular outer contour. The first half 3402 and the second half 3403 both have anti-rotation inner edges that partially or completely conform to the outer contour of the fixing member 3405.

The control mechanism of this application can cooperate with each other to achieve control over the expanding, releasing, or retrieval of the artificial implant in the body. The structure has been further optimized and improved, making the control of the pulling wire smoother.

The catheter sheath in the above embodiment is used to establish a temporary channel for the delivery system to intervene and deliver the artificial implant to the surgical site through this channel. The application site and structure of artificial implant are not strictly limited, and combined with this application, they have at least longer axial dimensions and are suitable for longer intervention paths.

As shown in Fig. 89 to Fig. 100, the present application provides a catheter sheath 70 based on long-distance intervention, which has a distal end 32 and a proximal end 31 opposite to each other. The catheter sheath 70 includes a catheter body 51 and a hemostatic valve 55. The catheter body 51 includes a main section 510 and a deformation section 520. The hemostatic valve 55 is connected to the proximal end of the main section 510, and the catheter wall of the main section 510 has a structural reinforcement layer at least near the distal end. The reinforcement layer adopts a metal tube 511 with a hollow structure.

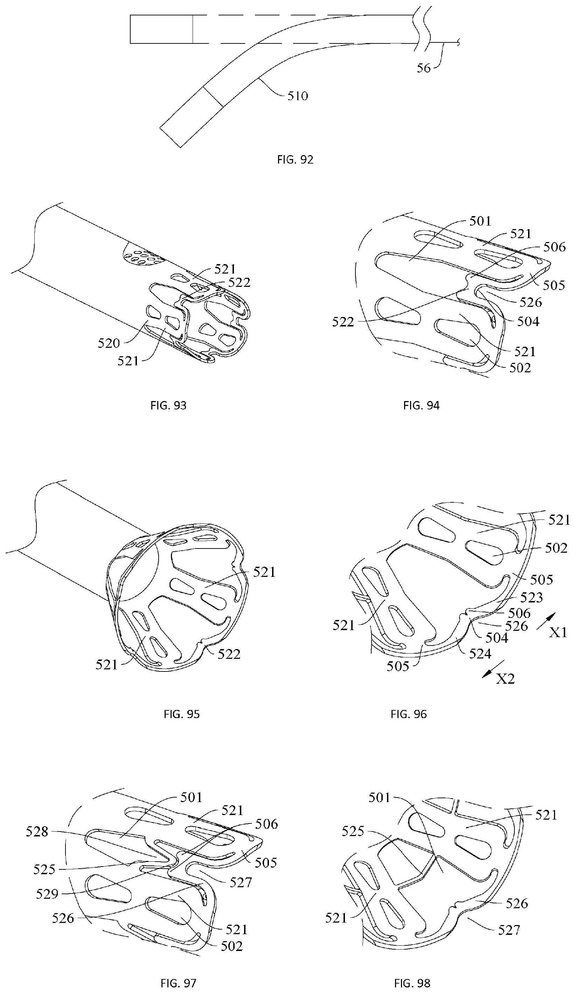

The deformation section 520 is located at the distal end of the main section 510. The deformation section 520 includes multiple elastic pieces 521 arranged at intervals along the circumference of the catheter body 51. The proximal ends of the elastic pieces 521 are connected to the metal tube 511. Each elastic piece 521 has a relative initial state (as shown in Fig. 93) and an expanded state (as shown in Fig. 95). In the expanded state, the distal ends of the elastic pieces 521 are relatively far away, and the deformation section 520 as a whole is a flared configuration. In the initial state, the elastic pieces 521 are converged and the deformation section 520 as a whole is configured as a straight cylinder or contracted radially and inwardly.

The hemostatic valve 55 can use existing technology, including a housing and a sealing member located inside the housing. The sealing member can be an elastic member or a deformable member driven by fluid. When the catheter assembly of the delivery system extends through the hemostatic valve, the sealing member prevents blood leakage.

The hemostatic valve 55 is provided with a first connecting channel 551 for injecting fluid to drive the sealing member and a second connecting channel 552 for exhaust.

The length of the catheter sheath in this application can reach a distant surgical site, and the deformation section after switching to the expanded state can assist in the retrieval of the artificial implant.

Taking aortic valve replacement as an example, the catheter sheath 70 enters from the femoral artery of the lower limb and extends until the distal end of the catheter body 51 to the aortic arch (such as the root of the ascending aorta). The catheter sheath of this embodiment can establish a long-distance channel, facilitating the delivery of catheter assembly and artificial implant in the delivery system. At the same time, it can also assist in the retrieval of the artificial implant.

Moreover, due to the fact that the deformation section after switching to the expanded state can assist in the retrieval of the artificial implant, the structure of the catheter assembly can be further simplified. For example, compared to conventional catheter assemblies, the outermost pipe fittings can be omitted.

The method of maintaining the connection between the proximal end of the artificial implant and the catheter assembly of the delivery system can be through conventional T-shaped connecting ears or through wire control, which can keep the restraint on the proximal end of the artificial implant until the pulling wire is completely released.

In one embodiment, the length of the catheter body 51 is at least 60cm, for example, the length is in range of 60-90cm.

In one embodiment, the distal end of the catheter body 51 has a pre-molded shape that matches the shape of the aortic arch, reducing the safety hazards of the catheter sheath during interventional delivery and achieving better shape matching after positioning.

As shown in Fig. 3, the distal end of the main section 510 is a three-layer structure, which includes an outer membrane layer 513, a reinforcement layer (i.e. metal tube), and an inner membrane layer 514 from the outside to the inside. The metal tube 511 is cut from nickel titanium alloy tube or woven from nickel titanium alloy wire, forming a hollow structure of the metal tube. The hollow structure not only increases flexibility and facilitates passing through turning points, but also allows the reinforcement layer to better adhere to the inner and outer membrane layers. The outer membrane layer 513 and inner membrane layer 514 can be coated with lubricating materials such as Elasthane, Pe11ethane, PeBax, or Grilamide, respectively.

The distal end of the metal tube 511 is aligned with the distal end of the main section 510, and the length of metal tube 511 is as follows:

When the artificial implant is retrieved into the catheter body 51, and the distal end of the artificial implant is aligned with the main section 510, the proximal end of the metal tube 511 exceeds or aligns with the proximal end of the artificial implant;

Alternatively, after the intervention delivery of the catheter sheath 70 is completed, the length of the metal tube 511 is sufficient to cover the aortic arch;

Alternatively, the metal tube 511 has a length equal to the main section 510.

As shown in Fig. 92, in one embodiment, the catheter sheath 70 includes a steering member that drives the catheter body 51 to bend. The distal end of the steering member extends to and acts on the distal end of the main section 510 to bend it. The proximal end of the steering member extends out of the hemostatic valve, and is connected to and controlled by the extension handle, which is set at the proximal end of the hemostatic valve 55. A locking mechanism is provided between the extension handle and the hemostatic valve 55 to limit their axial positioning. The locking mechanism may be a buckle or the like. The extension handle has a first channel for the delivery system to pass through. The extension handle may be integrated with the hemostatic valve 55.

The steering member 56 includes pulling wires, tubes, or a combination of pulling wires and tubes. For example, the steering member 56 is a pulling wire, and the distal end of the pulling wire extends and is fixed to the distal end of the main section. The extension handle controls the pulling line to act on the main section 510, which bends from the dashed line to the solid line.

In one embodiment, each elastic sheet 521 and the metal tube 511 are integrally or separate structures. Among them, the integrated structure is easy to process and shape, and can also provide structural strength.

In one embodiment, two adjacent elastic sheets 521 are connected by a connecting member 522. When the deformation section 520 is switched to the expanded state, the connecting member 522 make the elastic sheets 521 to interact with each other and expand uniformly, so that, duringretrieval of the artificial implant, the elastic sheets 521 are uniformly stressed, avoiding excessive deformation of the elastic sheets 521.

As shown in Fig. 93 to Fig. 96, in one embodiment, the connecting member 522 between adjacent elastic sheets is a V-shaped structure with a first opening facing the distal end. There is a gap opening 501 between adjacent elastic sheets, with the middle of the gap opening 501 widening and the ends narrowing. The elastic sheet 521 gradually widens near its own proximal end, which can improve the connection strength and ensure necessary rebound force. The gap opening 501 adopts an arc-shaped edge at the proximal end to disperse stress and improve safety.

In the initial state of the elastic sheet 521, the middle 504 of the connecting member 522 is folded and stored in the gap area between adjacent elastic sheets. In the expanded state, the middle 504 of the connecting member 522 of each elastic piece 521 is unfolded (relative to the initial state).

The connection member between the connecting piece 522 and the elastic piece 521 is adjacent to the distal end of the elastic piece 521, eliminating the isolated end or sharp structure of the elastic piece 521 and avoiding puncturing the structural gap of the artificial implant.

The end of the connecting piece 522 is connected to the elastic piece 521 on the corresponding side, and the connection point adjacent to the distal end of the elastic piece 521 can be understood as being close to or just at the distal end of the elastic piece 521. Especially in the expanded state, it can avoid the formation of an isolated protruding part at the distal end of the elastic piece 521, reducing the risk of interference with the artificial implant.

The shape of the middle 504 is not strictly limited, mainly used to connect and pull two elastic sheets 521 in the expanded state. In the initial state, the middle 504 needs to be folded and stored, so a foldable structure is adopted. The folding process can be driven by the elasticity of the elastic sheet 521 or combined with the predetermined elastic material of the middle 504 to assist in folding. After folding, the middle 504 is stored between adjacent elastic sheets and extends correspondingly towards the proximal end.

The connection method between the elastic sheet 521 and the connecting member 522 affects the distribution of stress and the folding effect of the connecting member 522. In one embodiment, the distal edge 505 of each elastic sheet 521 is arc-shaped, and two ends of the connecting member extend roughly along the tangential direction of the arc and are connected to the distal edge of the corresponding elastic sheet. Based on Fig. 9, the connecting member 522 has a V-shaped structure consisting of a first section 523 and a second section 524. The first section 523 extends along direction X1 towards the distal edge 505 of the elastic sheet 521, and the second section 524 extends along direction X2 towards the distal edge 505 of the elastic sheet 521. The first section 523 and the second section 524 intersect with each other integrally.

The curved shape of the distal edge 505 only expresses the general trend or overall shape characteristics, and is not strictly limited. The connecting pieces extend tangentially and intersect with each other, providing better radial convergence force and more reasonable stress distribution.

In another embodiment, the first section 523 and the second section 524 of adjacent connecting members 522 are connected to the distal edge of the same elastic sheet, and the distal edge of the joint smoothly transitions. A smooth outer edge can avoid safety hazards, for example, the first section 523 and the second section 524 are connected to the distal edge 505 of the same elastic piece 521. Due to their intersection, the joint is relatively smooth, avoiding interference from sharp or abrupt points on the artificial implant.

Furthermore, all connecting members 522 extend continuously in the circumferential direction of the catheter body. As shown in the drawings, due to the smooth joint of adjacent connecting members 522, all connecting members 522 are connected in a circular shape. Although there may be slight turning and undulating in the middle 504, it does not affect the overall trend.

In the initial state, each elastic sheet has two corners on two sides of the middle 504 of the connecting member 522, and protrusions 506 extending towards the proximal end are provided at each corner. The protrusion 506 can strengthen the structure of the corner to avoid fatigue damage caused by repeated bending.

In the expanded state of the elastic sheet, the two protrusions corresponding to the same U-shape are adjacent to or in contact with each other, which can limit the unfolding angle of the connecting member 522, preventing it from folding in reverse under extreme or abnormal conditions.

There is a gap opening 501 between adjacent elastic sheets. In the initial state of the elastic sheet, the first section 523 and the second section 524 extend into the gap opening 501 along an arc-shaped path from their own ends. That is, the connecting member 522 is arc-shaped at the distal edge 505 adjacent to the elastic sheet 521, which conforms well to the shape characteristics of the elastic sheet 521. The connecting member 522 extends close to the edge of the elastic sheet 521, and thus occupies less space. When unfolded, the arc-shaped structure will not have excessive stress concentration, reducing the safety hazards of fatigue fracture.

In the initial state of the elastic piece, the proximal end side of the connecting member 522a is adjacent to the middle of the gap opening 501 in the axial direction. In the initial state, the gap opening is a roughly strip-shaped opening, which is closed at the distal end by the connecting member 522. Each elastic piece 521 can expand radially outward to adapt to the gradual deformation of the artificial implant during expansion, and to prevent the artificial implant from suddenly collapsing during the end of expansion. In addition, when it needs to be withdrawn, the elastic pieces 521 expand radially outward to form a horn mouth to guide the retrieval of the artificial implant.

In the expanded state of the elastic piece, the vertex angle of the V-shaped portion of the connecting member 522 is greater than or equal to 120 degrees. Of course, based on the previous text, the vertex angle adopts a rounded structure, roughly U-shaped.

Each elastic sheet has a hollow area. The elastic sheets are arranged along the circumference, with a number of 3-6, such as 5.

The hollow area 502 of the elastic sheet 521 facilitates the deformation of the elastic sheet and reduces the external expansion resistance. In one embodiment, the hollow area 502 is in the shape of elongated hole, circular hole, ellipse hole, or water droplet hole. On the same elastic sheet, the hollow area 502 is one or multiple isolated areas, and the inner edges of the hollow areas are smooth, which can avoid cracking caused by excessive stress concentration during deformation. The total area of the hollow areas on the same elastic sheet is less than 50% of the elastic sheet. The hollow area 502 has multiple through holes, and the through holes on the same elastic sheet can be arranged at intervals along the axial direction or circumferential direction of the catheter body.

The outer membrane layer 513 and inner membrane layer 514 can further extend towards the distal end to cover the inner and outer sides of the deformation section 520. To reduce the risk of tearing, the membrane layer between adjacent elastic sheets can be treated with incisions to release deformation stress.

In another embodiment, as shown in Fig. 10 and Fig. 11, two adjacent elastic sheets 521 are connected by two connecting members 522. The two connecting members 522 include a first connecting member 526 and a second connecting member 525 arranged along the axial direction.

The specific structure of the first connecting member 526 can refer to the previous embodiments. In this embodiment, the second connecting member 525 is connected to the middle of the elastic sheet 521 in the axial direction (hereinafter referred to as the waist). The second connector 525 is a V-shaped structure and has a second opening 528, wherein the first opening 527 and the second opening 528 are opposite to each other.

The V-shaped inflection point of the second connecting member 525 has a relatively obvious turning trend, and the second opening 528 faces the proximal end, which can reduce the resistance and hidden dangers caused by the outward tilting of the inflection point during retraction. The deformations of the first connecting member 526 and the second connecting member 525 are different, which limits different radial deformation of the part where the elastic sheet is connected to it, resulting in the deformation section 520 as a whole presenting a conical shape with an arc-shaped generatrix, slowing down the bending angle at the turning point between the deformation section and the main section.

The waist of the elastic sheet 521 is inwardly retracted in the circumferential direction compared to its distal and proximal ends, and the root of the second connecting member 525 transitions into an arc shape with the waist.

To avoid potential resistance caused by the two protrusions 506 when pushing the catheter assembly and artificial implant to the distal end of the catheter sheath, the vertex 529 of the second connecting member 525 is located inside the two protrusions 506, which can provide guidance.

In another embodiment, as shown in Fig. 12, unlike the previous embodiments, the deformation section 520 as a whole is a mesh structure, and the elastic sheet 521 can be understood as a partial mesh structure in the circumferential direction. The deformation of the mesh structure can adapt to the state switching of the elastic sheet.

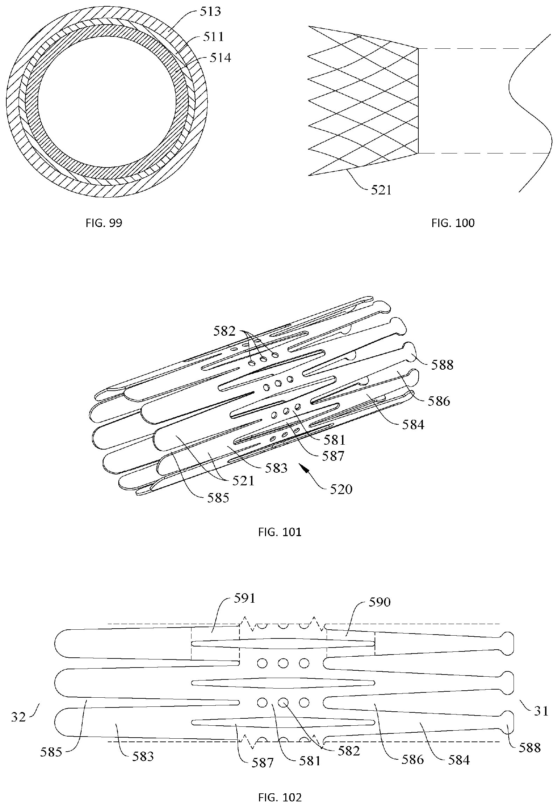

As shown in Fig. 101 to Fig. 102, in another embodiment, adjacent elastic sheets 521 are connected at their middles and form a connecting portion 581. The connecting portion 581 is provided with a first opening 582, which can be used to release stress when the deformation section 520 expands or contracts. The first opening is a circular hole, with a quantity of multiple. The multiple first openings 582 are arranged at intervals along the axial direction of the deformation section, and the size of the first openings 582 are the same.

In one embodiment, the elastic sheet 521 extends from the connecting portion 581 towards the distal end and gradually converges to form a third section 583. The distal end of the elastic sheet 521 is a circular arc structure. The elastic piece 521 extends from the connecting portion 581 towards the proximal end and gradually converges to form a fourth section 584. The convergence trend of the fourth section is greater than that of the third section.

In one embodiment, a second gap 585 is provided between adjacent third sections 583, and a third gap 586 is provided between adjacent fourth sections 584. In the initial state, the second gap 585 and the third gap 586 form a V-shape.

In one embodiment, a second opening 587 is provided on the same elastic sheet 521 for releasing stress during deformation. The second opening 587 extends along the axial direction of the deformation section, with two ends thereof extending with equal distances to the third section 583 and the fourth section 584, respectively. The second opening 587 gradually narrows into a V-shaped structure from the middle to two axial ends.

The proximal end of the fourth section 584 is provided with a connector 588 for connecting with the main section, and the connector 588 in the figure is a T-shaped structure. It can also refer to the previous embodiment, where the proximal end of the fourth section 584 is connected to the main section, that is, the deformation section is integrally formed with the main section.

Two ends of the second opening 587 coincide with the second gap 585 and the third gap 586 in the axial position, so that the third section 583 and the fourth section 584 have two first deformation bars 591 and two second deformation bars 592 arranged in a V-shape, respectively. When expanding outward, the first deformation bar 591 and the second deformation bar 592 move away from each other in the circumferential direction.

In one embodiment, there is at least one elastic sheet with a different length from the other elastic sheets, or the lengths of the elastic sheets are different from each other. The length of the elastic sheet means the length between two ends of the elastic sheet along the axial direction, and the proximal ends of the elastic sheets are located at the same axial position.

In the preferred embodiment, the elastic sheet includes first and second elastic sheets neighboring to each other along the circumferential direction, wherein the length of the first elastic sheet is greater than the length of the second elastic sheet.

The present application also provides a delivery system, including a catheter assembly loaded with an artificial implant at the distal end, a control handle connected to the proximal end of the catheter assembly to control the catheter assembly, and the catheter sheath 70 of the above embodiments. The catheter assembly can be inserted into the catheter sheath through a hemostatic valve. For the specific structure of the catheter assembly and the control handle, existing technology can be used. Moreover, due to the fact that the deformation section can assist in the retrieval of the artificial implant after switching to the expanded state, the structure of the catheter assembly can be further simplified. For example, compared to conventional catheter assembly, the outermost pipe fittings can be omitted.

The catheter sheath in this application is long to reach a relatively distant surgical site, such as extending to the ascending aorta, thereby constructing a longer channel for the delivery of the catheter assembly and the artificial implant.

The various technical features of the above embodiments can be combined in any way. In order to make the description concise, not all possible combinations of the technical features in the above embodiments have been described. However, as long as there is no contradiction in the combination of these technical features, they should be considered within the scope of this specification. When the technical features of different embodiments are reflected in the same figure, it can be regarded that the figure also discloses the combination examples of the various embodiments involved.

The above embodiments only express several embodiments of the present application, and their descriptions are more specific and detailed, but should not be understood as limiting the scope of the patent application. It should be pointed out that for those ordinary skilled in the art, modifications and improvements can be made without departing from the concept of this application, which should be within the protection scope of this application.

## Claims

1. A control mechanism for an artificial implant, comprising:
a base defining a locking hole;
a pulling wire having a free end capable of passing through and winding around the artificial implant or being detached from the artificial implant; and
a locking member as a whole located at a distal end of the base, the locking member rotatably engaged with the base to lock or unlock the free end of the pulling wire.

2. The control mechanism of claim 1, further comprising a first shaft, a second shaft, and a third shaft that are slidably nested in sequence from the inside out, the base is connected to a distal end of the third shaft, the pulling wire is connected to a distal end of the second shaft, and the locking member is connected to a distal end of the first shaft.

3. The control mechanism of claim 1, wherein the base has a locking area, the locking member includes a positioning portion that enters or exits the locking area during its rotation, and the positioning portion cooperates with the free end of the pulling wire to restrict the artificial implant;
the base includes a distal end and a proximal end opposite to each other, as well as an axial direction extending between the distal end and the proximal end, a first cavity is defined in the base, and a first opening is defined in a proximal end side of the base and communicates with the first cavity, and a second opening is defined in an outer circumferential surface of the base and communicates with the first cavity;
the pulling wire extends through the first cavity, one end of the pulling wire extends proximally out of the base through the first opening, and another end/free end of the pulling wire extends out of the base through the second opening for connecting the artificial implant.

4. The control mechanism of claim 3, wherein the locking area is located at the second opening.

5. The control mechanism of claim 4, wherein there are multiple second openings arranged at intervals along a circumferential direction of the base, and multiple ribs are formed between adjacent second openings.

6. The control mechanism of claim 5, wherein all of the ribs converge and are fixed to each other at the distal end of the base.

7. The control mechanism of claim 6, wherein all of the ribs converge to form an annular structure.

8. The control mechanism of claim 5, wherein an open direction of the locking hole is along the circumferential direction of the base.

9. The control mechanism of claim 5, wherein at least one rib defines the locking hole.

10. The control mechanism of claim 5, wherein each rib defines the locking hole.

11. The control mechanism of claim 5, wherein there are one locking hole or multiple locking holes on one rib.

12. The control mechanism of claim 5, wherein there are multiple locking holes arranged in sequence along an extending direction of the rib where the multiple locking holes are defined.

13. The control mechanism of claim 3, wherein there are multiple ribs configured with the locking holes, the positioning portion as a whole comprises a head end and a tail end opposite to each other, and the head end is engaged into or disengaged from the locking holes in sequence along with the rotation of the locking member.

14. The control mechanism of claim 3, wherein the free end of the pulling wire has a first state being restricted to the base and a second state being released from the base, and the restriction on the artificial implant is released when the cooperation of the locking member and the free end of the pulling wire is released;
in the first state, the free end extends out of the base through a corresponding second opening, winds around the artificial implant, and returns to a locking area corresponding to the same second opening, or returns to a locking area corresponding to an adjacent second opening.

15. The control mechanism of claim 14, wherein the free end of the pulling wire in the first state is located in a current locking area, and two ends of the positioning portion located in the current locking area are inserted into the locking holes at corresponding sides, respectively.

16. The control mechanism of claim 3, wherein there are multiple locking areas arranged at intervals along a circumferential direction of the base, and the positioning portion enters or exits the locking areas in sequence along with the movement of the locking member.

17. The control mechanism of claim 1, wherein the base has a locking area, the locking member has a positioning portion that enters or exits the locking area during its rotation, and the positioning portion cooperates with the free end of the pulling wire to restrict the artificial implant;
the positioning portion has a curved shape and extends along the circumferential direction the base, and enters or exits the locking hole along with the rotation of the locking member.

18. The control mechanism of claim 17, wherein the positioning portion comprises at least one arc-shaped structure that cooperates with the base.

19. The control mechanism of claim 17, wherein the positioning portion is configured as a helical structure.

20. The control mechanism of claim 19, wherein the helical structure is wound at least once.

21. The control mechanism of claim 19, wherein the helical structure is a multi-turn structure, and the turns are arranged along an axial direction of the base.

22. The control mechanism of claim 19, wherein the helical structure is a cylindrical helical structure or at least partially a conical helical structure.

23. The control mechanism of claim 19, wherein, along a winding direction of the helical structure, the positioning portion as a whole comprises a distal end and a head end opposite to each other, and a connecting portion is fixed to one of the head end and the distal end.

24. The control mechanism of claim 23, wherein the connecting portion is tubular-shaped, and the distal end of the positioning portion is mounted around an outer circumference of the connecting portion or abuts against a proximal end of the connecting portion.

25. The control mechanism of claim 24, wherein the connecting portion is movably mounted around the distal end of the first shaft, and is capable of being separated from the distal end of the first shaft in the axial direction.

26. The control mechanism of claim 17, wherein the free end of the pulling wire has a first state being restricted to the base and a second state being released from the base, and the restriction on the artificial implant is released when the cooperation of the locking member and the free end of the pulling wire is released;
in the first state, a limit mechanism is provided between the locking member and the base to prevent the locking member from rotating in an insertion direction.

27. The control mechanism of claim 26, wherein the positioning portion as a whole has a tail end and a head end opposite to each other, and a connecting portion is fixed to one of the tail end and the head end, a positioning mechanism is provided between the connecting portion and the base to maintain their axial positions;
the positioning mechanism comprises a first end surface provided at a proximal end of the connecting portion and a second end surface provided at the distal end of the base, wherein the second end face abuts against the first end surface.

28. The control mechanism of claim 27, wherein the connecting portion is tubular-shaped with constant diameter or at least expands radially outward at its proximal end, a proximal end of the connecting portion serves as the connecting section, the distal end of the base comprises an extension section, and the connecting section and the extension section are nested with each other.

29. The control mechanism of claim 1, wherein the free end of the pulling wire has a first state being restricted to the base and a second state being released from the base, and the restriction on the artificial implant is released when the cooperation of the locking member and the free end of the pulling wire is released;
the pulling wire further comprises a control end opposite to the free end, the control end is movable relative to the base;
when the free end is in the first state, a length of the pulling wire exposed outside the base is adjustable by operating the control end; and
when the free end is in the second state, the pulling wire is detachable from the artificial implant by operating the control end.

30. The control mechanism of claim 29, wherein there are multiple pulling wires, the free ends of the pulling wires move independently of each other, while the control ends of the pulling wires move synchronously.

31. The control mechanism of claim 1, wherein the base has a locking area, the locking member has a positioning portion that enters or exits the locking area during its rotation, and the positioning portion cooperates with the free end of the pulling wire to restrict the artificial implant;
the free end has a ring, when the free end is in the first state, the positioning portion enters the ring; and when the free end is in the second state, the positioning portion exits the ring.

32. The control mechanism of claim 31, wherein the ring is configured independently or formed by winding the pulling wire.

33. The control mechanism of claim 31, wherein the positioning portion is helical-shaped with multiple turns, an axial gap is defined between adjacent turns; the pulling wire in the first state extends out of the base through a current axial gap, winds around the artificial implant, and is mounted around a turn adjacent to the current axial gap.

34. The control mechanism of claim 31, wherein the pulling wire in the first state extends out of the base through a current locking area, winds around the artificial implant, and returns to the current locking area.

35. The control mechanism of claim 1, wherein the artificial implant has an axial direction, and one end of the artificial implant in the axial direction is configured as a wire control end, the wire control end has eyelets for extending of the pulling wire therethrough, and the artificial implant has:
a collapsed state, wherein the wire control end is radially folded close to the base;
an expanded state, wherein the wire control end expands radially relative to the base;
a round-trip path of the pulling wire winding around the artificial implant do not coincide.

36. The control mechanism of claim 35, wherein there are multiple eyelets being spaced from each each other, and in the expanded state, the pulling wire passes through at least two eyelets.

37. The control mechanism of claim 35, wherein the pulling wire winds around the artificial implant to form a triangular-shaped round-trip path.

38. The control mechanism of claim 35, wherein a number of the eyelets is twice that of the pulling wires, and in the expanded state, each pulling wire corresponds to two eyelets.

39. A locking mechanism for connecting an artificial implant to a delivery system, comprising:
a first shaft configured with a loading section fixed to its distal end, the first loading section opening towards a proximal end for accommodating a distal part of the artificial implant;
a pulling wire having a free end being capable of winding around and extending through the artificial implant or detached from the artificial implant;
a third shaft being slidably mounted around the first shaft, a locking assembly being set on the third shaft, wherein the artificial implant is bound to the locking assembly by the pulling wire during loading, and the locking assembly comprises a locking member and a base, and has a locking state that the locking member and the base cooperate with each other to restrict the pulling wire from being detached from the artificial implant and a releasing state that the locking member and the base are disengaged to allow the pulling wire to be detached from the artificial implant; and
a linkage assembly acting between the first shaft and the locking member to selectively link the first shaft and the locking member.

40. The locking mechanism of claim 39, wherein the locking member as a whole is located at the distal end of the base, and rotatably engages with the base to lock or release the free end of the pulling wire.

41. The locking mechanism of claim 40, wherein the linkage assembly comprises two mating parts, one of the two mating parts is connected to the loading section and another one of the two mating parts is connected to the locking member, and axial movement of the loading section makes the two mating parts engage with or disengage from each other.

42. The locking mechanism of claim 41, wherein the two mating parts comprise a linkage key and a linkage groove which are separable through axial sliding, and the linkage key and the linkage groove are rotatably linked to each other when the linkage key is inserted into the linkage groove.

43. The locking mechanism of claim 42, wherein there are multiple linkage keys connected to each other through a tubular component, and the multiple linkage keys are arranged along a circumferential direction of the tubular component.

44. The locking mechanism of claim 43, wherein there are multiple linkage grooves connected to each other through the tubular component, and the multiple linkage grooves are arranged along the circumferential direction of the tubular component.

45. The locking mechanism of claim 42, wherein at least a part of the locking member serves as the connecting portion, and the linkage key or the linkage groove is set at a distal end of the connecting portion.

46. The locking mechanism of claim 45, wherein the connecting portion is located outside the base.

47. The locking mechanism of claim 45, wherein the linkage groove is defined in a pipe wall at the distal end of the connecting portion, and the linkage key is provided on an inner circumferential wall of the loading section.

48. The locking mechanism of claim 42, wherein the linkage groove comprises a flared structure formed at its open side.

49. The locking mechanism of claim 41, wherein the two mating parts comprises a linkage key and a linkage groove that are separable through rotating, and the linkage key and the linkage groove are axially linked to each other when the linkage key is inserted into the linkage groove.

50. The locking mechanism of claim 39, wherein a support member is provided on an inner circumference of the loading section and fixed to the distal end of the first shaft, and one of the two mating parts is provided on the support member.

51. The locking mechanism of claim 50, wherein a limit structure is provided between the support member and the loading section to prevent axial separation of the support member and the loading section.

52. The locking mechanism of claim 51, wherein the limit structure comprises a positioning piece protruding radially from an outer circumference of the support member, and a positioning groove defined in an inner circumference of the loading section to cooperate with the positioning piece.

53. The locking mechanism of claim 52, wherein the support member is a cylindrical structure, and an outer circumference of the support member is partially flipped to form the positioning piece.

54. The locking mechanism of claim 53, wherein the support member comprises an outer cylinder connected to the loading section, and an inner cylinder configured with a fitting portion and fixed to the first shaft.

55. The locking mechanism of claim 54, wherein the inner cylinder and the outer cylinder are separate structures.

56. An interventional delivery system for an artificial implant, comprising:
a catheter sheath for constructing an interventional channel, a proximal end of the catheter sheath being provided with a fixed seat;
a steering assembly configured with a distal end thereof being controllable to change its orientation, the steering assembly sliding through the fixed seat and extending towards a proximal end;
an inner shaft assembly configured with a distal end thereof being provided with a loading section for connecting the artificial implant, the loading section being always exposed to the distal end of the steering assembly; and
a control handle connecting proximal ends of the steering assembly and the inner shaft assembly, the control handle being located at a proximal end side of the fixed seat, and a distance between the control handle and the fixed seat being adjustable.

57. The interventional delivery system of claim 56, wherein the inner shaft assembly comprises a first shaft, a second shaft, and a third shaft that are slidably nested in sequence from the inside out,
a distal end of the first shaft is linked with a locking member;
a distal end of the second shaft is connected to a pulling wire; and
a distal end of the third shaft is fixed with a base, the locking member is movably mounted on the base, the base defines a locking hole that matches the locking member; and wherein
the pulling wire is wound around the artificial implant from the second shaft and then constrained to the base by the locking member, and the second shaft and the first shaft slidably match the third shaft.

58. The interventional delivery system of claim 57, wherein the locking member as a whole is located at the distal end of the base, and the locking member rotatably cooperates with the base to lock or release the free end of the pulling wire.

59. A control method for separating an artificial implant from an interventional delivery system, comprising:
providing an artificial implant and an interventional delivery system for delivering the artificial implant, wherein the artificial implant comprises an inner frame defining a blood flow channel, an outer circumference of the inner frame is provided with a plurality of arm portions, a first gap is defined between the arm portions and the inner frame for accommodating native tissue, leaflets for controlling the blood flow channel are connected to the inner frame, a distal end of the inner frame is held in a compressed state by a tubular-shaped loading section, and a proximal end of the inner frame is held in a compressed state by the pulling wire;
expanding at least one arm portion to align the first gap with the native tissue;
driving the loading section towards the distal end to separate the loading section from the inner frame, so as to make a distal part of the inner frame expand;
elongating a part of the pulling wire exposed out of the locking member to make a proximal part of the inner frame expand, thereby the inner frame as a whole deforming to an expected amplitude, while always maintaining control of the pulling wire to the inner frame;
moving the loading section towards the proximal end to transfer at least a part of the loading section to a proximal end side of the leaflet through the blood flow channel; and
releasing the connection between the pulling wire and the inner frame.

60. A control method for recovery of an artificial implant using an interventional delivery system, comprising:
providing an artificial implant and an interventional delivery system for delivering the artificial implant, wherein the interventional delivery system comprises:
a catheter sheath for constructing an interventional channel, a fixed seat being provided at a proximal end of the catheter sheath;
an inner shaft assembly configured with a loading section at its distal end, wherein at least a part of the artificial implant before expansion is accommodated in the loading section and connected to the inner shaft assembly in a releasable manner;
a control handle connected to a proximal end of the inner shaft assembly, the control handle located at a proximal end side of the fixed seat, and a distal end of the control handle provided with a push mechanism for acting on the fixed seat;
wherein the artificial implant is kept at least partly in connection with the inner shaft assembly, and during recovery of the artificial implant, the catheter sheath is driven to move relative to the inner shaft assembly, allowing the artificial implant to be accommodated in the catheter sheath.

61. The control method of claim 60, wherein the distal end of the catheter sheath is close to or in contact with the artificial implant, and drives the push mechanism to abut against the fixed seat, and further pushes the fixed seat towards the distal end until the artificial implant is accommodated in the catheter sheath.

62. The control method of claim 60, wherein the artificial implant is connected to the inner shaft assembly through a pulling wire, and during recovery of the artificial implant, the pulling wire is tightened to bring a proximal end side of the artificial implant together.

63. The control method of claim 60, wherein before recovery of the artificial implant, the artificial implant is in an expanded state and is only connected to the inner shaft assembly through a pulling wire; and the pulling wire is tightened to bring a proximal end side of the artificial implant together.

64. The control method of claim 60, wherein the artificial implant comprises an inner frame defining the blood flow channel, an outer circumference of the inner frame is provided with multiple arm portions, a proximal end of the arm portion is fixed to the inner frame, a first gap is defined between a distal end of the arm portion and the inner frame for accommodating native tissue, and leaflets for controlling the blood flow channel are connected to the inner frame;
during recovery of the artificial implant, the proximal end of the arm portion first enters the catheter sheath, and the distal end of the arm portion adapts to the movement of the catheter sheath relative to the artificial implant and enters the catheter sheath.

65. A loading method for loading an artificial implant into a delivery system, comprising:
providing an artificial implant and a delivery system, wherein the artificial implant comprises an inner frame, an outer circumference of the inner frame is provided with multiple arm portions; and the delivery system comprises:
a catheter sheath with a fixed seat at its proximal end;
an inner shaft assembly movably extending through the catheter sheath, and a distal end of the inner shaft assembly being provided with a loading section; and
a control handle connected to a proximal end of the inner shaft assembly;
winding around a proximal end of the inner frame by means of a pulling wire;
wherein the delivery system further comprises a control mechanism, the control mechanism comprises a base connected to the inner shaft assembly, a locking member movably cooperating with the base, and multiple pulling wires, one end of the pulling wire is connected to the control handle, and another end of the pulling wire is locked to the base and the locking member after passing through and winding around the inner frame, and the multiple pulling wires are locked one by one.

66. The loading method of claim 65, wherein during the locking process, the locking member rotates relative to the base.

67. The loading method of claim 66, wherein during the locking process, the pulling wire is tightened to radially compress the proximal end of the inner frame.

68. The loading method of claim 67, wherein the multiple pulling wires are synchronously tightened.

69. The loading method of claim 66, wherein accommodating the distal end of the inner frame in the loading section is achieved by moving the catheter sheath to the distal end to accommodate the arm portion and the proximal end of the inner frame.

70. The loading method of claim 69, wherein the distal end of the inner frame and at least the distal end of the arm portion are accommodated in the loading section.

71. The loading method of claim 70, wherein the catheter sheath moves towards the distal end to accommodate the proximal end of the inner frame and the exposed portion of the arm portion.
